(19)

**Europäisches Patentamt**
**European Patent Office**
**Office européen des brevets**

(11)  **EP 2 553 493 B1**

(12)  # EUROPEAN PATENT SPECIFICATION

(45) Date of publication and mention
of the grant of the patent:
**24.08.2016  Bulletin 2016/34**

(51) Int Cl.:
***G01T 1/164*** *(2006.01)*

(21) Application number: **11798535.8**

(22) Date of filing: **04.04.2011**

(86) International application number:
**PCT/US2011/031104**

(87) International publication number:
**WO 2011/162851 (29.12.2011 Gazette 2011/52)**

(54) **HIGH PERFORMANCE COMPUTING FOR THREE DIMENSIONAL PROTON COMPUTER TOMOGRAPHY (HPC-PCT)**

HOCHLEISTUNGSBERECHNUNG FÜR DREIDIMENSIONALE PROTONENCOMPUTERTOMOGRAPHIE

CALCUL À HAUTES PERFORMANCES POUR TOMOGRAPHIE PAR PROTONS EN TROIS DIMENSIONS (HPC-PCT).

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **02.04.2010  US 320542 P**

(43) Date of publication of application:
**06.02.2013  Bulletin 2013/06**

(73) Proprietors:
• **Northern Illinois University**
  **DeKalb, IL 60115 (US)**
• **Fermi Research Alliance, LLC**
  **Batavia, IL 60510-5011 (US)**
• **University of Wollongong**
  **Wollongong, NSW 2522 (AU)**

(72) Inventors:
• **KARONIS, Nicholas**
  **West Chicago**
  **IL 60185 (US)**
• **COUTRAKON, George**
  **Redlands**
  **CA 92373 (US)**
• **DUFFIN, Kirk**
  **Dekalb**
  **IL 60115 (US)**
• **ERDELYI, Bela**
  **Romeoville**
  **IL 60446 (US)**

• **NAGLICH, Kevin**
  **Elgin**
  **IL 60123 (US)**
• **PENFOLD, Scott**
  **Adelaide, SA 5000 (AU)**
• **RUBINOV, Paul**
  **Batavia**
  **IL 60657 (US)**
• **RYKALIN, Victor**
  **Aurora**
  **IL 60502 (US)**
• **ZUTSHI, Vishnu**
  **Dekalb**
  **IL 60115 (US)**

(74) Representative: **Grund, Martin**
**Grund**
**Intellectual Property Group**
**Postfach 44 05 16**
**80754 München (DE)**

(56) References cited:
**US-A- 6 078 052       US-A1- 2007 040 830**
**US-A1- 2007 045 547   US-A1- 2009 134 334**
**US-B2- 6 635 877**

**(Cont. next page)**

- SCHULTE R ET AL: "Design of a proton computed tomography system for applications in proton radiation therapy", 2003 IEEE NUCLEAR SCIENCE SYMPOSIUM CONFERENCE RECORD. / 2003 IEEE NUCLEAR SCIENCE SYMPOSIUM AND MEDICAL IMAGING CONFERENCE. PORTLAND, OR, OCT. 19 - 25, 2003; [IEEE NUCLEAR SCIENCE SYMPOSIUM CONFERENCE RECORD], NEW YORK, NY : IEEE, US, 19 October 2003 (2003-10-19), page 1579, XP010735912, ISBN: 978-0-7803-8257-2
- PEMLER P ET AL: "A detector system for proton radiography on the gantry of the Paul-Scherrer-Institute", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NL, vol. 432, no. 2-3, 11 August 1999 (1999-08-11), pages 483-495, XP027317643, ISSN: 0168-9002 [retrieved on 1999-08-11]
- TALAMONTI C ET AL: "Proton radiography for clinical applications", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NL, vol. 612, no. 3, 11 January 2010 (2010-01-11), pages 571-575, XP026831753, ISSN: 0168-9002 [retrieved on 2009-08-15]
- LI TIANFANG ET AL: "Reconstruction for proton computed tomography by tracing proton trajectories: A Monte Carlo study", MEDICAL PHYSICS, AIP, MELVILLE, NY, US, vol. 33, no. 3, 22 February 2006 (2006-02-22), pages 699-706, XP012092035, ISSN: 0094-2405, DOI: 10.1118/1.2171507
- CIRRONE ET AL: "The Italian project for a proton imaging device", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NL, vol. 576, no. 1, 12 May 2007 (2007-05-12), pages 194-197, XP022072110, ISSN: 0168-9002, DOI: 10.1016/J.NIMA.2007.01.151
- RYAN J M ET AL: "A scintillating plastic fiber tracking detector for neutron and proton imaging and spectroscopy", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NL, vol. 422, no. 1-3, 11 February 1999 (1999-02-11), pages 49-53, XP004161799, ISSN: 0168-9002, DOI: 10.1016/S0168-9002(98)01061-4

**Description**

GRANT INFORMATION

Research in this application was supported in part by a grant from the Department of Army USA Medical Research Mat Command (Grant No. W81XWH-10-1-0170). The Government has certain rights in the invention.

BACKGROUND OF THE INVENTION

1. TECHNICAL FIELD

The present invention relates to three-dimensional computing. In particular, the present invention relates to proton-computed tomography.

2. BACKGROUND ART

Generating an internal 3D image of an object with proton computed tomography (pCT) starts by firing many protons through the object. Each proton's path (i.e., trace history) is recorded and the collection of all the trace histories are used as input to a computer program that generates the image. Some posit that images generated through pCT will be more effective in proton-based treatments of cancer than images produced through X-rays.

The algorithms that produce images from proton trace histories are complex and involve many stages. The computer memory and speed needed to produce a quality 3D image of an object for which proton-based cancer treatment can be applied (e.g., a human head or pelvis) within clinically meaningful time frames exceeds the capacity of even the most powerful desktop computers available today. Therefore, there is a need for an algorithm to make images from proton trace histories more effectively.

Detectors for pCT in the prior art employ larger bulky plastic cube shaped tubes (such as in Pemler, et al.) with large and bulky photon sensors that require much larger volumes that present problems when mounted on a proton gantry with limited space around the patient. Pemler, et al. describes the possibility to perform radiography with protons and used a single X-Y plane in front of the object being analyzed and a single X-Y plane after. Pemler, et al. also uses vacuum photomultipliers, square fibers, and combinatorial readout. Amaldi, et al. describes a Proton Range Radiography system with an imaging area of 10 cm x 10 cm with a stack of thirty thin plastic scintillators. The current pCT detector at Loma Linda uses silicon wafers that are limited in available area (9 cm x 9 cm), thus requiring a mosaic of overlapping tiles to achieve large area (27 x 36 cm). The maximum size now produced of silicon wafers is 10 cm x 10 cm. The tiles are shingle overlapped or placed with edges butting, which create dead space in the detector or double layers that require "mathematical removal" during image calculations.

Therefore, there also remains a need for a detector for pCT that eliminates dead space and double layers created by the arrangement of tiles.

SUMMARY OF THE INVENTION

The present invention provides for a proton computed tomography (pCT) detector system as defined in claim 1, including two tracking (2D) detectors in sequence on a first side of an object to be imaged, two tracking (2D) detectors in sequence on an opposite side of the object to be imaged, and a calorimeter, wherein the tracking detectors include plastic scintillation fibers.

The present invention also provides for a method of imaging an object as defined in claim 16 by emitting protons from a source through two tracking detectors, through and around the object, and through two opposite tracking detectors, detecting energy of the protons with a calorimeter, and imaging the object.

DESCRIPTION OF THE DRAWINGS

Other advantages of the present invention are readily appreciated as the same becomes better understood by reference to the following detailed description when considered in connection with the accompanying drawings wherein:

FIGURE 1 is a layout of the pCT detector system of the present invention;
FIGURE 2 is a three-dimensional representation of the pCT detector system core idea of the present invention;
FIGURES 3A-3C are three-dimensional representations of one plane of the tracking detector of the present invention;
FIGURE 4 is a three-dimensional representation of the system of the present invention;
FIGURE 5 is flow-chart showing the process of generating images with the scanner system;

FIGURE 6A is a front view of a silicon strip detector, and FIGURE 6B is a graph of a proton beam detected;

FIGURE 7 is a perspective view of fibers of the tracking detector; and

FIGURE 8 is perspective view of a scintillating plate as part of the calorimeter.

## DETAILED DESCRIPTION OF THE INVENTION

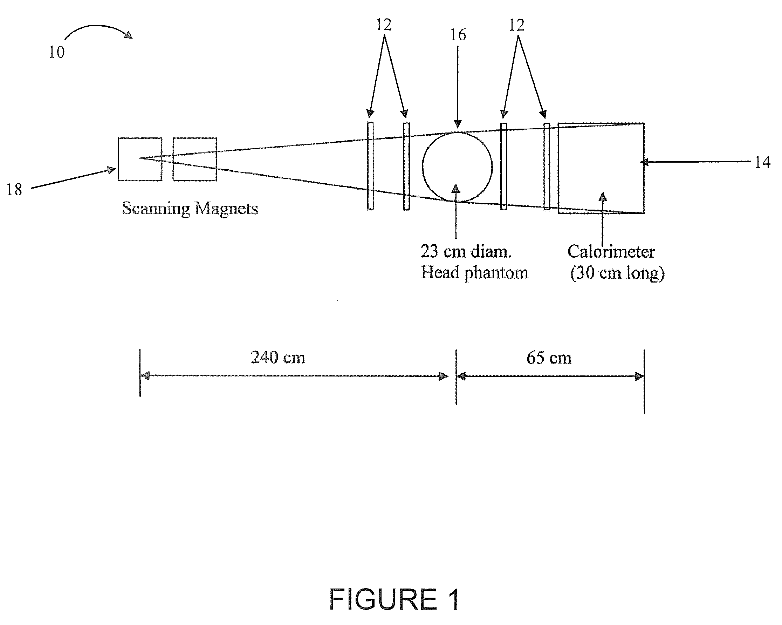

The present invention provides for a pCT detector system 10, shown generally in FIGURE 1, for generating 3D images from proton trace histories. The pCT detector system 10 preferably includes four tracking detectors 12, a calorimeter 14, and a computer cluster (not shown).

The tracking detectors 12 are used to detect the coordinates in the X and Y plane of the protons. Each detector 12 has two planes, an X plane and a Y plane, with the fibers (strips) oriented in the appropriate direction for X and Y measurements of the proton tracks. Each detector is fabricated by placing one X and one Y plane in close proximity with a thin foam-like material in between.

There are preferably four tracking detectors 12 arranged around the object 16 to be imaged, two on one side of the object 16 and two on the opposite side of the object 16. This arrangement is further described below. There can also be more than two tracking detectors 12 on each side of the object 16 in order to create redundancy. The tracking detectors 12 include 1 mm diameter thin circular plastic scintillation fibers 22, closely packed in a row, and covering the full area of the imaging field, preferably 27 cm by 36 cm, or 18 cm x 36 cm. The tracking detectors 12 can be any other suitable size. The core of the fibers is preferably polystyrene with cladding of PMMA (Poly(methyl methacrylate)). The tracking detectors 12 also include appropriate electronics in order to detect the position of the protons and relay this data to a computer cluster.

The use of scintillation fibers for proton imaging has been reported in the prior art, but the tracking detectors 12 of the present invention offer higher spatial resolution and larger area coverage than previous detectors and eliminate the tile construction of the prior art. Up to six meters can be covered with these plastic scintillating fibers 22 without dead area. The smaller circular fibers 22 produce better resolution to sub-millimeter level over the larger square tubes used in prior art devices. One crucial difference between the present invention and the prior art is the use of SiPM (or solid state photomultiplier) 20 while reading out scintillating fibers 22. Previous devices were based on using bulky vacuum photomultipliers with poor light sensitivity in green diapazon of the light spectrum. The prior art used thick square scintillating fibers in order to increase light output. The tracking detectors 12 of the present invention use SiPM 20 with as much as two time higher light sensitivity and allows for use of thinner fibers 22.

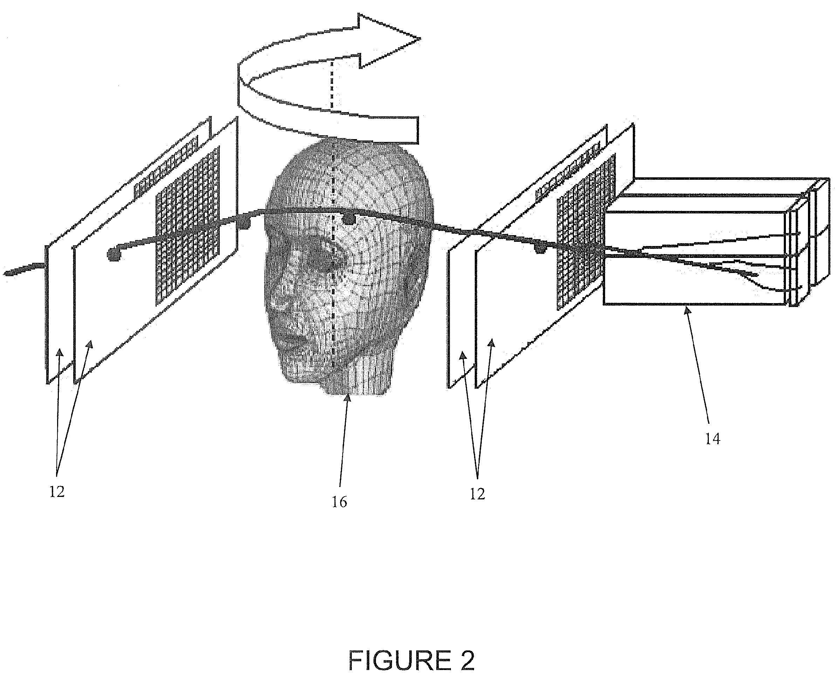

The tracking detectors 12 are arranged so that two are placed in sequence on a first side of an object 16 to be imaged and two are placed in sequence on an opposite side of the object 16, as shown in FIGURES 1 and 2. Scanning magnets (or a source) 18 that emit the proton beams of the system 10 are located at a position opposite to the calorimeter 14 on either side of the outer tracking detectors 12. For example, the scanning magnets 18 can be located 240 cm from the center of the object 16, and the calorimeter 14 can be 65 cm from the center of the object 16. The inner tracking detectors 12 can be 15 cm from the center of the object 16 and the outer tracking detectors 12 can be 30 cm from the center of the object 16. Other distances can be used as appropriate.

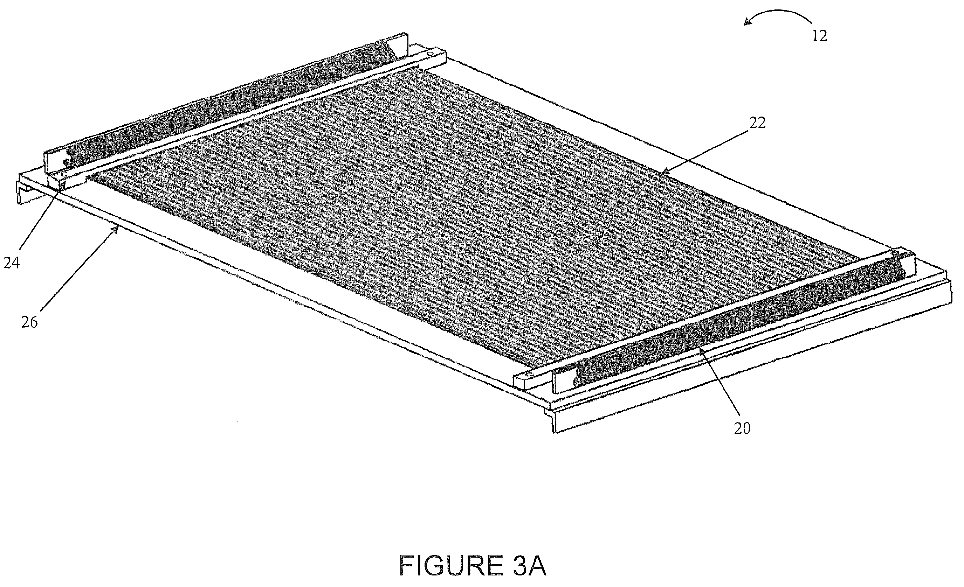

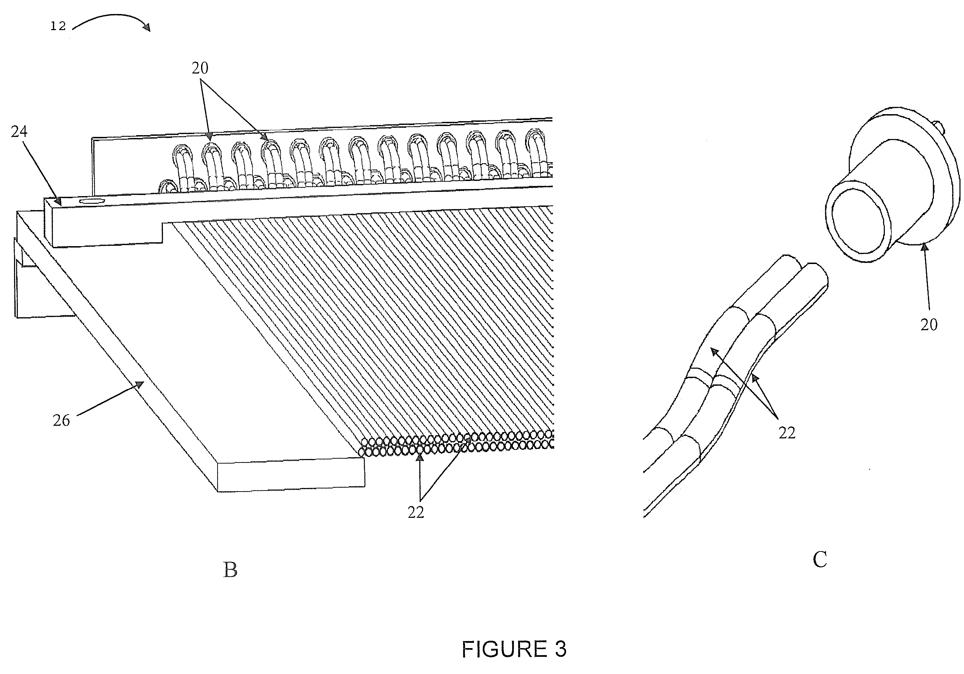

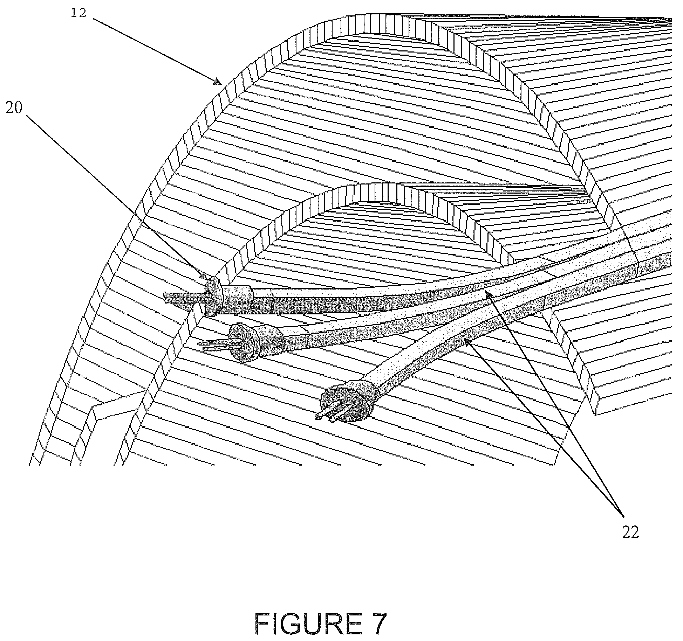

The tracking detectors 12 include the use of low cost Silicon Photomultipliers (SiPM) 20 attached to the plastic scintillating fibers 22 for signal amplification in lieu of phototubes as used in the prior art, as shown in FIGURES 3A-3C. The SiPMs 20 provide continuous coverage and remove the tile construction effect. SiPMs are unaffected by magnetics contrary to phototubes. SiPMs have been historically used in particle detectors. SiPMs are intrinsically fast, of order 100 nanosecond resolving time between events. Therefore, this design allows much higher data acquisition rates than previous detector systems. The plastic scintillating fibers 22 can have a cross-sectional shape of a square (shown in FIGURE 7), circle, or hexagon. The tracking detectors 12 with SiPMs 20 can include two scintillating fibers 22 simultaneously, alternatively, one fiber 22 can be used for one SiPM 20. Preferably, the fiber 22 diameter is in the range of 0.75 mm - 1 mm. The fibers 22 can be polystyrene scintillating fibers that produce light when protons cross their thickness. Two layers of fibers 22 increase detection efficiency in contrast to one layer. Another orthogonal double layer of fibers 22 can be included in order to have a 2D coordinate of the protons in space. As shown in FIGURES 3A-3B, the tracking detectors can include a mechanical support 24 for the SiPM, as well as a Rohacell support 26 that serves as a support for scintillating fiber 22 placing. Any other supports can be used as known in the art in order to create the tracking detectors 12.

In one example of a tracking detector 12, the area of 27x36 cm$^2$ (tracking part) is covered. If the picked diameter of the scintillating fiber 22 is 1 mm then 270 fibers, 270 SiPMs, and 270 channels of electronics are needed. This is true for a one projection only (X). The 2D plane includes X and Y projections (270 + 360 = 630 channels). The total tracking detector 12 includes approximately 630 X 4= 2520 channels.

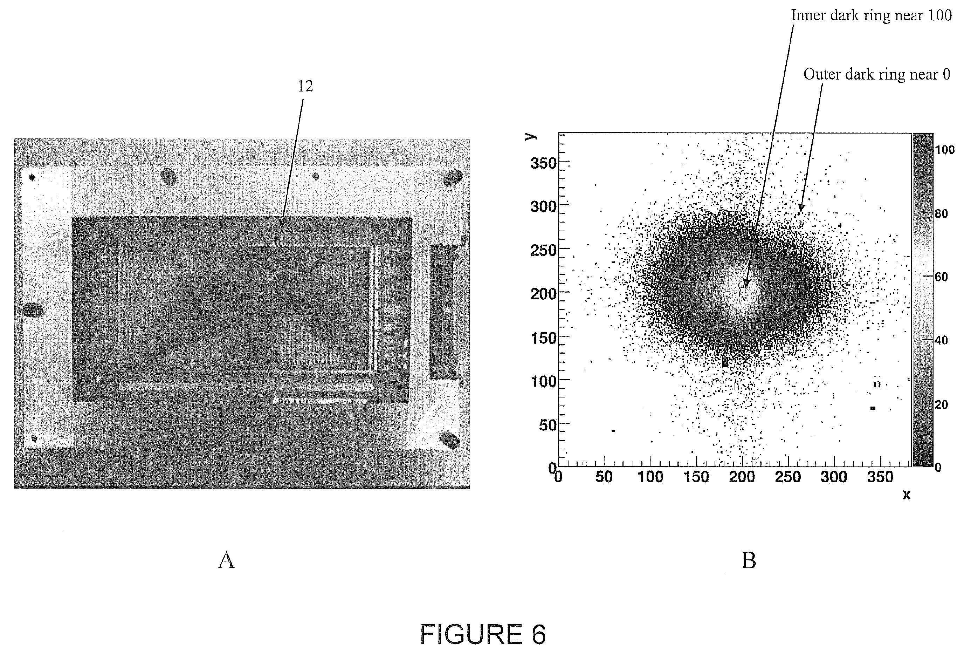

FIGURE 6A shows a side view of the tracking detector 12 of the present invention. In this detector 12, there are two layers of silicon strips. The silicon sensors which connect to the SiPms 20 are 89.5 mm x 89.5 mm with a strip pitch of 238 $\mu$m and 400 $\mu$m thickness. The strips of each layer are individually connected to six ASICs*64 strips. FIGURE 6B shows a view of a proton beam detected by the tracking detector 12 with X and Y coordinates.

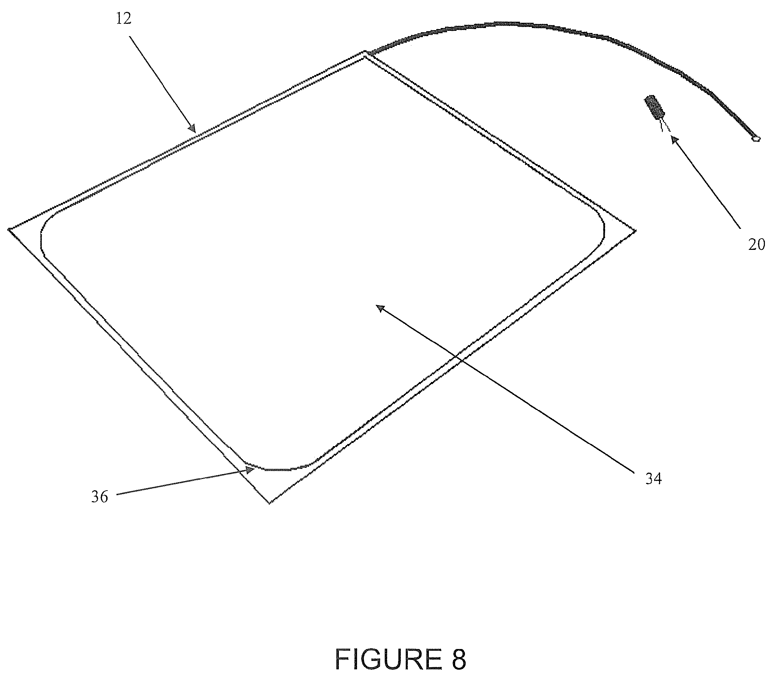

The calorimeter 14 is used to detect proton energy that is emitted from the source 18. SiPM 20 with a diameter of 1.2 mm, can provide a readout (digital or analog) through 1.2 mm wavelength shifter (WLS) fibers 36. The SiPM 20 used in the calorimeter 14 can be the same type as used in the tracking detectors 12. The calorimeter 14 can be arranged in a stack (at least two) of 3 mm scintillator plates 34, as shown in FIGURE 8. In this case, the total number of channels are about 120. This is arrived at by the following. One scintillating plate 34 includes 1 WLS fiber and 1 SiPM. The total number of plates is 120. So, there are 120 plates, 120 WLS fibers, 120 SIPM, and 120 channels of electronics (120 channels). The calorimeter 14 can further include appropriate electronics to relay data to the computer cluster.

In prior art devices (Amaldi, Premier), different combinations of readout systems were used. There has never before been the use of SiPM and scintillator fibers and SiPM and WLS fibers in combination. Amaldi, et al. used SiPM and WLS fibers for the calorimeter readout, but gas photomultipliers were used as a tracking detector. Premier, et al. used vacuum photomultipliers to readout scintillating fibers and vacuum photomultipliers to readout the calorimeter. In both mentioned cases, the targeted system name was a radiograph whereas the present invention is a 3D tomograph.

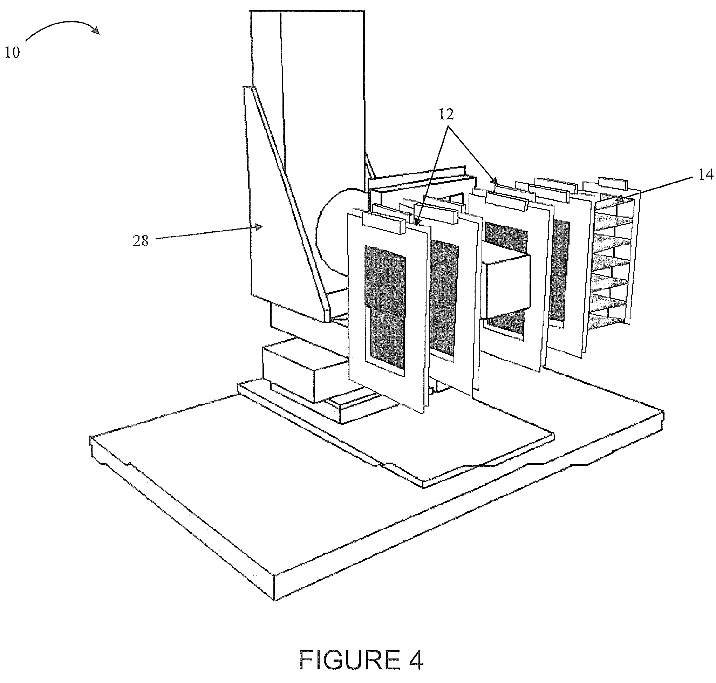

FIGURE 4 shows the system 10 with a rotational stage 28 that holds and rotates an object 16 during proton exposition.

Unique features of this detector include large area coverage without dead zones or overlapping detectors that can produce artifacts in the reconstructed images. This solution offers better signal to noise than a previous approach that uses silicon strip detectors. Thus, scintillation fibers attached to silicon photo multipliers reduce background noise to produce cleaner images and lower dose to the patient for imaging. This is the first pCT detector to record protons at a rate of 2 million per second and reduce imaging time to less than 10 minutes, an acceptable time for patient imaging.

The present invention provides for a method of imaging an object by emitting protons from a source through two tracking detectors, through and around the object, and though two opposite tracking detectors, detecting the protons with a calorimeter, and imaging the object.

More specifically, protons are emitted from a source 18 (scanning magnets) through two tracking detectors 12, through and around the object 16 to be imaged, through additional two tracking detectors 12, and finally pass through the energy detector (i.e., calorimeter 14) (FIGURE 1). Protons of sufficient energy can penetrate the human body and can be tracked upon entry and exit of the tracking detectors 12. The X and Y position of the protons are measured and detected on the X and Y planes of each of the tracking detectors 12. Preferably, the system 10 includes 4 detectors or eight planes of these fiber trackers to record the position of each proton track as it passes through each plane. The object 16 being imaged is located such that two detectors 12 are on each side of the image. The detectors 12 rotate about the object 360 degrees during operation.

Generating 3D proton computed tomography images requires data from a large number of proton histories to be stored in memory. Previous computer programs executed on stand-alone general purpose graphical processing unit (GPGPU) workstations implemented in prior art were constrained by the demand on computer memory. The approach described here is not limited to demands on computer memory in the same manner as the reconstruction is executed on multiple workstations simultaneously.

The resulting data recorded from the detector and the calorimeter can be fed into an image reconstruction software program on a computer cluster to allow high-resolution images to be formed in under 10 minutes after irradiation.

The computer program is written in terms of the Message Passing Interface (MPI) standard. Writing the program in terms of the MPI standard enables one to run the program on a cluster of many computers as opposed to a single computer. The program can be run on approximately 768 CPUs (computers) plus 96 GPUs (graphic processing units). In this way, it is possible to bring to bear the combined memory and computational power of many computers at one time, thus substantially reducing the time it takes to run the program (by orders of magnitude) as well as increasing the problem size (as measured by size of image space and the sharpness of the image) the program can image. Therefore, data acquired from the calorimeter 14 can be analyzed on a cluster of multiple computers with this standard.

A compact way was also developed in which to store in the computer's memory the information needed to generate the image. This compact design reduces the memory required to solve the problem by orders of magnitude. Previous computer programs implemented in the prior art, which executed on stand-alone workstations, were constrained by the amount of computer memory their programs required.

By contrast the compact memory representation enables one to deploy the computer program to solve the entire 3D image space at one time.

This results in images of higher quality as it allows one to use the information from the trace histories of more protons as compared to the "slice and assemble" technique used in the prior art.

The new computer program of the present invention is capable of producing high-quality 3D internal images for cancer patients being treated with proton therapy. Most notably, the new computer program, and specifically the two novel techniques of adding MPI and the compact memory representation, is able to produce quality images faster than any other known methods and, in fact, so fast (i.e., less than 10 minutes) that the program can not only be used to augment or replace X-ray generated images used in proton treatment of cancer today, but medical care providers can find new uses for such images to improve the quality of care delivered to the patient (e.g., just before treating the patient with proton therapy generate one more set of images to refine the treatment plan). pCT imaging and this new computer program can become the state of the art, and thus become the de facto standard, for generating images for every cancer patient globally treated with proton therapy.

The computer program is further described in Example 1 below, and an example of code is shown in EXAMPLE 2. Briefly, a foreman computer distributes equal amounts of proton histories to multiple worker computers and the foreman computer itself. An Integrated Electron Density (IED) and Most Likely Path (MLP) are computed by each of the computers. A solution vector is solved for and stored on computer readable memory in each of the computers. Copies of the solution vector from the worker computers are sent to the foreman computer and combined and stored on computer readable media. The combined solution vector is tested by the foreman computer, and if the combined solution vector is determined to be done, an image is then produced of the object. If it is determined that the combined solution vector is not done, the combined solution vector is transmitted to the worker computers, and each of the above steps are repeated until the combined solution vector is determined to be done by the foreman computer, and an image is produced of the object.

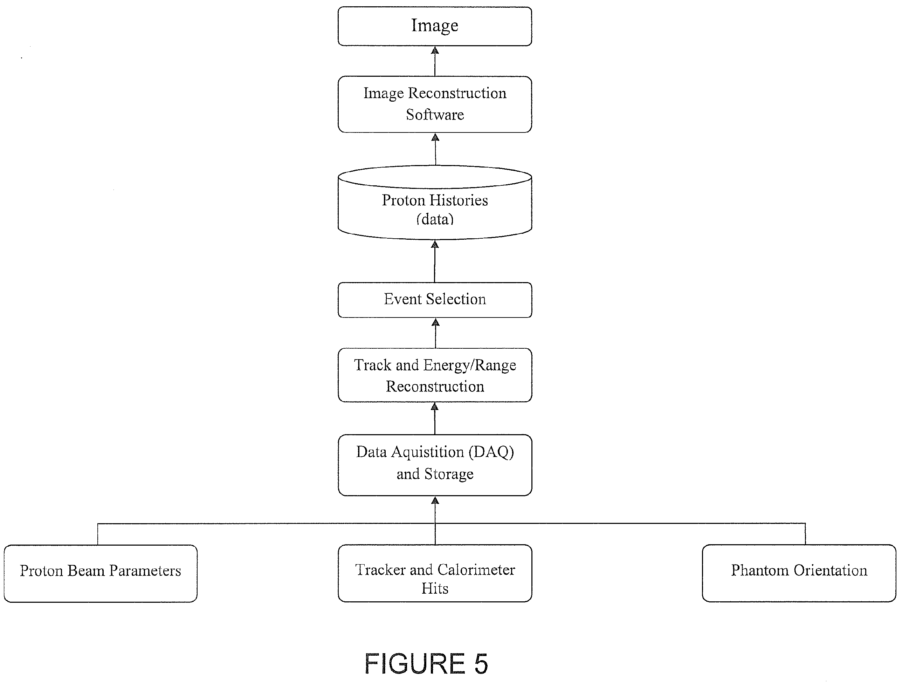

FIGURE 5 shows a flow chart of the general process of the present invention. The proton detection process is shown in the bottom row, and data from the tracking detectors 12 and calorimeter 14 is analyzed from the top using parallel processors and the algorithms described above. Finally, an image is generated, which looks generally like a CT image.

Thus, the advantages of this approach are that it significantly reduces both the time and memory, each by orders of magnitude, required to generate an image. These dramatic reductions in both time and space not only change how the images are produced, but also how they can be used in cancer therapy.

The invention is further described in detail by reference to the following experimental examples. These examples are provided for the purpose of illustration only, and are not intended to be limiting unless otherwise specified. Thus, the invention should in no way be construed as being limited to the following examples, but rather, should be construed to encompass any and all variations which become evident as a result of the teaching provided herein.

EXAMPLE 1

Memory and CPU Count Estimate for Block Methods March 2010

## 1 The Approach

Our proposed solution to implement string averaging methods is to use MPI with set of worker processes where one worker is designated as the foreman. The foreman reads the proton histories and distributes them evenly across all the workers, saving an equal share for himself. From their portion of the histories each worker computes (one time only) IED and MLP and initializes their solution vector which represents the *entire* voxel space (i.e., each worker has its own copy of the entire solution).

The program then enters an iteration loop in which:

1. Each worker uses their IED and MLP to modify their copy of the solution. This is an iterative process with its own stopping criterion.

2. Each worker that is not the foreman sends their copy of the solution to the foreman.

3. The foreman collects and combines with his own the solution vectors from all the other workers and tests combined solution vector. If combined solution is "done", then foreman tells all the other workers to end. If "not done" the foreman broadcasts the combined solution vector to all the other workers who, in turn and along with the foreman, use that as their starting point for their next iteration.

## 2 Input Parameters

We define the following input paramters that characterize the imaging space and the computational resources.

*Plane detector parameters*

$P_h$, $P_w$      height and width (e.g., in cm), respectively, of the plane detectors
$P_r$      resolution of detector strips (e.g., 238 $\mu$)

*Imaging parameters*

$I_a$    number of discrete angles (360°) from which imaging protons are fired

*Voxel space parameters*

$V_h, V_w, V_d$    height, width, and depth (e.g., in cm), respectively, of voxel space
$V_r$        desired resolution of image (e.g., 1 mm)
$V_s$        MLP image resolution oversample rate (e.g., 2)
$V_p$        protons per voxel; number of imaging protons travelling through each voxel

*Computational resource parameters*

$M$    memory per process
$f$    number of bytes to store a single precision float
$p$    number of bytes to store a pointer

**3 Memory Contraint and Process Count**

In this section we compute the number of histories each worker can process, which we denote with $H_w$, as constrained the memory per process $M$, and from $H_w$ and the total number of histories determine the number processes will be required, denoted by $P$, to solve the problem.

**Indices and Counting.** We find a repeated need to compute the minimum number of bytes needed for counting and to also index various discretized spaces. For notational convenience we therefore define these terms

- $V$; the number of voxels in our voxel space,

- $b_v$; the number of bytes needed to index the linearlized voxel space,

- $b_{ph}$ and $b_{pw}$; the number of bytes needed to index dimensions $P_h$ and $P_w$, respectively, and

- $b_{Ia}$; the number of bytes needed to index the discrete firing angles

as follows:

$$V \quad = \quad \left\lceil \frac{V_h V_w V_d}{V_r^3} \right\rceil \qquad b_v \quad = \quad \left\lceil \frac{\lceil log_2 V \rceil}{8} \right\rceil$$

$$b_{ph} \quad = \quad \left\lceil \frac{\lceil log_2 \frac{P_h}{P_r} \rceil}{8} \right\rceil \qquad b_{pw} \quad = \quad \left\lceil \frac{\lceil log_2 \frac{P_w}{P_r} \rceil}{8} \right\rceil \qquad b_{Ia} \quad = \quad \left\lceil \frac{\lceil log_2 I_a \rceil}{8} \right\rceil$$

**Input.** Each proton history is characterized by an 11-tuple comprised of four $< x, y >$ tuples (one for each detector plane), input and output energies ($E_{in}$ and $E_{out}$), and a projection angle. These values require $b_{ph} + b_{pw}$ bytes of storage for each of the four $< x, y >$ tuples plus three floats for the last three values. Each history is processed to determine the 3-D surface of the phantom (i.e., space carving). In this process the voxel space is represented by an array, one element for each voxel, and the elements of the array are set or cleared if the phantom does nor does not occupy the voxel, respectively. We choose initially to represent each voxel with a byte rather than a bit to reduce the time to access the array. Therefore, the memory ($I_m$) required to store the processed input projection histories for each worker is

$$I_m = H_w[4(b_{ph} + b_{pw}) + 3f] + V$$

$$= H_w[4(\lceil \frac{\lceil log_2 \frac{P_h}{P_r}\rceil}{8}\rceil + \lceil \frac{\lceil log_2 \frac{P_w}{P_r}\rceil}{8}\rceil) + 3f] + \lceil \frac{V_h V_w V_d}{V_r^3}\rceil \qquad (1)$$

**IED.** Each proton history is used to compute an Integrated Electron Density (IED), which is stored as a float. The memory needed to store the IEDs ($IED_m$) for each worker is

$$IED_m = H_w f \qquad (2)$$

**MLP.** Each proton history is used to compute the proton's Most Likely Path (MLP) by identifying those voxels through which the proton passed. Assuming near-linear trajectories, the set of voxels "touched" by a single proton's path is very small compared to the total voxel space and so a sparse representation is appropriate. We choose initially to list each voxel in a proton's path by identifying the voxel by its index in the linearized voxel space (i.e., requiring $b_v$ bytes for each index value). Proton history data is generated by firing many imaging protons from each of a number of different angles. Again assuming near-linear trajectories the upper limit of voxels through which a single proton will pass can be reasonably approximated from the length of the diagonal ($l_d$) through the voxel space cube[1] (i.e., $l_d = \sqrt{V_h^2 + V_w^2 + V_d^2}$). Proton histories are evaluated at a resolution (i.e., step size s) that is determined by the desired imaging resolution and the MLP oversampling rate (i.e., $s = \frac{V_r}{V_s}$). In addition to identifying those voxels through which protons pass, it has been shown that the quality of the final image can be improved by also recording an estimate of the proton path's chord length through each voxel (i.e., as opposed to simply identifying those voxels that were "touched"). Initially, we choose to compute an estimate the chord length, a floating point number, as a function of the voxel cube dimensions ($V_h$, $V_w$, $V_d$) and the firing angle. Without assuming anything regarding how the proton histories will be partitioned over the set of worker processes (e.g., a worker may receive all histories from a single firing angle or from many, or all, firing angles) a simple memory layout that "connects" a chord length and the angle that produced it to the voxel indices of the protons fired from that angle is to maintain a vector of chord lengths and two additional vectors in tandem as follows;

- *chord lengths* - This is a vector of floats that records the computed chord length for each discrete firing angle (i.e., length = $I_a$).
- *firing angle index* - This is a vector of integer indices into the first vector of chord lengths that records the angle from which each proton history was fired (i.e., length = $H_w$).
- *voxel index array* - This is a vector of pointers where each proton history points to the vector of voxel indices (estimated length $I_d$) the proton "touched" on its path (i.e., length = $H_w$).

Under these approxmiations and simple memory layout scheme, an upper limit approximation of the memory needed to store the MLP voxels and their chord lengths ($MLP_m$) for each worker is

$$MLP_m = I_a f + H_w\{b_{Ia} + p + (\lfloor \frac{l_d}{s}\rfloor + 1)b_v\}$$

$$= I_a f + H_w\{(\lceil \frac{\lceil log_2 I_a\rceil}{8}\rceil) + p + (\lfloor \frac{\sqrt{V_h^2 + V_w^2 + V_d^2}}{\frac{V_r}{V_s}}\rfloor + 1)(\lceil \frac{\lceil log_2 V\rceil}{8}\rceil)\}$$

$$= I_a f + H_w\{(\lceil \frac{\lceil log_2 I_a\rceil}{8}\rceil) + p$$

$$+ (\lfloor \frac{V_s\sqrt{V_h^2 + V_w^2 + V_d^2}}{V_r}\rfloor + 1)(\lceil \frac{\lceil log_2(\lceil \frac{V_h V_w V_d}{V_r^3}\rceil)\rceil}{8}\rceil)\} \qquad (3)$$

[1]In practice MLP evaluations will start and end with the entry and exit 3-tuples < x, y, z > generated from the space carving pre-processing of the input.

Solution Vector. The solution vector is an array of floats whose length is determined by the number of voxels V. We note that each worker maintains its own copy of the entire solution vector, and so, the memory needed to store the solution vector ($V_m$) in each worker is

$$V_m = Vf = (\lceil \frac{V_h V_w V_d}{V_r^3} \rceil)f \qquad (4)$$

Histories per worker. Each worker, including the foreman, must have enough space to store each of the above memory components, however, we note that once the input has been processed its memory may be re-used for the solution vector.

$$IED_m + MLP_m + MAX(I_m, V_m) \leq M \qquad (5)$$

Re-writing inequality (5) we see that we can compute the maximum number of histories each worker can process, $H_w$, as constrained by their memory $M$ as follows:

$$\underbrace{H_w f}_{IED_m} + I_a f + \underbrace{H_w \{(\lceil \frac{\lceil log_2 I_a \rceil}{8} \rceil) + p + (\lfloor \frac{2\sqrt{V_h^2 + V_w^2 + V_d^2}}{V_r} \rfloor + 1)(\lceil \frac{\lceil log_2(\lceil \frac{V_h V_w V_d}{V_r^3} \rceil) \rceil}{8} \rceil)\}}_{MLP_m}$$

$$+ MAX(\underbrace{H_w [4(\lceil \frac{\lceil log_2 \frac{P_h}{P_r} \rceil}{8} \rceil + \lceil \frac{\lceil log_2 \frac{P_w}{P_r} \rceil}{8} \rceil) + 3f] + \lceil \frac{V_h V_w V_d}{V_r^3} \rceil}_{I_m}, \underbrace{(\lceil \frac{V_h V_w V_d}{V_r^3} \rceil)f}_{V_m}) \leq M \quad (6)$$

Number of processes. The number of voxels V and the number of protons per voxel $V_p$ tells us how many input proton histories there will be in all. That, when combined with maximum number of histories each worker can process as constrained by their memory, $H_w$, tells us how many worker processes we will need for to solve the problem which we denote $P$.

$$P = (\lceil \frac{V_p V}{H_w} \rceil) \qquad (7)$$

## 4 Case Study

We compute here $H_w$ and $P$ using the following values:

$$P_h, P_w = 20\ cm$$

$$P_r = .238\ mm$$

$$I_a = 180$$

$$V_h, V_w, V_d = 20\ cm$$

$$V_r = 1\ mm$$

$$V_s = 2$$

$$V_p = 10$$

$$M = 2\,GB$$

$$f, p = 4\,bytes$$

Substituting those values into inequality (6) we get[2]

$$\underbrace{4H_w}_{IED_m} + 180(4) + \underbrace{H_w\{(\lceil\frac{\lceil log_2 180\rceil}{8}\rceil) + 4 + (\lfloor\frac{2\sqrt{3(20^2)}}{.1}\rfloor + 1)(\lceil\frac{\lceil log_2(\lceil\frac{20^3}{.1^3}\rceil)\rceil}{8}\rceil)\}}_{MLP_m}$$

$$+ MAX(\underbrace{H_w[4(\lceil\frac{\lceil log_2\frac{20}{.0238}\rceil}{8}\rceil + \lceil\frac{\lceil log_2\frac{20}{.0238}\rceil}{8}\rceil) + 3(4)] + \lceil\frac{20^3}{.1^3}\rceil}_{I_m}, \underbrace{(\lceil\frac{20^3}{.1^3}\rceil)4}_{V_m})$$

$$= \underbrace{4H_w}_{IED_m} + \underbrace{720 + H_w\{1 + 4 + (693)(4)\}}_{MLP_m} + MAX(\underbrace{28H_w + 8x10^6}_{I_m}, \underbrace{32x10^6}_{V_m})$$

$$= 2,781H_w + 720 + MAX(28H_w + 8x10^6, 32x10^6) \leq 2\,GB \qquad (8)$$

Case I $28H_w + 8\text{x}10^6 \geq 32\text{x}10^6$: Rewriting inequality (8)

$$2,781H_w + 720 + 28H_w + 8x10^6 \leq 2\,GB$$

$$H_w \leq \frac{2\,GB - 720 - 8x10^6}{2,809}$$

$$H_w \leq 761,652.9$$

Case II $28H_w + 8\text{x}10^6 < 32\text{x}10^6$: Rewriting inequality (8)

$$2,781H_w + 720 + 32x10^6 \leq 2\,GB$$

$$H_w \leq \frac{2\,GB - 720 - 32x10^6}{2,781}$$

$$H_w \leq 760,691.5$$

Since the memory required to store the solution under Case II $V_m = 32\text{x}10^6$ is greater than the amount of memory to store the input $I_m = 28H_w + 8\text{x}10^6 \approx 29\text{x}10^6$ we use the value $H_w = 760,692$ computed under Case II. Applying that value to equation (7) we find that we need

$$P = \lceil\frac{V_p V}{H_w}\rceil$$

$$= \lceil\frac{80,000,000}{760,692}\rceil$$

$$= 106$$

[2]Rounded $(\lceil \frac{\lceil log_2(\lceil \frac{20^3}{.1^3}\rceil)\rceil}{8} \rceil)$ up from 3 to 4 so that value can be stored in int.

Appendix - for spreadsheet

From inequality (6) we solve for $H_w$ under the two following cases:

Case I Solving for $H_w$ in (6) assuming $I_m \geq V_M$:

$$\underbrace{H_w f}_{IED_m} + \underbrace{I_a f + H_w\{(\lceil \frac{\lceil log_2 I_a \rceil}{8}\rceil) + p + (\lfloor \frac{V_s \sqrt{V_h^2 + V_w^2 + V_d^2}}{V_r} \rfloor + 1)(\lceil \frac{\lceil log_2(\lceil \frac{V_h V_w V_d}{V_r^3}\rceil)\rceil}{8}\rceil)\}}_{MLP_m}$$

$$\underbrace{+ H_w[4(\lceil \frac{\lceil log_2 \frac{P_h}{P_r}\rceil}{8}\rceil + \lceil \frac{\lceil log_2 \frac{P_w}{P_r}\rceil}{8}\rceil) + 3f] + \lceil \frac{V_h V_w V_d}{V_r^3}\rceil \leq M}_{I_m}$$

$$H_w[f + \{(\lceil \frac{\lceil log_2 I_a \rceil}{8}\rceil) + p + (\lfloor \frac{V_s \sqrt{V_h^2 + V_w^2 + V_d^2}}{V_r} \rfloor + 1)(\lceil \frac{\lceil log_2(\lceil \frac{V_h V_w V_d}{V_r^3}\rceil)\rceil}{8}\rceil)\}$$
$$+ [4(\lceil \frac{\lceil log_2 \frac{P_h}{P_r}\rceil}{8}\rceil + \lceil \frac{\lceil log_2 \frac{P_w}{P_r}\rceil}{8}\rceil) + 3f]] \leq M - I_a f - \lceil \frac{V_h V_w V_d}{V_r^3}\rceil$$

$$\frac{M - I_a f - \lceil \frac{V_h V_w V_d}{V_r^3}\rceil}{[4f + (\lceil \frac{\lceil log_2 I_a \rceil}{8}\rceil) + p + (\lfloor \frac{V_s \sqrt{V_h^2 + V_w^2 + V_d^2}}{V_r} \rfloor + 1)(\lceil \frac{\lceil log_2(\lceil \frac{V_h V_w V_d}{V_r^3}\rceil)\rceil}{8}\rceil) + 4(\lceil \frac{\lceil log_2 \frac{P_h}{P_r}\rceil}{8}\rceil + \lceil \frac{\lceil log_2 \frac{P_w}{P_r}\rceil}{8}\rceil)]} > H_w$$

or expressed using more convenient notation

$$\frac{M - I_a f - V}{4f + b_{Ia} + p + (\lfloor \frac{V_s(l_d)}{V_r} \rfloor + 1)b_v + 4(b_{ph} + b_{pw})} > H_w \qquad (9)$$

Substituting the parameters from section 4 we compute $H_w$ as follows:

$$\frac{2\, GB - (180)(4) - \lceil \frac{20^3}{.1^3}\rceil}{[4(4) + (\lceil \frac{\lceil log_2 180\rceil}{8}\rceil) + 4 + (\lfloor \frac{2\sqrt{3(20^2)}}{.1} \rfloor + 1)(\lceil \frac{\lceil log_2(\lceil \frac{(20)^3}{.1^3}\rceil)\rceil}{8}\rceil) + 4(\lceil \frac{\lceil log_2 \frac{20}{.0238}\rceil}{8}\rceil + \lceil \frac{\lceil log_2 \frac{20}{.0238}\rceil}{8}\rceil)]} > H_w$$

$$\frac{2,139,482,928}{[16 + (1) + 4 + (692 + 1)(4) + 4(2 + 2)]} > H_w$$

$$761,652.9 > H_w$$

Case II Solving for $H_w$ in (6) assuming $I_m < V_M$:

$$\underbrace{H_w f}_{IED_m} + I_a f + \underbrace{H_w \{(\lceil \frac{\lceil log_2 I_a \rceil}{8} \rceil) + p + (\lfloor \frac{V_s\sqrt{V_h^2 + V_w^2 + V_d^2}}{V_r} \rfloor + 1)(\lceil \frac{\lceil log_2(\lceil \frac{V_h V_w V_d}{V_r^3} \rceil) \rceil}{8} \rceil)\}}_{MLP_m}$$

$$+ \underbrace{(\lceil \frac{V_h V_w V_d}{V_r^3} \rceil)f}_{V_m} \leq M$$

$$H_w \{ f + (\lceil \frac{\lceil log_2 I_a \rceil}{8} \rceil) + p + (\lfloor \frac{V_s\sqrt{V_h^2 + V_w^2 + V_d^2}}{V_r} \rfloor + 1)(\lceil \frac{\lceil log_2(\lceil \frac{V_h V_w V_d}{V_r^3} \rceil) \rceil}{8} \rceil)\}$$

$$\leq M - I_a f - (\lceil \frac{V_h V_w V_d}{V_r^3} \rceil)f$$

$$\frac{M - \{I_a + (\lceil \frac{V_h V_w V_d}{V_r^3} \rceil)\}f}{f + (\lceil \frac{\lceil log_2 I_a \rceil}{8} \rceil) + p + (\lfloor \frac{V_s\sqrt{V_h^2 + V_w^2 + V_d^2}}{V_r} \rfloor + 1)(\lceil \frac{\lceil log_2(\lceil \frac{V_h V_w V_d}{V_r^3} \rceil) \rceil}{8} \rceil)} > H_w$$

or expressed using more convenient notation

$$\frac{M - (I_a + V)f}{f + b_{Ia} + p + (\lfloor \frac{V_s(l_d)}{V_r} \rfloor + 1)b_v} > H_w \tag{10}$$

Substituting the parameters from section 4 into (10) we again compute $H_w$ as follows[3]

$$\frac{2\ GB - \{180 + (\lceil \frac{20^3}{.13} \rceil)\}4}{4 + (\lceil \frac{\lceil log_2 180 \rceil}{8} \rceil) + 4 + (\lfloor \frac{2\sqrt{3(20^2)}}{.1} \rfloor + 1)(\lceil \frac{\lceil log_2(\lceil \frac{20^3}{.13} \rceil) \rceil}{8} \rceil)} > H_w$$

$$\frac{2,115,482,928}{4 + (1) + 4 + (693)(4)} > H_w$$

$$760,691.5 > H_w$$

[3]Rounded $(\lceil \frac{\lceil log_2(\lceil \frac{20^3}{.13} \rceil) \rceil}{8} \rceil)$ up from 3 to 4 so that value can be stored in int.

EXAMPLE 2

```
#include <stdio.h>
#include <stdlib.h>
#include <string.h>
#include <mpi.h>
const int RandomizedRange = 10000;
/* command line configurables */
int Randomized; /* -r <nelem> */
int N;
int parse_command_line_args(int argc, char **argv, int my_id)
{
int i;
```

```
int error;
/* default values */
Randomized = 0;
N = 0;
for (i = 1, error = 0; !error && i < argc; i ++)
{
if (!strcmp(argv[i], "-r"))
{
  Randomized = 1;
  if (i + 1 < argc && (N = atoi(argv[i+1])) > 0)
      i ++;
  else
      error = 1;
      }
      else
  error = 1;
} /* endfor */
if (error && !my_id)
{
/* only Master prints usage message */
fprintf(stderr, "\n\tusage: %s {-r <nelem>}\n\n", argv [0]);
fprintf(stderr, "where\n\n");
fprintf(stderr,
"\t-r <nelem>\t- Randomized input with N=nelem\n\n");
} /* endif */
return error;
} /* end parse_command_line_args() */
struct stats
{
float mean;
float sse;
unsigned int n;
}; /* end struct stats */
/*
```

"Note on a Method for Calculating Corrected Sums of Squares and Products," B.P. Welford, Technometrics, Vol 4, No. 3, Aug 1962, 419-420.

The Art of Computer Programming, D.E. Knuth, vol 2, 2nd Edition, p 216

The Art of Computer Programming, D.E. Knuth, vol 2, 3rd Edition, p 232

Reccurrence forumla that keeps a running mean (M_k) and running SSE (S_k) for some stream of values X_i For x_1

$$M\_1 = x\_1$$

$$S\_1 = 0$$

For x_i i>1

$$M\_i = M\_{(i-1)} + [(x\_i - M\_{(i-1)})/i]$$

$$S\_i = S\_{(i-1)} + [(x\_i - M\_{(i-1)}) * (x\_i - M\_i)]$$

At any time k>1

running mean = M_k
running_sample_ (i.e., not _population_) variance = S_k/(k-1)

"Updating Formulae and a Pairwise Algorithm for Computer Sample Variances," T.F. Chan, G.H. Golub, and R.J. LeVeque,Technical Report STAN-CS-79-773, Department of Computer Science, Stanford University, 1979,ftp://reports.stanford.edu/pub/cstr/reports/cs/tr/79/773/CS-TR-79-773.pdf

Given two sample sets where the mean and SSE have been computed for each; <M_k,1 S_k,1 N_1> and <M_k,2 S_k,2 N_2> we can compute the combined mean and SSE as follows

```
                          N = N_1 + N_2


                          d = M_k,2 - M_k,1


              combined mean = M_k,1 + d*N_2/N


          combined SSE  = S_k,1 + S_k,2 + d^2*N_1*N_2/N

*/
static void push(float *m, float *s, unsigned int *n, float x)
{
   *n = *n + 1;
   if (*n == 1)
{
   *m = x;
   *s = 0.0;
}
else
{
   float old_mean = *m;
   float d = x - old_mean;
   *m += (d/(*n));
   *s += (d*(x - *m));
} /* endif */
/* printf("after pushing %f RunningMean %f RunningSSE %f\n", x, *m, *s); */
return;
} /* end push() */
void combine_stats(void *operand_vec,
          void *operand_result_vec,
          int *len,
          MPI_Datatype *dtype)
          {
          struct stats *op = (struct stats *) operand_vec;
          struct stats *op_res = (struct stats *) operand_result_vec;
int i;
unsigned int combined_n;
float delta;
for (i = 0; i < *len; i ++)
{
   combined_n = op[i].n + op_res[i].n;
   delta = op[i].mean - op_res[i].mean;
   op_res[i].mean += (delta * op[i].n / combined_n);
   op_res[i].sse += (op[i].sse +
             (delta * delta * op[i].n * op_res[i].n / combined_n));
   op_res[i].n = combined_n;
} /* endfor */
return;
} /* end combine_stats() */
#if 0
void combine_stats (void *operand_vec,
```

```
                void *operand_result_vec,
                int *len,
                MPI_Datatype *dtype)
{
    struct stats *op = (struct stats *) operand_vec;
    struct stats *op_res = (struct stats *) operand_result_vec;
    int i;
     for (i = 0; i < *len; i ++)
      {
            op_res[i].mean += op[i].mean;
            op_res[i].sse += op[i].sse;
            op_res[i].n += op[i].n;
      } /* endfor */
      return;
} /* end combine_stats() */
#endif
static float SampleData[ ] = {17.0, 19.0, 24.0};
int main(int argc, char **argv)
{
    int numprocs, my_id;
    int i;
    struct stats s[2];
    MPI_Op op;
    float *src_data;
    /* for derived struct */
     MPI_Datatype Stats_mpi;
     int block_lengths[3]; /* number of "things" in each element of */
                    /* our new struct. for us, 1 each */
     MPI_Aint displs[3]; /* displacement of each element */
     MPI_Datatype types[3]; /* type of each element */
     MPI_Aint start_addr;
     MPI_Aint addr;
     MPI_Init(&argc, &argv);
     MPI_Comm_size(MPI_COMM_WORLD, &numprocs);
     MPI_Comm_rank(MPI_COMM_WORLD, &my_id);
     printf ("hello, world: my_id %d numprocs %d\n", my_id, numprocs);
if (parse_command_line_args (argc, argv, my_id))
{
    MPI_Finalize();
     exit(1);
} /* endif */
if (Randomized)
{
   if ((src_data = malloc (N*sizeof(float))) == NULL)
    {
      fprintf(stderr, "ERROR: failed malloc %n bytes\n", N*sizeof(float));
     exit (1) ;
   } /* endif */
   for (i = 0; i < N; i ++)
    {
       /* about 1/2 positive and 1/2 negative */
       src_data[i] = (random () % RandomizedRange) - (RandomizedRange/2);
       printf("my_id %d: sample_data %f\n", my_id, src_data[i]);
    }
}
else
{
   src_data = SampleData;
   N = sizeof(SampleData)/sizeof(float);
} /* endif */
/***********************************/
/* compute local running mean and SSE */
/***********************************/
```

```
s[0].n = s[1].n = 0;
for (i = 0; i < N; i ++)
{
    /* just push the same sample data into both 'regions' of s[] */
    push(&(s[0].mean), &(s[0].sse), &(s[0].n), src_data[i]);
    push(&(s[1].mean), &(s[1].sse), &(s[1].n), src_data[i]);
} /* endfor */
/***************************************/
/* compute global running mean and SSE */
/***************************************/
block_lengths[0] = block_lengths[1] = block_lengths[2] = 1;
types[0] = MPI_FLOAT;
types[1] = MPI_FLOAT;
types[2] = MPI_UNSIGNED;
MPI_Address(&(s[0].mean), &start_addr);
displs[0] = 0;
MPI_Address(&(s[0].sse), &addr);
displs[1] = addr - start_addr;
MPI_Address(&(s[0].n), &addr);
displs[2] = addr - start_addr;
/* build new derived datatype */
MPI_Type_struct(3, /* nelements */
            block_lengths,
            displs,
            types,
            &Stats_mpi) ;
MPI_Type_commit(&Stats_mpi);
MPI_Op_create((MPI_User_function*) combine_stats, // my user-defined func
            1,                                     // commute flag
            &op);                                  // op handle
MPI_Allreduce(s, // operand
        s, // result
        2, // count
        Stats_mpi, // data type
        op, // operator
        MPI_COMM_WORLD);
MPI_Op_free(&op);
printf ("myid %d: after s[0].mean %f s[0].sse %f s[0].n %d\n", my_id, s[0].mean, s[
0].sse, s[0].n);
printf ("myid %d: after s[1].mean %f s[1].sse %f s[1].n %d\n", my_id, s[1].mean, s[
1].sse, s[1].n);
if (my_id == 0)
{
    /* computing mean and SSE the old-fashioned way for checksums */
    float sum = 0.0;
    float sse = 0.0;
    float mean;
    for (i = 0; i < N; i ++)
        sum += (src_data[i] * numprocs);
    mean = sum/(N*numprocs);
    for (i = 0; i < N; i ++)
        sse += ((src_data[i] - mean)*(src_data[i] - mean)*numprocs);
printf("myid %d: 2-pass mean %f sse %f n %d\n",
    my_id, mean, sse, N*numprocs);
} /* endif */
if (Randomized)
{
    free(src_data);
} /* endif */
MPI_Finalize();
  exit(0);
} /* end main() */
#ifndef PCT_IED_H
```

```
#define PCT_IED_H
#include "pct.h" // struct entry_exit
#if defined(_cplusplus)
extern "C" {
#endif
   void compute_IED(int nhistories, float *input_floats, float *IED);
   void adjust_IED(int nhistories,
         unsigned char * history_bitmask,
         unsigned short *input_ushorts,
         float *input_floats,
         float * IED,
         struct entry_exit_t *entry_exit);
#if defined(_cplusplus)
}
#endif
#endif /* PCT_IED_H */
#ifndef _mlp_m_
#define _mlp_m_
/* here we use 'unsigned int's to store voxel indices */
class MLP_m
{
    public:
        MLP_m(int my_nh);
        -MLP_m();
        /********************/
        /* proton histories */
        /********************/
        void set_my_nhistories_for_MLP(int mnh_for_MLP)
            { my_nhistories_for_MLP = mnh_for_MLP; }
        void add_voxel_idx_to_history(int h_idx, unsigned int v_idx);
        /* iterators */
         unsigned int *get_first_voxel_idx_from_history(int h_idx);
         unsigned int *get_next_voxel_idx_from_history(int h_idx,
                                   unsigned int *last_voxel_idx_p);
         /* faster iterator - assumes voxel idxs for a given history */
         /* stored in a contiguous mem block */
         unsigned int get_history_idx_to_ntouched_voxels_idxs(int h_idx);
        /*****************/
        /* chord lengths */
        /*****************/
        float get_chord_length(int h_idx);
        void register_firing_angle(int h_idx, float theta);
         /**************************/
         /* memory management stats */
         /**************************/
         int get_curr_mem_block_idx() { return curr_mem_block_idx; }
         int get_n_mem_blocks() { return n_mem_blocks; }
         unsigned long get_n_recorded_voxel_idxs()
            { return n_recorded_voxel_idxs; }
         int get_my_nhistories_for_MLP() { return my_nhistories_for_MLP; }
         unsigned long get_n_copies() { return n_copies; }
         unsigned long get_n_copied_bytes() { return n_copied_bytes; }
         unsigned long get_nbytes_allocd_for_voxel_idxs();
         int get_n_allocd_blocks() { return curr_mem_block_idx+1; }
         unsigned int get_block_nbytes(int b_idx);
    private:
      /********************/
      /* proton histories */
      /********************/
      /* my_nhistories = as many as I was given from input */
      /* my_nhistories_for_MLP = number of histories remaining after */
                          /* throwing out as many as we know did */
                          /* not touch the phantom ... used only */
```

```
                                  /* for initial big block malloc */
        int my_nhistories;
        int my_nhistories_for_MLP;
        unsigned long n_recorded_voxel_idxs; // for estimating subsequent
                                             // big block malloc's
        unsigned int **history_idx_to_touched_voxels_idxs; // len=my_nhistories
        unsigned int *history_idx_to_ntouched_voxels_idxs; // len=my_nhistories
        int n_mem_blocks;
        unsigned int **mem_block_pointers; // len = n_mem_blocks
        unsigned int *mem_block_nidx_lengths; // len = n_mem_blocks
        /* slot for next voxel idx */
        int curr_mem_block_idx;
        unsigned int *next_voxel_idx_p;
        /*****************/
        /* chord lengths */
        /*****************/
        /* int n_firing_angles; */
        float *chord_lengths;                      // len = N_FIRING_ANGLES
        int *history_idx_to_chord_length_idx_map; // len = my_nhistories
         void compute_chord_lengths();
         /**************************/
         /* memory management stats */
         /**************************/
         unsigned long n_copies;
         unsigned long n_copied_bytes;
     };
     #endif
     #ifndef PCT_PCT_H
     #define PCT_PCT_H


     #ifdef _MAIN_
     #define EXTERN
     #else
     #define EXTERN extern
     #endif


     #include <iostream>
     #include <cmath>
     #include <math.h>
     #include <sstream>
     #include <vector>
     #include <iomanip>
     #include <fstream>
     #include <string>
     #include <cstdio>
     #include <cstdlib>
     #include <assert.h>
     #include <stdio.h>
     #include <stdlib.h>
     #include <string.h>
     #include <unistd.h>

     #include <time.h>
     #include <sys/time.h>

     #ifndef NO_MPI
     #include <mpi.h>
     #endif


     #include "mlp_m.h"

 using namespace std;
```

```
/*****************/
/* Problem Space */
/*****************/


/*** START DEFINING THE SENSORS ***/
// Data space definitions
// Sensor definitions - distances in CM, origin in center of volume
const float TRACK_Z1 = -30.0; // sensor plane closest to proton gun
const float TRACK_Z2 = -20.0; // 2nd sensor plane from gun
const float TRACK_Z3 = 20.0; // 3rd sensor plane from gun
const float TRACK_Z4 = 30.0; // 4th and last sensor plane from gun
const float TRACK_Z1_X = TRACK_Z1 +0.1; // Slightly different distances for
const float TRACK_Z1_Y = TRACK_Z1 - 0.1; // x and y sensor planes
const float TRACK_Z2_X = TRACK_Z2 + 0.1;
const float TRACK_Z2_Y = TRACK_Z2 - 0.1;
const float TRACK_Z3_X = TRACK_Z3 - 0.1;
const float TRACK_Z3_Y = TRACK_Z3 + 0.1;
const float TRACK_Z4_X = TRACK_Z4 - 0.1;
const float TRACK_Z4_Y = TRACK_Z4 + 0.1;


// X-axis is HORIZONAL as you stand behind the gun facing it shooting direction
// X-axis range values in CM
const float MIN_TRACK_X = -15.00;
const float MAX_TRACK_X = 15.00;
const int MAX_TRACK_X_IDX = 3000;


// Y-axis is VERTICAL as you stand behind the gun facing it shooting direction
// Y-axis range values in CM
const float MIN_TRACK_Y = -15.00;
const float MAX_TRACK_Y = 15.00;
const int MAX_TRACK_Y_IDX = 3000;


const float RANGE_TRACK_X = MAX_TRACK_X - MIN_TRACK_X;
const float RANGE_TRACK_Y = MAX_TRACK_Y - MIN_TRACK_Y;


// sensor resolution in CM
const float TRACK_DX = (RANGE_TRACK_X/MAX_TRACK_X_IDX);
const float TRACK_DY = (RANGE_TRACK_Y/MAX_TRACK_Y_IDX);


/*** END DEFINING THE SENSORS ***/

/*** START DEFINING VOXEL SPACE BETWEEN SENSORS ***/
/* The data reconstruction space has two coordinate systems. */

/* The first is measured in centimeters and has the same XYZ orientation */
/* as the sensor grid at beam rotation angle of 0. The origin is the at */
/* the center of the space between the sensor planes. X is positive to */
/* the right (as seen from the central origin). Y is positive up. Z is */
/* positive in the direction of the beam. */

/* The second coordinate system is based on a plane-major, row major ordering */
/* scheme with planes parallel to the beam rotation plane. The column axis */

/* is parallel to X. Row axis is parallel to Z. Plane axis is parallel to Y. */

/* The reconstruction volume is an array a[MAX_PLANE] [MAX_ROW] [MAX_COL] */

/* Minimum index values are always 0. Maximum values depend on the */
/* reconstruction bounds and the reconstruction resolution. */
// The desired bounds on the reconstruction volume may not be exactly
// what is used, due to sampling constraints.
// In code, use VOLUME_{MIN,MAX}_{X,Y,Z}. See below.
const float DESIRED_VOLUME_MIN_X = -14.00;
```

```
const float DESIRED_VOLUME_MAX_X = 14.00;
const float DESIRED_VOLUME_MIN_Z = -14.00;
const float DESIRED_VOLUME_MAX_Z = 14.00;
  // The height
const float DESIRED_VOLUME_MIN_Y = -7.00;
const float DESIRED_VOLUME_MAX_Y = 10.00;


// Voxel sampling sizes in cm
const float D_COL = 0.1;
const float D_ROW = 0.1;
const float D_PLANE = 0.1;
const float D_DATA[3] = {D_COL, D_ROW, D_PLANE} ;


// NICK: these (later) need to be determined by specified distances and
// NICK: specified resolutions.
/* const int MAX_ROWS = 300; // Should be even */
/* const int MAX_COLS = 300; */
/* const int MAX_PLANES = 300; */
const int MAX_COLS =
  (int) ((DESIRED_VOLUME_MAX_X - DESIRED_VOLUME_MIN_X) / D_COL);
const int MAX_ROWS =
  (int) ((DESIRED_VOLUME_MAX_Z - DESIRED_VOLUME_MIN_Z) / D_ROW);
const int MAX_PLANES =
  (int) ((DESIRED_VOLUME_MAX_Y - DESIRED_VOLUME_MIN_Y) / D_PLANE);


const int MAX_DATA[3] = {MAX_COLS, MAX_ROWS, MAX_PLANES};


const float VOLUME_MIN_X = DESIRED_VOLUME_MIN_X;
const float VOLUME_MAX_X = VOLUME_MIN_X + MAX_COLS * D_COL;
const float VOLUME_MIN_Y = DESIRED_VOLUME_MIN_Y;
const float VOLUME_MAX_Y = VOLUME_MIN_Y + MAX_PLANES * D_PLANE;
const float VOLUME_MIN_Z = DESIRED_VOLUME_MIN_Z;
const float VOLUME_MAX_Z = VOLUME_MIN_Z + MAX_ROWS * D_ROW;


// NICK: VOXEL_CUBE_DIAG_VOXEL_LEN and NVOXELS (later) need to be corrected
// NICK: when MAX_{ROWS, COLS, PLANES} are convereted from nvoxels (which is
// NICK: what they are now) to cm ... VOXEL_CUBE_DIAG_VOXEL_LEN and
// NICK: must both be measured in nvoxels
const float VOXEL_CUBE_DIAG_VOXEL_LEN = sqrt ((MAX_ROWS*MAX_ROWS)
                                  + (MAX_COLS*MAX_COLS)
                                  + (MAX_PLANES*MAX_PLANES));
const int NVOXELS = MAX_COLS*MAX_ROWS*MAX_PLANES;
/*** END DEFINING VOXEL SPACE BETWEEN SENSORS ***/


// for space carving and entry-exit, cut-off between "hit" or "miss" phantom
const float ENERGY_THRESHOLD = 190.0;
const float ENERGY_DIFF_THRESHOLD = 2.7;


const float ELECTRON_DENSITY_WATER = 1.0;
const float ELECTRON_DENSITY_AIR = 0.001;
const float INITIAL_SOLUTION_INSIDE_PHANTOM = ELECTRON_DENSITY_WATER;
const float INITIAL_SOLUTION_OUTSIDE_PHANTOM = ELECTRON_DENSITY_AIR;
// for MLP
// constants in nature
const float RADIATION_LENGTH_WATER = 36.1; //cm
// not used const float RADIATION_LENGTH_AIR = 28818; //units?
// distance in CM between planes on one side of phantom
const float SSD_SEPARATION =        (TRACK_Z2-TRACK_Z1);
// distance in CM between innermost planes -
const float SEPARATION =            (TRACK_Z3-TRACK_Z2);
// no longer used const float DIMENSION = 256;
// something in CM
// no longer used const float RECON_SIZE = 21;
```

```
// no longer used const float PIXEL_SIZE = RECON_SIZE/DIMENSION;
// in cm
const float STEP_LENGTH = .05; //originally -> 0.5 * PIXEL_SIZE;
                                      const int NUM_STEPS = (int) (SEPARATION/STEP_LENGTH) ;
const int N_FIRING_ANGLES = 180; // should be factor or multiple of 360
const float THETA_IDX_PER_DEGREE = (((float) N_FIRING_ANGLES)/360.0);
inline int degrees_theta_to_idx(float theta)
   { return ((int) (theta*THETA_IDX_PER_DEGREE + 0.5)); }
inline float idx_to_degrees_theta(int idx)
   { return (((float) idx)/THETA_IDX_PER_DEGREE); }
/***********************/
/* For Linear Solvers */
/***********************/
const float DEFAULT_RELAXATION = 0.3;
/*************************/
/* For Memory Management */
/*************************/
const int MLP_MEMBLOCK_PTRS_PER_ALLOC = 4;
// const float PORTION_HISTORIES_FOR_MLP = 0.95; // estimate portion
                                               // of histories remaining
                                               // _after_ throwing out
                                               // histories we know
                                               // don't touch phantom
const float PORTION_HISTORIES_FOR_MLP = 0.60;
/* const float PORTION_VOXEL_CUBE_DIAG_FOR_MLP = 0.60; */
/* const float PORTION_VOXEL_CUBE_DIAG_FOR_MLP = 0.40; */
const float PORTION_VOXEL_CUBE_DIAG_FOR_MLP = 0.30;
/* for 38M, setting PORTION_VOXEL_CUBE_DIAG_FOR_MLP = 0.20, */
/* results in initial alloc .2GB when we need .3GB */
/* const float PORTION_VOXEL_CUBE_DIAG_FOR_MLP = 0.20; */
EXTERN char Nowstring [100] ;
EXTERN char Relaxparamstring[100];
/* mem mgmt counters */
EXTERN unsigned long N_mallocs;
EXTERN unsigned long N_reallocs;
EXTERN unsigned long N_allocated_bytes;
EXTERN unsigned long Largest_single_mem_request;
EXTERN char Largest_single_mem_request_fname[500];
EXTERN int Largest_single_mem_request_line_no;
#ifdef DEBUG
EXTERN unsigned long N_frees;
EXTERN unsigned long N_freed_bytes;
EXTERN unsigned long N_allocated_bytes_high_water_mark;
EXTERN unsigned long N_outstanding_allocated_bytes;
struct alloc_entry
{
void *p;
unsigned long len;
};
const int N_ALLOC_ENTRIES = 1000;
EXTERN struct alloc_entry Allocs[N_ALLOC_ENTRIES];
EXTERN int Next_alloc_idx;
#endif
/* theoretically possible (but very unlikely) that the first alloc'd */
/* block is not big enough to hold all the voxel idxs touched by the */
/* the first history alone. */
/* in that case we need to REALLOC the first block (not malloc another */
/* because all the voxel idx's from a single history must reside in a */
/* single allocated block (i.e., they cannot span multiple blocks). */
/* we could change that 'no span' design decision if we want to, but */
/* that will at least require us to change the MLP::get_{first,next} */
/* interators used by the linear solvers _and_ (worst yet) add another */
/* vector len=my_nhistories of idxs that would index mem_block_pointers[ ] */
```

```
/* to record the 'starting' block for the iterators. */
/* we also use this value to pad the estimate for remaining block allocs. */
/* ... in short, set this value relatively low (i.e., -10%). */
/* const float PERCENT_PAD_SUBSEQUENT_BLOCK_ALLOCS = 0.10; */
const float PERCENT_PAD_SUBSEQUENT_BLOCK_ALLOCS = 0.20;
/* computed from previous settings */
const int AVERAGE_TOUCHED_VOXELS_PER_HISTORY_EST =
(int) (PORTION_VOXEL_CUBE_DIAG_FOR_MLP_VOXEL_CUBE_DIAG_VOXEL_LEN);
/**************************/
/* Other Useful Constants */
/**************************/
const int DEFAULT_MAXHISTORIES_PER_WORKER          = 1000000;
const int UNSPECIFIED_NINPUT_HISTORIES             = -1;
const int UNSPECIFIED_MAX_NINPUT_HISTORIES_PER_FILE = -1;
/**************/
/* MPI Stuff */
/**************/
const int ForemanRank = 0;
EXTERN int Myid, Nprocs;
/* MPI_Abort codes */
const int ABORT_INVALID_CMD_LINE_ARGS = 1;
const int ABORT_FAILED_MALLOC = 2;
const int ABORT_FAILED_FOPEN = 3;
const int ABORT_TOO_MANY_HISTORIES = 4;
const int ABORT_INSUFFICIENT_INPUT = 5;
const int ABORT_INVALID_IDX = 6;
const int ABORT_INVALID_PTR = 7;
const int ABORT_INVALID_ARG = 8;
const int ABORT_TRACE_OUTSIDE_VOLUME = 9;
const int ABORT_INCONSISTENT_INTERNAL_STATE = 10;
const int ABORT_STATS_ERROR = 11;
/* MPI Message tags */
const int INPUT_FLOAT_TAG = 1;
const int INPUT_USHORT_TAG = 2;
/********************/
/* Input Data Layout */
/********************/
/* Describing input_ushorts */
const int X1_DISPL = 0;
const int Y1_DISPL = 1;
const int X2_DISPL = 2;
const int Y2_DISPL = 3;
const int X3_DISPL = 4;
const int Y3_DISPL = 5;
const int X4_DISPL = 6;
const int Y4_DISPL = 7;
const int INPUT_USHORT_STRIDE = 8;
/* Describing input_floats */
const int EIN_DISPL = 0;
const int EOUT_DISPL = 1;
const int THETA_DISPL = 2;
const int INPUT_FLOAT_STRIDE = 3;
/*****************************/
/* Utility Functions and Macros */
/*****************************/
// A 3-D array in C/C++ defined a[NPLANES][NROWS][NCOLS] is stored in memory
// with changes in the NPLANES dimension having the largest stride
// and changes in the NCOLS dimension with the smallest stride.
inline int convert_3D_voxel_to_idx(int p_idx, int r_idx, int c_idx)
{
    return ((p_idx * MAX_ROWS * MAX_COLS)
        + (r_idx * MAX_COLS)
        + c_idx);
```

```
  } /* end convert_3D_voxel_to_idx() */
  inline void convert_sensor_to_voxel(float dst[3],
                                      float src[3],
                                      float cos_theta,
                                      float sin_theta)
{
  // Input is in cm.
  // Convert to world coordinates
  // Assuming clockwise rotation about y axis as seen from positive y
  // Correct assumption?
  /*
  dst[0] = src[0] * cos_theta - src[2] * sin_theta;
  dst[1] = src[0] * sin_theta + src[2] * cos_theta;
  dst[2] = src[1];
  */
  // No. Rotation is counterclockwise about y axis as seen from positive y
  dst[0] = src[0] * cos_theta + src[2] * sin_theta;
  dst[1] = -src[0] * sin_theta + src[2] * cos_theta;
  dst[2] = src[1];
  // Translate to data origin and scale to data space.
  dst[0] = (dst[0] - VOLUME_MIN_X) / D_COL;
  dst[1] = (dst[1] - VOLUME_MIN_Z) / D_ROW;
  dst[2] = (dst[2] - VOLUME_MIN_Y) / D_PLANE;
  return;
} // end convert_sensor_to_voxel()
/* input_ushort accessors */
inline unsigned short get_x1(unsigned short *v, int idx)
  { return v[(idx*INPUT_USHORT_STRIDE) + X1_DISPL]; }
inline unsigned short get_y1(unsigned short *v, int idx)
  { return v[(idx*INPUT_USHORT_STRIDE) + Y1_DISPL]; }
inline unsigned short get_x2(unsigned short *v, int idx)
  { return v[(idx*INPUT_USHORT_STRIDE) + X2_DISPL]; }
inline unsigned short get_y2(unsigned short *v, int idx)
  { return v[(idx*INPUT_USHORT_STRIDE) + Y2_DISPL]; }
inline unsigned short get_x3(unsigned short *v, int idx)
  { return v[(idx*INPUT_USHORT_STRIDE) + X3_DISPL]; }
inline unsigned short get_y3(unsigned short *v, int idx)
  { return v[(idx*INPUT_USHORT_STRIDE) + Y3_DISPL]; }
inline unsigned short get_x4(unsigned short *v, int idx)
  { return v[(idx*INPUT_USHORT_STRIDE) + X4_DISPL]; }
inline unsigned short get_y4(unsigned short *v, int idx)
  { return v[(idx*INPUT_USHORT_STRIDE) + Y4_DISPL]; }
/* input_floats accessors */
inline float get_ein(float *v, int idx)
  { return v[(idx*INPUT_FLOAT_STRIDE) + EIN_DISPL]; }
inline float get_eout(float *v, int idx)
  { return v[(idx*INPUT_FLOAT_STRIDE) + EOUT_DISPL]; }
inline float get_theta(float *v, int idx)
  { return v[(idx*INPUT_FLOAT_STRIDE) + THETA_DISPL]; }
/* Abort stuff */
#ifdef NO_MPI
#define PCT_ABORT(CODE) assert(0);
#else
#define PCT_ABORT(CODE) MPI_Abort(MPI_COMM_WORLD, (CODE));
#endif
/* Bitmask stuff */
#define CLEAR(v, b) (v)[(b)/8] &= (-(0x1 << (7-((b)%8))) & (0xff))
#define SET(v, b) (v)[(b)/8] |= (0x1 << (7-((b)%8)))
#define TEST(v, b) ((v)[(b)/8] & (0x1 << (7-((b)%8))))


/* Memory Management */

/* these two I want kept a #define so that _LINE_ and FILE_resolve */
```

```
/* to the place in the source code where the potential error occurred */
#ifdef NO_MPI
#ifdef DEBUG
/* NO_MPI DEBUG */
/* must use assert(0) instead of MPI_Abort() */
#define PCT_MALLOC(P, S, T)                     \
{ \
  if ((S) <= 0)                                 \
  { \
      fprintf(stderr,                           \
   "       ERROR: %s %d: proc %d: "             \
          "malloc called for %ld bytes\n",      \
          _FILE_, _LINE_, Myid,                 \
          (long) (S));                          \
      assert(0);                                \
  }                                             \
  if (!((P) = (T *) malloc((S))))               \
  { \
      fprintf(stderr,                           \
          "ERROR: %s %d: proc %d: "             \
          "failed malloc %ld bytes\n",          \
          _FILE_, _LINE_, Myid,                 \
          (long) (S));                          \
      assert(0);                                \
  }                                             \
  N_mallocs ++;                                 \
  N_allocated_bytes += (S);                     \
  if (Next_alloc_idx == N_ALLOC_ENTRIES)        \
  { \
      fprintf(stderr,                           \
          "ERROR: %s %d: proc %d: "             \
          "Next_alloc_idx %d (max %d)\n",       \
          _FILE_, LINE_, Myid,                  \
          Next_alloc_idx,                       \
          N_ALLOC_ENTRIES-1);                   \
     assert(0);                                 \
  }                                             \
  Allocs[Next_alloc_idx].p (P);                 \
  Allocs[Next_alloc_idx].len = (S);             \
  Next_alloc_idx ++;                            \
  N_outstanding_allocated_bytes += (S);         \
  if (N_outstanding_allocated_bytes             \
    > N_allocated_bytes_high_water_mark)        \
  { \
    N_allocated_bytes_high_water_mark =      \
        N_outstanding_allocated_bytes;     \
  }                                             \
  if ((unsigned long) (S)                       \
      > Largest_single_mem_request)             \
  { \
    Largest_single_mem_request = (S);        \
    strcpy(Largest_single_mem_request_fname, _FILE_); \
     Largest_single_mem_request_line_no = _LINE_;     \
  }                                             \
}
#else
/* NO_MPI !DEBUG */
#define PCT_MALLOC(P, S, T)                     \
{ \
  if (!((P) = (T *) malloc((S))))               \
  { \
      fprintf(stderr,                           \
          "ERROR: %s %d: proc %d: "             \
```

```
                "failed malloc %ld bytes\n",     \
                _FILE_, _LINE_, Myid,            \
                (long) (S));                     \
        assert(0);                               \
    }                                            \
    N_mallocs ++;                                \
    N_allocated_bytes += (S);                    \
    if ((unsigned long) (S)                      \
        > Largest_single_mem_request)            \
    { \
        Largest_single_mem_request = (S);        \
        strcpy(Largest_single_mem_request_fname, _FILE_); \
        Largest_single_mem_request_line_no = _LINE_;      \
    }                                            \
}
#endif
#else
#ifdef DEBUG
/* !NO_MPI DEBUG */
/* can use MPI_Abort() */
#define PCT_MALLOC(P, S, T)                      \
{ \
  if ((S) <= 0)                                  \
  { \
        fprintf(stderr,                          \
            "ERROR: %s %d: proc %d: "            \
            "malloc called for %ld bytes\n",     \
            _FILE_, _LINE_, Myid,                \
            (long) (S));                         \
        MPI_Abort(MPI_COMM_WORLD, ABORT_INVALID_ARG); \
  }                                              \
  if (!((P) = (T *) malloc((S))))                \
  { \
     fprintf(stderr,                             \
        "ERROR: %s %d: proc %d: "                \
        "failed malloc %ld bytes\n",             \
        _FILE_, _LINE_, Myid,                    \
        (long) (S));                             \
     MPI_Abort(MPI_COMM_MORLD, ABORT_INVALID_IDX); \
  }                                              \
  N_mallocs ++;                                  \
  N_allocated_bytes += (S);                      \
  if (Next_alloc_idx == N_ALLOC_ENTRIES)         \
  { \
        fprintf(stderr,                          \
          "ERROR: %s %d: proc %d: "              \
          "Next_alloc_idx %d (max %d)\n",        \
          _FILE_, _LINE_, Myid,                  \
          Next_alloc_idx,                        \
          N_ALLOC_ENTRIES-1);                    \
        MPI_Abort(MPI_COMM_WORLD, ABORT_INVALID_IDX); \
  }                                              \
  Allocs[Next_alloc_idx].p = (P);                \
  Allocs[Next_alloc_idx].len = (S);              \
  Next_alloc_idx ++;                             \
  N_outstanding_allocated_bytes += (S);          \
  if (N_outstanding_allocated_bytes              \
     > N_allocated_bytes_high_water_mark)        \
  { \
        N_allocated_bytes_high_water_mark =      \
            N_outstanding_allocated_bytes;       \
  }                                              \
  if ((unsigned long) (S)                        \
```

```
                > Largest_single_mem_request)              \
        { \
            Largest_single_mem_request = (S);          \
            strcpy(Largest_single_mem_request_fname, _FILE_); \
            Largest_single_mem_request_line_no = _LINE_; \
        }                                              \
    }
    #else
    /* !NO_MPI !DEBUG */
    #define PCT_MALLOC(P, S, T) \
    { \
      if (!((P) = (T *) malloc((S))))              \
      { \
            fprintf(stderr,                          \
              "ERROR: %s %d: proc %d: "              \
              "failed malloc %ld bytes\n",           \
              _FILE_, _LINE_, Myid,                  \
              (long) (S));                           \
            MPI_Abort(MPI_COMM_WORLD, ABORT_FAILED_MALLOC); \
      }                                              \
      N_mallocs ++;                                  \
      N_allocated_bytes += (S);                      \
      if ((unsigned long) (S) \
          > Largest_single_mem_request) \
      { \
          Largest_single_mem_request = (S); \
          strcpy(Largest_single_mem_request_fname, _FILE_); \
          Largest_single_mem_request_line_no = _LINE_; \
      } \
    }
    #endif
    #endif
    #ifdef NO_MPI
    #ifdef DEBUG
    /* NO_MPI DEBUG */
    /* must use assert(0) instead of MPI_Abort() */
    #define PCT_REALLOC(P, S, T)                         \
    { \
      int aidx;                                          \
      if ((S) <= 0)                                      \
      { \
          fprintf(stderr,                                \
            "ERROR: %s %d: proc %d: "                    \
            "realloc called for %ld bytes\n",            \
            _FILE_, _LINE_, Myid,                        \
            (long) (S));                                 \
          assert(0);                                     \
      }                                                  \
      for (aidx = 0;                                     \
           aidx < Next_alloc_idx                         \
           && Allocs[aidx].p != (P);                     \
           aidx ++)                                      \
      ;                                                  \
      if (aidx == Next_alloc_idx)                        \
      { \
          fprintf(stderr,                                \
            "WARNING: %s %d: proc %d: "                  \
            "free() could not find addr %p\n",           \
            _FILE_, _LINE_, Myid,                        \
            (void *) (P));                               \
      }                                                  \
      N_outstanding_allocated_bytes -= Allocs[aidx].len; \
      N_freed_bytes += Allocs[aidx].len;                 \
```

```
    N_frees ++;                                                \
    if (!((P) = (T *) realloc((P), (S))))          \
    { \
        fprintf(stderr,                                        \
          "ERROR: %s %d: proc %d: "                  \
          "failed realloc %ld bytes\n",              \
          _FILE_, _LINE_, Myid,                      \
          (long) (S));                               \
        assert(0);                                   \
    }                                                \
    Allocs[aidx].p = (P);                            \
    Allocs[aidx].len = (S);                          \
    N_reallocs ++;                                   \
    N_allocated_bytes += (S);                        \
    N_outstanding_allocated_bytes += (S);            \
    if (N_outstanding_allocated_bytes               \
        > N_allocated_bytes_high_water_mark        ) \
    { \
        N_allocated_bytes_high_water_mark =          \
          N_outstanding_allocated_bytes;             \
    }                                                \
    if ((unsigned long) (S) \
        > Largest_single_mem_request) \
    { \
        Largest_single_mem_request = (S); \
        strcpy(Largest_single_mem_request_fname, _FILE_); \
        Largest_single_mem_request_line_no = _LINE_; \
    } \
}
#else
/* NO_MPI !DEBUG */
#define PCT_REALLOC(P, S, T) \
{ \
    if (!((P) = (T *) realloc((P), (S)))) \
    { \
        fprintf(stderr,                            \
                "ERROR: %s %d: proc %d: "          \
                "failed realloc %ld bytes\n",      \
                __FILE_, _LINE_, Myid,             \
                (long) (S));                       \
        assert(0);                                 \
    }                                              \
    N_reallocs ++;                                 \
    N_allocated_bytes += (S);                      \
    if ((unsigned long) (S) \
        > Largest_single_mem_request) \
    { \
        Largest_single_mem_request = (S); \
        strcpy(Largest_single_mem_request_fname, _FILE_); \
        Largest_single_mem_request_line_no = _LINE_; \
    } \
}
#endif
#else
#ifdef DEBUG
/* !NO_MPI DEBUG */
/* can use MPI_Abort() */
#define PCT_REALLOC(P, S, T)                               \
{ \
    int aidx;                                              \
    if ((S) <= 0)                                          \
    { \
        fprintf(stderr,                                    \
```

```
        "ERROR: %s %d: proc %d: "                      \
        "realloc called for %ld bytes\n",              \
        _FILE_, _LINE_, Myid,                          \
        (long) (S));                                   \
    MPI_Abort(MPI_COMM_WORLD, ABORT_INVALID_ARG);      \
  }                                                    \
  for (aidx = 0;                                       \
    aidx < Next_alloc_idx                              \
    && Allocs[aidx].p != (P);                          \
        aidx ++)                                       \
  ;                                                    \
  if (aidx == Next_alloc_idx)                          \
  { \
    fprintf(stderr,                                    \
      "WARNING: %s %d: proc %d: "                      \
      "free() could not find addr %p\n",               \
      _FILE_, _LINE_, Myid,                            \
      (void *) (P));                                   \
  }                                                    \
  N_outstanding_allocated_bytes -= Allocs[aidx].len;   \
  N_freed_bytes += Allocs[aidx].len;                   \
  N_frees ++;                                          \
  if (!((P) = (T *) realloc((P), (S))))                \
  { \
    fprintf(stderr,                                    \
      "ERROR: %s %d: proc %d: "                        \
      "failed realloc %ld bytes\n",                    \
      _FILE_, _LINE_, Myid,                            \
      (long) (S));                                     \
    MPI_Abort(MPI_COMM_WORLD, ABORT_FAILED_MALLOC);    \
  }                                                    \
  Allocs[aidx].p = (P);                                \
  Allocs[aidx].len = (S);                              \
  N_reallocs ++;                                       \
  N_allocated_bytes += (S);                            \
  N_outstanding_allocated_bytes += (S);                \
    if (N_outstanding_allocated_bytes                  \
      > N_allocated_bytes_high_water_mark)             \
    { \
      N_allocated_bytes_high_water_mark =              \
       N_outstanding_allocated_bytes;                  \
    } \
    if ((unsigned long) (S) \
        > Largest_single_mem_request) \
    { \
      Largest_single_mem_request = (S); \
      strcpy(Largest_single_mem_request_fname, _FILE_); \
      Largest_single_mem_request_line_no = _LINE_; \
    } \
  }
  #else
  /* !NO_MPI !DEBUG */
  #define PCT_REALLOC(P, S, T)                         \
  { \
    if (!((P) = (T *) realloc((P), (S))))              \
    { \
      fprintf(stderr,                                  \
        "ERROR: %s %d: proc %d: "                      \
        "failed realloc %ld bytes\n",                  \
        _FILE_, _LINE_, Myid,                          \
        (long) (S));                                   \
      MPI_Abort(MPI_COMM_WORLD, ABORT_FAILED_MALLOC); \
    }                                                  \
```

```
      N_reallocs ++;                                                  \
      N_allocated_bytes += (S);                                       \
      if ((unsigned long) (S) \
          > Largest_single_mem_request) \
      { \
          Largest_single_mem_request = (S); \
          strcpy(Largest_single_mem_request_fname, _FILE_); \
          Largest_single_mem_request_line_no = _LINE_; \
      } \
}
#endif
#endif
#ifdef DEBUG
#define PCT_FREE(P                                            ) \
{ \
    int aidx;                                                        \
    for (aidx = 0;                                                   \
       aidx < Next_alloc_idx                                         \
    &   & Allocs[aidx].p != (P);                                     \
         aidx ++)                                                    \
    ;                                                                \
    if (aidx == Next_alloc_idx)                                      \
    { \
        fprintf(stderr,                                              \
          "WARNING: %s %d: proc %d: "                                \
          "free() could not find addr %p\n",                         \
          _FILE_, _LINE_, Myid,                                      \
          (void *) (P));                                             \
    }                                                                \
    N_outstanding_allocated_bytes -= Allocs[aidx].len;    \
    N_freed_bytes                 += Allocs[aidx].len;    \
    Allocs[aidx].p = NULL;                                           \
    N_frees ++;                                                      \
    free((P));                                                       \
}
#else
#define PCT_FREE(P) free((P));
    #endif
    /*******************/
    /* Data Structures */
    /******************/ /
    // For space carving
    // A high energy trace
    #define HIGH_TRACE (1<<0)
    // A low energy trace
    #define LOW_TRACE (1<<1)
    // All energy levels turned on
    #define ALL_TRACE (HIGH_TRACE | LOW_TRACE)
    struct entry_exit_t
    {
       float entry_x, entry_y, entry_z, exit_x, exit_y, exit_z;
    };
    struct stats
    {
       float mean;
       float sse;
       unsigned int n;
    #ifdef DEBUG
       /* for sanity checking only, not needed for stats */
       float min, max;
    #endif
    };
    /* partitioning internal exit detector plane into regions */
```

29

```
const int REGION_ROWS = 30;
const int REGION_COLS = 30;
const int STAT_DELTA_X = 0;
const int STAT_DELTA_Y = 1;
const int STAT_DELTA_ENERGY = 2;
const int STAT_LAST_UNUSED = 3;
const int STAT_NSTATS = STAT_LAST_UNUSED;
/* linearized layout - from fastest changing idx -> slowest changing idx */
/* x,y,energy {0,1,2} -> REGION_COLS -> REGION_ROWS -> N_FIRING_ANGLES */
const int N_STAT_CELLS          = (STAT_NSTATS
                                  * REGION_ROWS * REGION_COLS
                                  * N_FIRING_ANGLES);
const int STAT_CELL_IDX_ANGLE_STRIDE = (STAT_NSTATS
                                      * REGION_ROWS * REGION_COLS);
const int STAT_CELL_IDX_ROW_STRIDE = (STAT_NSTATS * REGION_COLS);
const int STAT_CELL_IDX_COL_STRIDE = STAT_NSTATS;
const float MAX_SIGMA = 3.0;
/* Voxel level debugging */
/* This is to help isolate calculations that */
/* work with a specific voxel in the data space. */
/* The particular voxel index to be examined. The constants below
refer to the plane, row, and column number respectively. */
#ifdef DEBUG_VOXEL
#define DEBUG_VOXEL_IDX (225 * (MAX_ROWS*MAX_COLS) + 201 * MAX_COLS + 42)
#endif
#ifndef NO_MPI
/* MPI Profiling */
/* MPI functions we profile */
const int MPI_INIT_IDX = 0;
const int MPI_COMM_SIZE_IDX = 1;
const int MPI_COMM_RANK_IDX = 2;
const int MPI_ADDRESS_IDX = 3;
const int MPI_TYPE_STRUCT_IDX = 4;
const int MPI_TYPE_COMMIT_IDX = 5;
const int MPI_OP_CREATE_IDX = 6;
const int MPI_OP_FREE_IDX = 7;
/* put msg passing ones at end for pretty report */
const int MPI_ALLREDUCE_IDX = 8;
const int MPI_BCAST_IDX = 9;
const int MPI_SEND_IDX = 10;
const int MPI_RECV_IDX = 11;
const int MPI_LASTUNUSED_IDX = 12;
const int Nfuncs = MPI_LASTUNUSED_IDX;
struct func_profile
{
  char func_name[50];
  unsigned call_count;
  double total_time_secs;
  int does_communicate;
  unsigned long total_bytes;
}; /* end struct func_profile */
EXTERN struct func_profile func_profiles[Nfuncs];
#endif
/* Input/Output data location */
/* PADS */
/* const char InputDirBase[] = "/home/karonis/38M/Input"; */
const char InputDirBase[] = "/gpfs/pads/projects/CI-CCR000048/karonis/Input";
/* const char InputDirBase[] = "/home/karonis/pCT/"; */
/* const char OutputDirBase[] = "/home/karonis/38M/Output"; */
const char OutputDirBase[] = "/gpfs/pads/projects/CI-CCR000048/karonis";
/* const char OutputDirBase[] = "/autonfs/home/karonis/pCT/Code/"; **/
/* Mac */
 /* const char InputDirBase[] = "/Volumes/Software/pCT/"; */
```

```
/* const char OutputDirBase[] = "/Volumes/Software/pCT/Code/"; */
/* laptop */
/* const char InputDirBase[] = "/Users/karonis/pCT/"; */
/* const char OutputDirBase[] = "/Users/karonis/pCT/Code/"; */
/* hopper.cs.niu.edu */
/* const char InputDirBase[] = "/home/hopper/research/pct/data/SimProjections/Input"; *
/
/* const char OutputDirBase[] = "/home/turing/karonis/pCT/Code/"; */
#endif /* PCT_PCT_H */
#define _MAIN_
#include "pct.h"
/*************************/
/* LOCAL GLOBAL VARIABLES */
/*************************/
/* for linear solver loop */
const int Niters = 10;
static int Iterations = 0;
static char InputDir[256], InputFnamePrefix[50], InputFnameSuffix[50];
static unsigned TotalHistories; // relevant for foreman only
const float MB = 1024.0*1024.0;
const float GB = MB*1024.0;
/* Timer Indexes and count */
const int SetupTimeIdx = 0;
const int IEDTimeIdx = 1;
const int VolumeBitmaskTimeIdx = 2;
const int TouchedPhantomTimeIdx = 3;
const int BinnedCleanupIdx = 4;
const int AdjustIEDIdx = 5;
onst int MLPTimeIdx = 6;
const int LinearSolverTimeIdx = 7;
const int LastUnusedIdx = 8;
const int Ntimers = LastUnusedIdx;
suseconds_t Timers[Ntimers] = {0};
unsigned long StartNhistories[Ntimers];
const char *TimerNames[Ntimers] =
    {
            "Set up",
            "IED",
            "Volume Bitmask",
             "Connect to Phantom",
              "Binned cleanup",
              "Adjust IED",
               "MLP",
                "Linear Solver"
        };
/*****************/ /
/* LOCAL FUNCTIONS */
/*****************/
static void accumulate_stats_val(float *m,
                                 float *s,
#ifdef DEBUG
                                 float *min,
                                 float *max,
#endif
                                 unsigned int *n,
                                 float val);
static void compute_and_bcast_vbm(int my_nhistories,
                                  unsigned char *history_bitmask,
                                  float *input_floats,
                                  unsigned short *input_ushorts,
                                  int volume_bitmask_len,
                                  char *volume_bitmask);
static void compute_nhistories(int *my_nhistories,
```

```
                                            int *histories_per_worker, // relevant foreman only
                                            int n_input_histories,
                                            int max_histories_per_foreman or_worker,
                                            int *must_fill_foreman);
        static float compute_stat_val(float *input_floats,
                                            unsigned short *input_ushorts,
                                            int h,
                                            int stat_displ);
        static int count_histories_in_bitmask(int my_nhistories,
                                            unsigned char *history_bitmask);
        static void fill_buffers_from_files(float *input_floats,
                                            unsigned short *input ushorts,
                                            int *deg,
                                            FILE **fp,
                                            int *histories_read_from_this_file,
                                             int max_histories_per_input_file,
                                            int nhistories_buffsize,
                                            int *my_nhistories,
                                            int must_fill);
         static void find_entry_exit_from_vbm_or_remove (int nhistories,
                                            unsigned char *history_bitmask,
                                            float *input_floats,
                                            unsigned short *input_ushorts,
                                            struct entry_exit_t *entry_exit,
                                            int volume_bitmask_len,
                                            char *volume_bitmask);
        static int get_stat_cell_idx(float *input_floats,
                                            unsigned short *input_ushorts,
                                            int h,
                                            int stat_displ);
        static FILE *open_input_file(int degree);
        static void parse_command_line_args(int argc,
                                            char *argv[],
                                            int *n_input_histories,
                                            int *max_histories_per_foreman or_worker,
                                            int *max_histories-per_input_file,
                                            float *linear_solver_relaxation);
        static void read_and_distribute_input(int max_histories_per_foreman_or_worker,
                                            int max_histories_per_input_file,
                                            int *my_nhistories,
                                            int must_fill_foreman,
                                            float *input_floats,
                                            unsigned short *input_ushorts);
                                            static void remove_based_on_binned_stat(int nhistories,
                                            unsigned char *history_bitmask,
                                            float *input_floats,
                                            unsigned short *input_ushorts,
                                            struct stats s[],
                                            float sigma_cutoff[],
                                            int stat_displ);
        static void remove_histories_from_binned_stats(int my_nhistories,
                                            unsigned char *history_bitmask,
                                            float *input_floats,
                                            unsigned short *input_ushorts);
        static int test_solution_for_done(float *solution);
        static inline void record_start_nhistories(unsigned long nhistories,
                                                const int idx,
                                                unsigned long my_nhistories)
        {
            StartNhistories[idx] = nhistories;
            printf("%d: %18s: starting nhistories %lid - %% my N histories %5.1f %%\n",
                Myid, TimerNames[idx], StartNhistories[idx],
                 (my_nhistories
```

```
                    ? ((float) nhistories)*100.0/((float) my_nhistories)
                    : 0.0 ));
    } /* end record_starting_nhistories() */
    // assumes after.tv_sec >= before.tv_sec
    //
    // there are only two cases ...
    // Case I: after.tv_usec >= before.tv_usec (no matter how many secs passed)
    // elapsed usecs = (after.tv_sec - before.tv_sec)*1,000,000
    // + (after.tv_usec - before.tv_usec)
    //
    // Case II: after.tv_usec < before.tv_usec (in this case after.tv_sec must be
    // greater than before.tv_sec)
    // elapsed usecs = (after.tv_sec - before.tv_sec)*1,000,000
    // - (before.tv_usec - after.tv_usec)
    // = (after.tv_sec - before.tv_sec)*1,000,000
    // + after.tv_usec - before.tv usec
    //
    // BOTH CASES ARE THE SAME
    static inline void record_time(struct timeval &before,
                        struct timeval &after,
                        const int idx)
    {
      Timers[idx] = (after.tv_sec - before.tv-sec)*1000000
            + after.tv_usec - before.tv_usec;
    } /* end record_time() */
    #ifndef NO_MPI
    /* need to write this because MPI_LAND in */
    /* MPI_Reduce is _not_ defined for MPI_CHAR */
    /* must be externally visable for MPI */
    void andem(void *operand_vec,
          void *operand_result_vec,
          int *len,
          MPI_Datatype *dtype)
    {
          char *op = (char *) operand_vec;
          char *op_res = (char *) operand_result_vec;
          int i;
        for (i = 0; i < *len; i ++)
           op_res[i] &= op[i];
          return;
    } /* end andem() */
    /* operator for MPI_Allreduce() */
    /*
    "Updating Formulae and a Pairwise Algorithm for Computer Sample Variances,"
    T.F. Chan, G.H. Golub, and R.J. LeVeque,
    Technical Report STAN-CS-79-773, Department of Computer Science,
    Stanford University, 1979,
    ftp://reports.stanford.edu/pub/cstr/reports/cs/tr/79/773/CS-TR-79-773.pdf
    Given two sample sets where the mean and SSE have been computed
    for each; <M_k,1 S_k,1 N_1> and <M_k,2 S_k,2 N_2>
    we can compute the combined mean and SSE as follows
    N = N_1 + N_2
    d = M_k,2 - M_k,1
    combined mean = M_k,1
+ d*N_2/N
    combined SSE = S_k,1 + S_k,2 + d^2*N-1*N_2/N
    */
    void combine_stats(void *operand_vec,
          void *operand_result_vec,
          int *len,
          MPI_Datatype *dtype)
    {
          struct stats *op = (struct stats *) operand_vec;
```

```
          struct stats *op_res = (struct stats *) operand_result_vec;
          int i;
          unsigned int combined_n;
          float delta;
          for (i = 0; i < *len; i ++)
          {
             if (op[i].n > 0 && op_res[i].n > 0)
             {
                  combined_n = op[i].n + op_res[i].n;
      delta = op[i].mean - op_res[i].mean;
      op_res[i].mean += (delta * op[i].n / combined_n);
      op_res[i].sse += (op[i].sse +
                  (delta * delta * op[i].n * op_res[i].n / combined_n));
      op_res[i].n = combined_n;
#ifdef DEBUG
      if (op_res[i].min > op[i].min) op_res[i].min = op[i].min;
      if (op_res[i].max < op[i].max) op_res[i].max = op[i].max;
#endif
    }
    else if (op[i].n > 0 && op_res[i].n == 0)
    {
      op_res[i].n = op[i].n;
      op_res[i].mean = op[i].mean;
      op_res[i].sse = op[i].sse;
#ifdef DEBUG
      op_res[i].min = op[i].min;
      op_res[i].max = op[i].max;
#endif
    } /* endif */
  } /* endfor */
  return;
} /* end combine_stats() */
#endif
/*********************/ /
/* EXTERNAL FUNCTIONS */
/*********************/ /
/* IED func */
//extern void compute_IED(int my_nhistories, float *input_floats, float *IED);
#include "ied.h"
// intersect2.cc
extern bool compute_entry_exit_from_vbm(int h,
        float *input_floats,
                            unsigned short *input_ushorts,
#ifdef DEBUG
                            int volume_bitmask_len,
#endif
                            char *volume_bitmask,
                            struct entry_exit_t *entry_exit);
extern void compute_initial_vbm(int my_nhistories,
                      unsigned char *history_bitmask,
                      float *input_floats,
                      unsigned short *input_ushorts,
                      int volume_bitmask_len,
                      char *volume_bitmask);
extern void morph_clean(int volume_bitmask_len, char *volume_bitmask);
/* MLP func */
extern void compute_mlp(int my_nhistories,
             unsigned char *history_bitmask,
              float *input_floats,
             unsigned short *input_ushorts,
              struct entry_exit_t *entry_exit,
             int volume_bitmask_len,
             char *volume_bitmask,
```

```
                MLP_m *mlp);
/*******************/ /
/* Linear solvers */
/*******************/ /
/* string averaging methods */
extern void carp(MLP_m *, float x[], float b[],
        int count[], unsigned char history_bitmask[],
        int n_rows, int n_cols, float relax);
extern void sap(MLP_m *, float x[], float b[],
        int count[], unsigned char history_bitmask[],
        int n_rows, int n_cols, float relax);
typedef void (* linear_solver_func ) (MLP_m *,
                                    float x[],
                                    float b[],
                                    int count[],
                                    unsigned char history_bitmask[],
                                    int n_rows,
                                    int n_cols,
                                    float relax);
const int CARP_IDX = 0;
const int SAP_IDX = 1;
linear_solver_func linear_solvers[] = {carp, sap};
int main(int argc, char *argv[])
{
    int i;
    float *input_floats;
    unsigned short *input_ushorts;
    int my_nhistories;
    int n_input_histories, max_histories_per_foreman_or_worker;
    int histories_per_worker; // relevant for foreman only
    int max_histories_per_input_file; // relevant for foreman only
    unsigned long linear_solver_iterations = 0; // relevant for foreman only
#ifdef DEBUG
    unsigned long nvoxels_inside_phantom = 0; // for foreman only
    unsigned long nvoxels_outside_phantom = 0; // for foreman only
    unsigned long nvoxels_inside_phantom_mlp_touched = 0; // for foreman only
    unsigned long nvoxels_outside_phantom_mlp_touched = 0; // for foreman only
    unsigned long nvoxels_inside_phantom_not_touched = 0; // for foreman only
    unsigned long nvoxels_outside_phantom_not_touched = 0; // for foreman only
#endif
    unsigned long start_n_hist;
    int must_fill_foreman;
    /* int volume_bitmask_len = MAX_ROWS*MAX_COLS*MAX_PLANES; */
    int volume_bitmask_len = NVOXELS;
    char *volume_bitmask;
    unsigned char *history_bitmask;
    int history_bitmask_len;
    float *IED;
    struct entry_exit_t *entry_exit;
    MLP_m *mlp;
    float linear_solver_relaxation;
    float *solution;
    int *count;
    int linear_solver_idx = CARP_IDX;
    struct timeval before, after;
    time_t total_time_start, total_time_secs;
    total_time_start = time(NULL); // Mark the start time
#ifdef NO_MPI
    Nprocs = 1;
    Myid = 0;
#else
    MPI_Init(&argc, &argv);
    MPI_Comm_size(MPI_COMM_WORLD, &Nprocs);
```

```
        MPI_Comm_rank(MPI_COMM_WORLD, &Myid);
#endif
        /**********/
        /* Set up */
        /**********/
        record_start_nhistories(0, SetupTimeIdx, 0);
        gettimeofday(&before, NULL);
        {
          N_mallocs = N_reallocs = N_allocated_bytes
                = Largest_single_mem_request = 0;
#ifdef DEBUG
          N_frees = Next_alloc_idx = N_freed_bytes =
          N_outstanding_allocated_bytes = N_allocated_bytes_high_water_mark = 0;
#endif
          if (Myid == ForemanRank) printf("%d: nprocs %d\n", Myid, Nprocs);
           if (Myid == ForemanRank)
           {
              /* recording current time to embed in output fnames */
              time_t now = time(NULL);
              struct tm *tp = localtime(&now);
              sprintf(Nowstring, "%4d-%02d-%02d_%02d:%02d:%02d",
                     1900+tp->tm_year,
                      1+tp->tm_mon,
                      tp->tm_mday,
                      1+tp->tm_hour,
                       1+tp->tm_min,
                       1+tp->tm_sec);
          } /* endif */
#ifndef NO_MPI
        MPI_Bcast(&Nowstring, // buff
         100, // count
        MPI_CHAR, // data type
        ForemanRank, // root
        MPI_COMM_WORLD);
#endif
        parse_command_line_args(argc,
                        argv,
                        &n_input_histories,
                        &max_histories_per_foreman_or_worker,
                        &max_histories_per_input_file,
                        &linear_solver_relaxation);
        sprintf(Relaxparamstring, "relaxparam-%4.2f", linear_solver_relaxation);
        compute_nhistories(&my_nhistories,
                     &histories_per_worker,
                     n_input_histories,
                     max_histories_per_foreman_or_worker,
                     &must_fill_foreman);
         /* at this point: */
         /* - for all work procs: my_nhistories == histories_per_worker */
         /* - for foreman: my_nhistories >= histories_per_worker */
                  mlp = new MLP_m(my_nhistories);
/* fprintf(stderr, "%d: after new MLP_m::MLP_m(): curr_mem_block_idx %d n_mem_blocks %d
\n", Myid, mlp->get_curr_mem_block_idx(), mlp->get_n_mem_blocks()); */
        if (Myid == ForemanRank)
           printf("%d: Input: %s/%s*%s\n",
       Myid, InputDir, InputFnamePrefix, InputFnameSuffix);
PCT_MALLOC(input_floats,
        my_nhistories
            *INPUT_FLOAT_STRIDE
            *sizeof(float),
        float)
PCT_MALLOC(input_ushorts,
        my_nhistories
```

```
                       *INPUT_USHORT_STRIDE
                       *sizeof(unsigned short),
               unsigned short)
     read_and_distribute_input(histories_per_worker,
                                max_histories_per_input_file,
                                &my_nhistories,
                                must_fill_foreman,
                                input_floats,
                                input_ushorts);
     if (Myid == ForemanRank)
          printf("%d: my_nhistories %d\n", Myid, my_nhistories);
     history_bitmask_len = (my_nhistories/8) + (my_nhistories % 8 ? 1 : 0);
     PCT_MALLOC(history__bitmask, history_bitmask_len, unsigned char)
     /* initially claim all histories are used in MLP */
     memset(history_bitmask, 0xff, history_bitmask_len);
}
gettimeofday(&after, NULL);
record_time(before, after, SetupTimeIdx);
/* fprintf(stderr, "%d: after setup: curr_mem_block_idx %d n_mem_blocks %d\n", Myid, ml
p->get_curr_mem_block_idx(), mlp->get_n__mem_blocks()); */
   /***********************************/
   /* gentlemen, start your engines .. */
   /* ... computing starts HERE */
   /***********************************/
   /*********************/
   /* IED - 'b' in Ax=b */
   /*********************/
   /* compute IED for _all_ histories */
   record_start_nhistories(my_nhistories, IEDTimeIdx, my_nhistories);
   gettimeofday(&before, NULL);
   /* if (0) */
   {
        PCT_MALLOC(IED, my_nhistories*sizeof(float), float)
        compute_IED(my_nhistories, input_floats, IED);
   }
   gettimeofday(&after, NULL);
   record_time(before, after, IEDTimeIdx);
/* fprintf(stderr, "%d: after IED: curr_mem_block_idx %d n_mem_blocks %d\n", Myid, mlp-
   >get_curr_mem_block_idx(), mlp->get_n_mem_blocks()); */
   /******************/
   /* volume bit mask */
   /******************/
   start_n_hist = count_histories_in_bitmask(my_nhistories, history_bitmask);
   record_start_nhistories(start_n_hist, VolumeBitmaskTimeIdx, my_nhistories);
   gettimeofday(&before, NULL);
   /* if (0) */
   {
        PCT_MALLOC(volume_bitmask, volume_bitmask_len, char)
        compute_and_bcast_vbm(my_nhistories,
                   history_bitmask,
                   input_floats,
                   input_ushorts,
                   volume_bitmask_len,
                   volume_bitmask);
   }
   gettimeofday(&after, NULL);
   record_time(before, after, VolumeBitmaskTimeIdx);
/* fprintf(stderr, "%d: after VBM: curr_mem_block_idx %d n_mem_blocks %d\n", Myid, mlp-
   >get_curr_mem_block_idx(), mlp->get_n_mem_blocks()); /
if (0)
{
   if (Myid == ForemanRank)
   {
```

```
    FILE *fp;
    char fname[300];
    sprintf(fname, "%s/volume_bitmask.%s.len.%d",
            OutputDirBase, Nowstring, volume_bitmask_len);
    if ((fp = fopen(fname, "w")) != NULL)
      {
          for (i = 0; i < volume_bitmask_len; i ++)
                fprintf(fp, "%d\n". volume_bitmask[i]);
          fclose(fp);
      }
    else
      {
          fprintf(stderr, "ERROR: foreman could not fopen >%s<\n", fname);
      } /* endif */
    /* printf("%d: foreman should print solution here\n", Myid); */
  } /* endif */
}
  /*********************************************************/
  /* remove remaining bad histories before MLP calculation */
  /*********************************************************/
  PCT_MALLOC(entry_exit,
      my_nhistories*sizeof(struct entry_exit_t),
      struct entry_exit_t);
   /* removing histories that don't touch phantom */
   start_n_hist = count_histories_in_bitmask(my_nhistories, history_bitmask);
   record_start_nhistories(start_n_hist, TouchedPhantomTimeIdx, my_nhistories);
   gettimeofday(&before, NULL);
   /* if (0) */
   {
       /* populates entry_exit */
       find_entry_exit_from_vbm_or_remove(my_nhistories,
                   history_bitmask,
                                   input_floats,
                                   input_ushorts,
                                   entry_exit,
                                   volume_bitmask_len,
                                   volume_bitmask);
   }
   gettimeofday(&after, NULL);
   record_time(before, after, TouchedPhantomTimeIdx);
   /* removing histories that are statistical outliers */
   start_n_hist = count_histories_in_bitmask(my_nhistories, history_bitmask);
   record_start_nhistories(start_n_hist, BinnedCleanupIdx, my_nhistories);
   gettimeofday(&before, NULL);
   /* if (0) */
   {
       /* printf("%d: before remove_histories_from_binned_stats()\n", Myid); */
       remove_histories_from_binned_stats(my_nhistories,
                                   history_bitmask,
                                   input_floats,
                                   input_ushorts);
        /* printf("%d: after remove_histories_from_binned_stats()\n", Myid); */
   }
   gettimeofday(&after, NULL);
   ecord_time(before, after, BinnedCleanupIdx);
  /* fprintf(stderr, "%d: after remove remaining: curr_mem_block_idx %d n_mem_blocks %d\n
", Myid, mlp->get_curr_mem_block_idx(), mlp->get_n_mem_blocks()); */

   /*****************/
   /* Adjust IED */
   /*****************/
   start_n_hist = count_histories_in_bitmask(my_nhistories, history_bitmask);
   record_start_nhistories(start_n_hist, AdjustIEDIdx, my_nhistories);
```

```
gettimeofday(&before, NULL);
/* if (0) */
{
    adjust_IED(my_nhistories, history_bitmask, input_ushorts, input_floats,
        IED, entry_exit);
}
gettimeofday(&after, NULL);
record_time(before, after, AdjustIEDIdx);
/* fprintf(stderr, "%d: after Adjust IED: curr_mem_block_idx %d n_mem_blocks %d\n",
Myid, mlp->get_curr_mem_block_idx(), mlp->get_n_mem_blocks()); */
/***********************/
/* MLP - 'A' in Ax = b */
/***********************/
 start_n_hist = count_histories_in_bitmask(my_nhistories, history_bitmask);
 record_start_nhistories(start_n_hist, MLPTimeIdx, my_nhistories);
 gettimeofday(&before, NULL);
 /* if (0) */
 {
     mlp->set_my_nhistories_for_MLP(count_histories_in_bitmask(my_nhistories,
                 history_bitmask));
     compute_mlp(my_nhistories,
         history_bitmask,
         input_floats,
         input_ushorts,
         entry_exit,
         volume_bitmask_len,
         volume_bitmask,
         mlp);
 }
 gettimeofday(&after, NULL);
 record_time(before, after, MLPTimeIdx);
* fprintf(stderr, "%d: after MLP: curr_mem_block_idx %d n_mem_blocks %d\n", Myid, mlp-
>get_curr_mem_block_idx(), mlp->get_n_mem_blocks()); */
 PCT_FREE(entry_exit);
 PCT_FREE(input_ushorts);
 PCT_FREE(input_floats);
 /***************************/
 /* solve for 'x' in Ax = b */
 /***************************/
 start_n_hist = count_histories_in_bitmask(my_nhistories, history_bitmask);
 record_start_nhistories(start_n_hist, LinearSolverTimeIdx, my_nhistories);
 gettimeofday(&before, NULL);
 /* if (0) */
 {
         char done = 0;
#ifndef NO_MPI
         int *dummy_i_recv_buff = (int *) NULL; // needed for bug in MVAPICH2
         float *dummy_f_recv_buff = (float *) NULL; // needed for bug in MVAPICH2
#endif
         PCT_MALLOC(solution, NVOXELS*sizeof(float), float)
         PCT_MALLOC(count, NVOXELS*sizeof(int), int)
         /* seed solution with zeros */
         /* bzero(solution, NVOXELS*sizeof(float)); */
         /* seed solution in phantom with ones and outside phantom 0.001 */
         for (i = 0; i < NVOXELS; i ++)
         {
             if (volume_bitmask[i])
             {
                 solution[i] = INITIAL_SOLUTION_INSIDE_PHANTOM;
#ifdef DEBUG
                 if (Myid == ForemanRank)
                     nvoxels_inside_phantom ++;
#endif
```

```
      }
      else
      {
                      solution[i] = INITIAL_SOLUTION_OUTSIDE_PHANTOM;
#ifdef DEBUG
                      if (Myid == ForemanRank)
                          nvoxels_outside_phantom ++;
#endif
      } /* endif */
      } /* endfor */
      while (!done)
      {
          linear_solver_iterations ++;
          /* printf("%d: starting linear solver iteration %ld\n", */
              /* Myid, linear_solver_iterations); */
          bzero(count, NVOXELS*sizeof(int));
          /* solve for 'x' in Ax = b */
          linear-solvers[linear_solver_idx](mlp, // A
                                   solution, // x
                                   IED, // b
                                   count,
                                   history_bitmask,
                                   my_nhistories,
                                   NVOXELS,
                                   linear_solver_relaxation);
#ifndef NO_MPI
          MPI_Reduce((Myid == ForemanRank
                      ? MPI_IN_PLACE // res clobbers oprnd in root
                      : solution), // operand for non-root
                       (Myid == ForemanRank
                      ? solution // result, relevant at root only
                      : dummy_f_recv_buff),
                       NVOXELS, // count
                       MPI_FLOAT, // data type
                       MPI_SUM, // operator
                       ForemanRank, // root
                       MPI_COMM_WORLD);
          MPI_Reduce((Myid == ForemanRank
                      ? MPI_IN_PLACE // res clobbers oprnd in root
                      : count), // operand for non-root
                    (Myid == ForemanRank
                       ? count // result, relevant at root only
                       : dummy_i_recv_buff),
                   NVOXELS, // count
                   MPI_INT, // data type
                   MPI_SUM, // operator
                   ForemanRank, // root
                   MPI_COMM_WORLD);
#endif
          if (Myid == ForemanRank)
          {
              /* NICK: not sure about count[i] == 0 here ... not sure */
              /* if that's an error or not :-( */
#ifdef DEBUG
              if (linear_solver_iterations == 1)
              {
                  /* volume_bitmask[] and count[] should be the same */
                  /* for all iterations, so suffices to do this only */
                  /* once and for the first iteration */
                  for (i = 0; i < NVOXELS; i ++)
                  {
                      if (count[i] > 0)
                      {
```

**40**

```
                                /* this voxel touched in MLP by >0 histories */
                                if (volume_bitmask[i])
                                {
                                    nvoxels_inside_phantom_mlp_touched ++;
                                }
                                else
                                {
                                    nvoxels_outside_phantom_mlp_touched ++;
                                } /* endif */
                            }
                            else
                             {
                                /* this voxel not touched in MLP by any histories */
                                if (volume_bitmask[i])
                                {
                                    nvoxels_inside_phantom_not_touched ++;
                                }
                                else
                                {
                                    nvoxels_outside_phantom_not_touched ++;
                                } /* endif */
                            } /* endif */
                    } /* endfor */
                } /* endif */
#endif
            for (i = 0; i < NVOXELS; i ++)
            {
                if (count[i] > 0)
                    solution[i] /= count[i];
                else
                {
                    /* count[i] == 0 means that none of the histories, */
                    /* from any proc, touched this voxel_idx=i ... */
                    /* ... however, to make the MPI_Reduce sum for */
                    /* count and solution_vector work easily, each proc */
                    /* (inside linear solver) set their local copy of */
                    /* solution_vector[i] = 0 for those voxels=i that */
                    /* it determined was not touched by any of _its_ */
                    /* histories ... the result of all this is that */
                    /* if _none_ of the histories touched a voxel then */
                    /* its summed value in the foreman is now 0.0 and */
                    /* it needs to be set back to the initialization */
                    /* guess based on whether we think the voxel is in */
                    /* the phantom or not */
                    if (volume_bitmask[i])
                    {
                        solution[i] = INITIAL_SOLUTION_INSIDE_PHANTOM;
                    }
                    else
                    {
                        solution[i] = INITIAL_SOLUTION_OUTSIDE_PHANTOM;
                    } /* endif */
                } /* endif */
            } /* endfor */
            done = test_solution_for_done(solution);
            /* if (0) */
            {
                if (!done)
                {
                    FILE *fp;
                    char fname[300];
                    sprintf(fname, "%s/solution_vector.%s.%s.%021d",
                        OutputDirBase,
```

```
                            Nowstring,
                            Relaxparamstring,
                            linear_solver_iterations);
                        if ((fp = fopen(fname, "w")) != NULL)
                        {
                            fprintf(fp, "%d %d %d\n",
                                    MAX_COLS, MAX_ROWS, MAX_PLANES);
                            for (i = 0; i < NVOXELS; i ++)
                                fprintf(fp, "%f\n", solution[i]);
                            fclose(fp);
                        }
                        else
                        {
                            fprintf(stderr,
                                    "ERROR: foreman could not fopen >%s<\n", fname);
                        } /* endif */
                    } /* endif */
                } /* endif */
            } /* endif */
        #ifndef NO_MPI
            // foreman bcast done
            MPI_Bcast(&done, // buff
                    1, // count
                    MPI_CHAR, // data type
                    ForemanRank, // root
                    MPI_COMM_WORLD);
            if (!done)
            {
                // foreman bcast done
                MPI_Bcast(solution, // buff
                        NVOXELS, // count
                        MPI_FLOAT, // data type
                         ForemanRank, // root
                         MPI_COMM_WORLD);
            } /* endif */
        #endif
            } /* endwhile */
            }
            gettimeofday(&after, NULL);
            record_time(before, after, LinearSolverTimeIdx);
        /* fprintf(stderr, "%d: after linsolv: curr_mem_block_idx %d n_mem_blocks %d\n", Myid,
            mlp->get_curr_mem_block_idx(), mlp->get_n_mem_blocks()); */
            PCT_FREE(volume_bitmask);
            PCT_FREE(count);
            PCT_FREE(history_bitmask);
            PCT_FREE(IED);
            /* printing solution */
            /* if (0) */
            {
            if (Myid == ForemanRank)
            {
            FILE *fp;
            char fname[300];
            sprintf(fname, "%s/solution_vector.%s.%s.final",
                OutputDirBase, Nowstring, Relaxparamstring);
            if ((fp = fopen(fname, "w")) != NULL)
            {
                fprintf(fp, "%d %d %d\n",
                        MAX_COLS, MAX_ROWS, MAX_PLANES);
                for (i = 0; i < NVOXELS; i ++)
                    fprintf(fp, "%f\n", solution[i]);
                fclose(fp);
            }
```

```
      else
      {
           fprintf(stderr, "ERROR: foreman could not fopen >%s<\n", fname);
      } /* endif */
      /* printf("%d: foreman should print solution here\n", Myid); */
      } /* endif */
      }
      PCT_FREE(solution);
      /* print summary stats */
      total_time_secs = time(NULL) - total_time_start;
      /* Time */
      printf("\n");
      printf("%d: ------- Performance Summary\n", Myid);
#ifdef OPT
      printf("%d: Build Version: Optimized\n", Myid);
#endif
#ifdef NOOPT
      printf("%d: Build Version: Not optimized\n", Myid);
#endif
#ifdef DEBUG
      printf("%d: Build Version: Debug\n", Myid);
#endif
      printf("%d: Input: %s/%s*%s\n",
      Myid, InputDir, InputFnamePrefix, InputFnameSuffix);
      if (Myid == ForemanRank)
      {
      printf("%d: Output filename timestamp: %s\n", Myid, Nowstring);
      if (max_histories_per_input_file
         == UNSPECIFIED_MAX_NINPUT_HISTORIES_PER_FILE)
      printf("%d: Max Hist per File: unspecified\n", Myid);
      else
      printf("%d: Max Hist per File: %d\n",
             Myid, max_histories_per_input_file);
             printf("%d: Total N histories: %d\n", Myid, TotalHistories);
             switch (linear_solver_idx)
             {
      case CARP_IDX:
         printf("%d: Linear Solver Method: CARP\n", Myid);
         break;
      case SAP_IDX:
         printf("%d: Linear Solver Method: SAP\n", Myid);
         break;
      default:
         printf("%d: Linear Solver Method: unknown :-(\n", Myid);
         break;
         } /* end switch() */
         printf("%d: Linear Solver Relaxation: %f\n",
      Myid, linear_solver_relaxation);
      printf("%d: Linear Solver Iterations: %ld\n",
      Myid, linear_solver_iterations);
      } /* endif */
      printf("%d: My N histories: %d\n",
      Myid, my_nhistories);
#ifdef HISTOGRAM
      printf("%d: GENERATED HISTOGRAM DATA\n", Myid);
#endif
      /* if (0) */
      {
      printf("%d: -- Timers\n", Myid);
      for (i = 0; i < Ntimers; i ++)
      {
      printf("%d: %18s: usecs: %ld (%ld secs) nhistories %ld\n",
          Myid, TimerNames[i], (long) Timers[i],
```

**43**

```
            (long) Timers[i]/1000000,
        StartNhistories[i]);
    if (StartNhistories[i] != 0)
        printf("%d: %18s usecs/history %ld\n",
            Myid, " ",
            (long) Timers[i]/StartNhistories[i]);
        } /* endfor */
        printf("%d: --\n", Myid);
        printf("%d: Total execution time (secs): %ld\n", Myid, total_time_secs);
        printf("%d:\n", Myid);
        }
        /* Memory */
        /* if (0) */
        {
        unsigned long nbytes_allocd_for_voxel_idxs =
                        mlp->get_nbytes_allocd_for_voxel_idxs();
         unsigned long n_histories_for_mlp = mlp->get_my_nhistories_for_MLP();
         unsigned long n_recorded_voxel_idxs = mlp->get_n_recorded_voxel_idxs();
         unsigned long nbytes_for_recorded_voxel_idxs =
                        sizeof(unsigned int)*n_recorded_voxel_idxs;
         int n_allocd_blocks = mlp->get_n_allocd_blocks();
         unsigned int n_bytes_block;
                        printf("%d: ------- Memory Summary\n", Myid);
                        printf("%d: -- mem for MLP voxel idx's\n", Myid);
                        printf("%d: N bytes allocd for voxel idxs: "
        "%ld (%7.1f MB %4.1f GB)\n",
    Myid, nbytes_allocd_for_voxel_idxs,
    ((float) nbytes_allocd_for_voxel_idxs)/MB,
    ((float) nbytes_allocd_for_voxel_idxs)/GB);
    printf("%d: N bytes used for voxel idxs: "
        "%ld (%7.1f MB %4.1f GB)\n",
    Myid, nbytes_for_recorded_voxel_idxs,
    ((float) nbytes_for_recorded_voxel_idxs)/MB,
    ((float) nbytes_for_recorded_voxel_idxs)/GB);
    printf("%d: N bytes unused for voxel idxs: "
        "%ld (%7.1f MB %4.1f GB)\n",
    Myid, nbytes_allocd_for_voxel_idxs-nbytes_for_recorded_voxel_idxs,
    ((float) (nbytes_allocd_for_voxel_idxs-nbytes_for_recorded_voxel_idxs))/MB,
    ((float) (nbytes_allocd_for_voxel_idxs-nbytes_for_recorded_voxel_idxs))/GB);
    printf("%d: Percent alloc used for voxel idxs: %4.1f %% \n",
    Myid,
    (nbytes_allocd_for_voxel_idxs != 0
        ? 100.0*((float) nbytes_for_recorded_voxel_idxs)
            /((float) nbytes_allocd_for_voxel_idxs)
        : 0.0));
    printf("%d: -- counts for MLP voxel idx's\n", Myid);
    printf("%d: N recorded voxel idxs: %ld\n", Myid, n_recorded_voxel_idxs);
    printf("%d: N histories for MLP: %ld\n", Myid, n_histories_for_mlp);
    printf("%d: Avg rec v_idxs/hist: %ld\n",
    Myid,
    (n_histories_for_mlp != 0
        ? n_recorded_voxel_idxs/n_histories_for_mlp
        : 0L));
#ifdef DEBUG
    if (Myid == ForemanRank)
    {
    printf("%d: -- Voxel space\n", Myid);
    printf("%d: N voxels inside phantom: %ld (%4.1f %%)\n",
        Myid, nvoxels_inside_phantom,
        (100.0*((float)) nvoxels_inside_phantom)/((float) NVOXELS)));
    printf("%d: N voxels outside phantom: %ld (%4.1f %%)\n",
        Myid, nvoxels_outside_phantom,
        (100.0*((float)) nvoxels_outside_phantom)/((float) NVOXELS)));
```

```
        printf("%d: N voxels MLP-touched inside phantom: "
               "%ld (%4.1f %% of inside voxels - want 100%%)\n",
           Myid, nvoxels_inside_phantom_mlp_touched,
            (100.0*((float) nvoxels_inside_phantom_mlp_touched)
               /((float) nvoxels_inside_phantom)));
        printf("%d: N voxels MLP-touched outside phantom: "
               "%ld (should be zero)\n",
           Myid, nvoxels_outside_phantom_mlp_touched);
        printf("%d: N voxels *not* MLP-touched inside phantom: "
               "%ld (%4.1f %% of inside voxels - want zero)\n",
           Myid, nvoxels_inside_phantom_not_touched,
            (100.0*((float) nvoxels_inside_phantom_not_touched)
               /((float) nvoxels_inside_phantom)));
        printf("%d: N voxels *not* MLP-touched outside phantom: "
           "%ld (%4.1f %% of outside voxels - should 100%%)\n",
           Myid, nvoxels_outside_phantom_not_touched,
            (100.0*((float) nvoxels_outside_phantom_not_touched)
               /((float) nvoxels_outside_phantom)));
               } /* endif */
#endif
           printf("%d: -- Block stats\n", Myid);
           printf("%d: N copies: %ld\n", Myid, mlp->get_n_copies());
           printf("%d: N bytes copied: %ld\n", Myid, mlp->get_n_copied_bytes());
           printf("%d: N allocd blocks: %d\n", Myid, n_allocd_blocks);
           for (i = 0; i < n_allocd_blocks; i ++)
               {
    n_bytes_block = mlp->get_block_nbytes(i);
    printf("%d: - blk %2d N bytes: %d (%7.1f MB %4.1f GB)\n",
        Myid, i, n_bytes_block,
         ((float) n_bytes_block)/MB,
         ((float) n_bytes_block)/GB);
         } /* endif */
         delete mlp;
         printf("%d: -- Basic memory stats\n", Myid);
         printf("%d: N calls to malloc(): %ld\n", Myid, N_mallocs);
         printf("%d: N calls to realloc(): %ld\n", Myid, N_reallocs);
#ifdef DEBUG
         printf("%d: N calls to free(): %ld\n", Myid, N_frees);
#endif
         printf("%d: N allocated bytes: %ld (%7.1f MB %4.1f GB)\n",
  Myid, N_allocated_bytes,
  ((float) N_allocated_bytes)/MB, ((float) N_allocated_bytes)/GB);
         printf("%d: Largest single mem req bytes: %ld (%7.1f MB %4.1f GB)\n",
  Myid, Largest_single_mem_request,
  ((float) Largest_single_mem_request)/MB,
  ((float) Largest_single_mem_request)/GB);
         printf("%d: : file: %s line: %d\n",
  Myid, Largest_single_mem_request_fname,
  Largest_single_mem_request_line_no);
#ifdef DEBUG
  printf("%d: N freed bytes: %ld (%7.1f MB %4.1f GB)\n",
  Myid, N_freed_bytes,
  ((float) N_freed_bytes)/MB, ((float) N_freed_bytes)/GB);
  printf("%d: N not freed bytes: %ld (%7.1f MB %4.1f GB)\n",
  Myid, N_allocated_bytes-N_freed_bytes,
  ((float) (N_allocated_bytes-N_freed_bytes))/MB,
  ((float) (N_allocated_bytes-N_freed_bytes))/GB);
  printf("%d: High water mark bytes: %ld (%7.1f MB %4.1f GB)\n",
  Myid, N_allocated_bytes_high_water_mark,
  ((float) N_allocated_bytes_high_water_mark)/MB,
  ((float) N_allocated_bytes_high_water_mark)/GB);
#endif
  printf("\n");
```

```
      }
#ifdef DEBUG
#ifndef NO_MPI
  /* MPI Profiles */
  /* if (0) */
  {
  printf("%d: -- MPI Functions\n", Myid);
  for (i = 0; i < Nfuncs; i ++)
  {
  if (func_profiles[i].does_communicate)
  {
      printf("%d: %15s: Ncalls %3d secs %f bytes %ld (%6.1f GB/sec)\n",
          Myid,
          func_profiles[i].func_name,
          func_profiles[i].call_count,
          func_profiles[i].total_time_secs,
          func_profiles[i].total_bytes,
          (func_profiles[i].total_time_secs > 0.0
              ? (func_profiles[i].total_bytes/GB)
                  /func_profiles[i].total_time_secs
              : 0.0)
              ) ;
  }
  else
  {
      printf("%d: %15s: Ncalls %3d secs %f\n",
          Myid,
          func_profiles[i].func_name,
          func_profiles[i].call_count,
          func_profiles[i].total_time_secs);
  } /* endif */
  } /* endfor */
  printf("\n");
  }
#endif
#endif
  /************/
  /* epilogue */
  /************/
#ifndef NO_MPI
  MPI_Finalize();
#endif
  exit(0);
} /* end main() */
/*
  "Note on a Method for Calculating Corrected Sums of Squares and Products,"
  B.P. Welford, Technometrics, Vol 4, No. 3, Aug 1962, 419-420.
  The Art of Computer Programming, D.E. Knuth, vol 2, 2nd Edition, p 216
  The Art of Computer Programming, D.E. Knuth, vol 2, 3rd Edition, p 232
  Reccurrence forumla that keeps a running mean (M_k) and running SSE (S_k)
  for some stream of values X_i
For x_1
  M_1 = x_1
  S_1 = 0
For x_i i>1
  M_i = M_(i-1) + [(x_i - M_(i-1))/i]
  S_i = S_(i-1) + [(x_i - M_(i-1)) * (x_i - M_i)]
At any time k>1
  running mean = M_k
  running _sample_ (i.e., not _population_) variance = S_k/(k-1)
*/
static void accumulate_stats_val(float *m,
                        float *s,
```

```
#ifdef DEBUG
                          float *min,
                          float *max,
#endif
                          unsigned int *n,
                          float val)
{
    *n = *n + 1;
     if (*n == 1)
     {
         *m = val;
         *s = 0.0;
#ifdef DEBUG
         *min = *max = val;
#endif
     }
     else
     {
         float old_mean = *m;
         float d = val - old_mean;
         *m += (d/(*n));
         *s += (d*(val - *m));
#ifdef DEBUG
          if (*min > val) *min = val;
          if (*max < val) *max = val;
#endif
     } /* endif */
     /* printf("after pushing %f RunningMean %f RunningSSE %f\n",val,*m,*s); */
     return;
} /* end accumulate_stats_val() */
static void compute_and_bcast_vbm(int my-nhistories,
                         unsigned char *history_bitmask,
                         float *input_floats,
                         unsigned short *input_ushorts,
                         int volume_bitmask_len,
                         char *volume_bitmask)
{
#ifndef NO_MPI
    MPI_Op op;
     char *dummy_recv_buff = (char *) NULL; // needed for bug in MVAPICH2
#endif
     // makevbm2.cc
     compute_initial_vbm(my_nhistories,
             history_bitmask,
              input_floats,
              input_ushorts,
             volume_bitmask_len,
             volume_bitmask);
#ifndef NO_MPI
    MPI_Op_create((MPI_User_function*) andem, // my user-defined func
              1,                                // commute flag
              &op);                             // op handle
     MPI_Reduce((Myid == ForemanRank
          ? MPI_IN_PLACE                    // result clobbers operand for root
          : volume_bitmask),                // operand for non-root
       (Myid == ForemanRank
          ? volume_bitmask                  // result, relevant at root only
          : dummy_recv_buff),
       volume_bitmask_len,                  // count
       MPI_CHAR,                            // data type
       op,                                  // operator
       ForemanRank,                         // root
       MPI_COMM_WORLD) ;
```

47

```
      MPI_Op_free(&op);
      #endif
      // formeman only morph_clean,
      if (Myid == ForemanRank)
      {
         // morph_clean2.cc
         morph_clean(volume_bitmask_len, volume-bitmask);
      } /* endif */
#ifndef NO_MPI
      // foreman bcast morph_cleaned volume bitmask
      MPI_Bcast(volume_bitmask, // buff
            volume_bitmask_len, // count
                   MPI_CHAR, // data type
                ForemanRank, // root
             MPI_COMM_WORLD);
#endif

#if 0
  // intersect2.cc
  // NICK: push all this into mlp code so that you do NOT
  // have to malloc and store everything in entry_exit
  compute_entry_exit_from_vbm(my_nhistories,
                      input_floats,
                      input_ushorts,
                      entry_exit);
#endif

  return;

} /* end compute_and_bcast_vbm() */
static void compute_nhistories(int *my_nhistories,
                   int *histories_per_worker, // relevant foreman only
                   int n_input_histories,
                   int max_histories_per_foreman_or_worker,
                   int *must_fill_foreman)
{
      /* max_histories_per_foreman_or_worker is always defined */
      /* ... in that context there are two cases: */
      /* (1) we don't know how many input histories there are: */
      /* read max_histories_per_foreman_or_worker into */
      /* each process, must fill all workers, foreman is */
      /* last to get his and so may be less than full */
      /* (2) we know how many input histories there are: */
      /* spread histories evenly across all Nprocs, make */
      /* sure under max_histories_per_foreman_or_worker, */
      /* all workers get the same number of histories and */
      /* foreman may get more due to rounding */

      if (n_input_histories == UNSPECIFIED_NINPUT_HISTORIES)
      {
         *my_nhistories =
               *histories_per_worker = max_histories_per_foreman_or_worker;

          *must_fill_foreman = 0;
}
else
{
           /* n_input_histories specified on command line */

            *histories_per_worker = n-input_histories/Nprocs;
             if (Myid == ForemanRank)
          {
            /* Foreman must absorb rounding slack */
```

```
                *my_nhistories = n_input_histories -
                                ((Nprocs-1)*(*histories_per_worker));
        }
        else
        {
           *my_nhistories = *histories_per_worker;
        } /* endif */

        *must_fill_foreman = 1;

   }/* endif */

   if (*my_nhistories > max_histories_per_foreman_or_worker)
   {
   fprintf(stderr,
       "ERROR: %s %d: proc %d: "
        "my_nhistories %d > max_histories_per_foreman_or_worker %d\n",
         _FILE_, _LINE_, Myid,
          *my_nhistories, max_histories_per_foreman_or_worker);
   PCT_ABORT(ABORT_TOO_MANY_HISTORIES)
   } /* endif */
   return;
} /* end compute_nhistories() */
   static float compute_stat_val(float *input_floats,
                         unsigned short *input_ushorts,
                         int h,
                         int stat_displ)
{

     float val;

     switch (stat_displ)
     {
         case STAT_DELTA_X:
     {
        int x1 = get_x1(input_ushorts, h);
        int x2 = get_x2(input_ushorts, h);
        int x3 = get_x3(input_ushorts, h);
        int x4 = get_x4(input_ushorts, h);

       val = ((float) (x4 - x3) - (x2 - xl));
     }
     break;
     case STAT_DELTA_Y:
     {
         int y1 = get_y1(input_ushorts, h);
         int y2 = get_y2(input_ushorts, h);
         int y3 = get_y3(input_ushorts, h);
         int y4 = get_y4(input_ushorts, h);
        val = ((float) (y4 - y3) - (y2 - yl));
     }
     break;
     case STAT_DELTA_ENERGY:
     {
         float ein = get_ein(input_floats, h);
         float eout = get_eout(input_floats, h);

        val = ein-eout;
     }
                     break;
             default:
                     val = 0.0; // supress annoying compiler warning
                     fprintf(stderr,
```

```
                    "ERROR: %s %d: proc %d: "
                    "remove_based_on binned_stat(): "
                    "passed unrecognizable stat_displ %d\n",
                    _FILE_, _LINE_, Myid,
                     stat_displ);
                PCT_ABORT(ABORT_STATS_ERROR)
                break;
  } /* end switch() */
  return val;
} /* end compute_stat_val() */
static int count_histories_in_bitmask(int my_nhistories,
                                unsigned char *history_bitmask)
{
  int n_full_bytes = my_nhistories / 8;
  int n_remaining_bits = my_nhistories % 8;
  int i;
  int rc = 0;
  /* first counting the bits in full bytes */
  for (i = 0; i < n_full_bytes; i ++)
  {
       switch (history_bitmask[i])
       {
             /* nbits = 0 */
             case 0x0: rc += 0; break;
          /* nbits = 1 */
          case 0x10: case 0x1: case 0x20: case 0x2: case 0x40: case 0x4:
          case 0x80: case 0x8:
              rc += 1; break;
          /* nbits = 2 */
          case 0x11: case 0x12: case 0x14: case 0x18: case 0x21: case 0x22:
          case 0x24: case 0x28: case 0x30: case 0x3: case 0x41: case 0x42:
          case 0x44: case 0x48: case 0x50: case 0x5: case 0x60: case 0x6:
          case 0x81: case 0x82: case 0x84: case 0x88: case 0x90: case 0x9:
          case 0xaO: case 0xa: case 0xc0: case 0xc
              rc += 2; break;
          /* nbits = 3 */
          case 0x13 case 0x15 case 0x16 case 0x19 case 0x1a: case 0x1c:
          case 0x23: case 0x25: case 0x26: case 0x29 case 0x2a: case 0x2c:
          case 0x31 case 0x32: case 0x34 case 0x38 case 0x43: case 0x45
          case 0x46: case 0x49: case 0x4a: case 0x4c: case 0x51 case 0x52:
          case 0x54: case 0x58 case 0x61: case 0x62: case 0x64: case 0x68:
          case 0x70 case 0x7: case 0x83 case 0x85 case 0x86: case 0x89:
          case 0x8a: case 0x8c: case 0x91: case 0x92: case 0x94: case 0x98
          case 0xa1 case 0xa2: case 0xa4: case 0xa8: case 0xb0 case 0xb:
          case 0xc1 case 0xc2: case 0xc4: case 0xc8: case 0xd0: case 0xd:
          case 0xe0 case 0xe:
               rc += 3; break;
          /* nbits = 4 */
          case 0x17: case 0x1b: case 0x41d: case 0x1e: case 0x27: case 0x2b:
          case 0x2d: case 0x2e: case 0x33: case 0x35: case 0x36: case 0x39:
          case 0x3a: case 0x3c: case 0x47: case 0x4b: case 0x4d: case 0x4e:
          case 0x53: case 0x55: case 0x56: case 0x59: case 0x5a: case 0x5c:
           case 0x63: case 0x65: case 0x66: case 0x69: case 0x6a: case 0x6c:
           case 0x71: case 0x72 case 0x74 case 0x78: case 0x87: case 0x8b:
           case 0x8d: case 0x8e: case 0x93: case 0x95: case 0x96: case 0x99:
           case 0x9a: case 0x9c: case 0xa3: case 0xa5: case 0xa6: case 0xa9:
           case 0xaa: case 0xac: case 0xb1: case 0xb2: case 0xb4: case 0xb8:
           case 0xc3: case 0xc5: case 0xc6: case 0xc9: case 0xca: case 0xcc:
           case 0xd1: case 0xd2: case 0xd4: case 0xd8: case 0xe1: case 0xe2:
           case 0xe4: case 0xe8: case 0xf0: case 0xf:
               rc += 4; break;
          /* nbits = 5 */
            case 0x1f: case 0x2f: case 0x37: case 0x3b: case 0x3d: case 0x3e:
```

```
                case 0x4f: case 0x57: case 0x5b: case 0x5d: case 0x5e: case 0x67:
                case 0x6b: case 0x6d: case 0x6e: case 0x73: case 0x75: case 0x76:
                case 0x79: case 0x7a: case 0x7c: case 0x8f: case 0x97: case 0x9b:
                case 0x9d: case 0x9e: case 0xa7: case 0xab: case 0xad: case 0xae:
                case 0xb3: case 0xb5: case 0xb6: case 0xb9: case 0xba: case 0xbc:
                case 0xc7: case 0xcb: case 0xcd: case 0xce: case 0xd3: case 0xd5:
                case 0xd6: case 0xd9: case 0xda: case 0xdc: case 0xe3: case 0xe5:
                case 0xe6: case 0xe9: case 0xea: case 0xec: case 0xf1: case 0xf2:
                case 0xf4: case 0xf8:
                    rc += 5; break;
                /* nbits = 6 */
                case 0x3f: case 0x5f: case 0x6f: case 0x77: case 0x7b: case 0x7d:
                case 0x7e: case 0x9f: case 0xaf: case 0xb7: case 0xbb: case 0xbd:
                case 0xbe: case 0xcf: case 0xd7: case 0xdb: case 0xdd: case 0xde:
                case 0xe7: case 0xeb: case 0xed: case 0xee: case 0xf3: case 0xf5:
                case 0xf6: case 0xf9: case 0xfa: case 0xfc:
                    rc += 6; break;
                /* nbits = 7 */
                case 0x7f: case 0xbf: case 0xdf: case 0xef: case 0xf7: case 0xfb:
                case 0xfd: case 0xfe:
                    rc += 7; break;
                 /* nbits = 8 */
                 case 0xff: rc += 8; break;
                 default: break;
        } /* end switch() */
    } /* endfor */
    /* now counting the bits in last non-full byte */
    for (i = 0; i < n_remaining_bits; i ++)
    if (TEST(history_bitmask+n_full_bytes, i))
        rc ++;
    return rc;
} /* end count_histories_in_bitmask() */
static void fill_buffers_from_files(float *input_floats,
                            unsigned short *input_ushorts,
                            int *deg,
                            FILE **fp,
                            int *histories_read_from_this_file,
                            int max_histories per_input file,
                            int nhistories_buffsize,
                            int *my_nhistories,
                            int must_fill) // must_fill == 0 iff foreman
{
    float *float_p;
    unsigned short *ushort_p;
    int done;
    float_p = input_floats;
    ushort_p = input_ushorts;
    done = 0;
    for (*my_nhistories = 0;
                !done && *my_nhistories < nhistories_buffsize;
                    *my_nhistories = *my_nhistories + 1)
    {
        if (max_histories_per_input_file
            != UNSPECIFIED_MAX_NINPUT_HISTORIES_PER_FILE
         && *histories_read_from_this_file >= max_histories_per_input_file)
    {
        /* reached limit of histories that can be read from this file */
        /* ... time to start reading from the next input file */
        fclose(*fp);
        *deg = *deg + 2;
*       fp = open_input_file(*deg);
        *histories_read_from_this_file = 0;
    } /* endif */
```

```
if (fscanf(*fp, "%hu %hu %hu %hu %hu %hu %hu %hu %f %f %f\n",
    ushort_p+X1_DISPL, ushort_p+Y1_DISPL,
    ushort_p+X2_DISPL, ushort_p+Y2_DISPL,
    ushort_p+X3_DISPL, ushort_p+Y3_DISPL,
    ushort_p+X4_DISPL, ushort_p+Y4_DISPL,
     float_p+EIN_DISPL, float_p+EOUT_DISPL, float_p+THETA_DISPL)
        == EOF)
{
/* ran out of data from open file, */
/* closing and moving to next file */
fclose(*fp);
*deg = *deg + 2;
*fp = open_input_file(*deg);
*histories_read_from__this_file = 0;
if (fscanf(*fp, "%hu %hu %hu %hu %hu %hu %hu %hu %f %f %f\n",
    ushort_p+X1_DISPL, ushort_p+Y1_DISPL
    ushort_p+X2_DISPL, ushort_p+Y2_DISPL,
    ushort_p+X3_DISPL, ushort_p+Y3_DISPL,
    ushort_p+X4_DISPL, ushort_p+Y4_DISPL,
    float_p+EIN_DISPL, float_p+EOUT_DISPL, float_p+THETA_DISPL)
            == EOF)
{
    if (must_fill)
     {
        /* ran out of data for non-foreman workers */
        fprintf(stderr,
            "ERROR: %s %d: proc %d: "
            "ran out of data data deg %d\n",
        _FILE_, _LINE_, Myid,
            *deg);
        PCT_ABORT(ABORT_TOO_MANY_HISTORIES)
     }
    else
     {
        done = 1;
     } /* endif */
   } /* endif */
} /* endif */
float_p += INPUT_FLOAT_STRIDE;
ushort_p += INPUT_USHORT_STRIDE;
*histories_read_from_this_file = *histories_read_from_this_file + 1;
} /* endfor */
if (done)
{
    *my_nhistories = *my_nhistories - 1;
} /* endif */
return;
} /* end fill_buffers_from_files() */
static void find_entry_exit_from_vbm_or_remove (int nhistories,
                                            unsigned char *history_bitmask,
                                            float *input_floats,
                                            unsigned short *input_ushorts,
                                            struct entry_exit_t *entry_exit,
                                            int volume_bitmask_len,
                                            char *volume_bitmask)
{
  int h;
#ifdef DEBUG
  int n_cleared_histories = 0;
#endif
  for (h = 0; h < nhistories ; h++)
  {
      if (TEST(history_bitmask, h))
```

```
                    {
                        // intersect2.cc
                        if (! compute_entry_exit_from_vbm(h,
                                                input_floats,
                                                input_ushorts,
#ifdef DEBUG
                                                volume_bitmask_len,
#endif
                                                volume_bitmask,
                                                entry_exit))
        {
            /* could NOT find entry/exit on phantom ... */
            /* declare a rogue history -- need to toss it */
            CLEAR(history_bitmask, h);
            /* NICK: need to write code to re-claim space used */
            /* NICK: in this block to record voxel ids thus */
            /* NICK: far for this history. */
#ifdef DEBUG
            n_cleared_histories ++;
#if 0
            fprintf(stderr,
                "ERROR: %s %d: proc %d: "
                "computed vbm_index %d (max %d) "
                "from history %d "
             "x1 %d y1 %d x2 %d y2 %d x3 %d y3 %d x4 %d y4 %d "
                "entry_x %f entry_y %f entry_z %f "
                "exit_x %f exit_y %f exit_z %f "
                "\n",
                _FILE_, LINE_, Myid,
                voxel_idx, volume_bitmask_len,
                h,
            get_x1(input_ushorts, h), get_y1(input_ushorts, h),
            get_x2(input_ushorts, h), get_y2(input_ushorts, h),
            get_x3(input_ushorts, h), get_y3(input_ushorts, h),
            get_x4(input_ushorts, h), get_y4(input_ushorts, h),
            entry_exit.entry_x, entry_exit.entry-y, entry_exit.entry_z,
            entry_exit.exit_x, entry_exit.exit_y, entry_exit.exit_z);
#endif
            /* MPI::COMM_WORLD.Abort(ABORT_INVALID_IDX); */
#endif
        } /* endif */
        } /* endif */
    } /* endfor */
#ifdef DEBUG
  printf("%d: could not connect to phantom n_cleared_histories %d\n",
        Myid, n_cleared_histories);
#endif
  return;
} /* end find_entry_exit_from_vbm_or_remove() */
static int get_stat_cell_idx(float *input_floats,
                    unsigned short *input_ushorts,
                    int h,
                    int stat_displ)
{
        /* binning based on INTERNAL ENTRY plane */
        int x = get_x2(input_ushorts, h);
        int y = get__y2(input_ushorts, h);
        int converted_x = (x * REGION_COLS)/MAX_TRACK_X_IDX;
        int converted_y = (y * REGION_ROWS)/MAX_TRACK_Y_IDX;
         float theta = get_theta(input_floats, h);
         int theta_idx = (int)(theta * THETA_IDX_PER_DEGREE + 0.5);
#ifdef DEBUG
    {
```

```
                         int rc = ( (STAT_CELL_IDX_ANGLE_STRIDE * theta_idx)
                 + (STAT_CELL_IDX_ROW_STRIDE * converted_y)
                 + (STAT_CELL_IDX_COL_STRIDE * converted_x)
                 + stat_displ);
                         if (rc < 0 || rc >= N_STAT_CELLS)
                         {
                             fprintf(stderr,
                                 "ERROR: %s %d: proc %d: "
                                 "computed invalid stats_idx %d "
                                 "from history %d x %d y %d theta %f stat_displ %d "
                                 "(must be 0 <= stats_idx < N_STAT_CELLS %d)\n",
                                 _FILE_, _LINE_, Myid,
                                 rc, h, x, y, theta, stat_displ, N_STAT_CELLS);
                             PCT_ABORT(ABORT_STATS_ERROR)
                 } /* endif */
                 return rc;
    }
#else
         return ((STAT_CELL_IDX_ANGLE_STRIDE * theta_idx)
                   + (STAT_CELL_IDX_ROW_STRIDE * converted_y)
                   + (STAT_CELL_IDX_COL_STRIDE * converted_x)
                   + stat_displ);
#endif
} /* end get_stat_cell_idx() */
static FILE *open_input_file(int degree)
{
  FILE *fp;
  char inputfilename[256];
  sprintf(inputfilename, "%s/%s%03d%s",
      InputDir, InputFnamePrefix, degree, InputFnameSuffix);
   if (!(fp = fopen(inputfilename, "r")))
   {
      fprintf(stderr, "ERROR: could not open input file >%s<\n",
           inputfilename);
      PCT_ABORT(ABORT_FAILED_FOPEN)
  } /* endif */
  return fp;
} /* end open_input_file() */
/* extern "C" int getopt(int argc, char * const argv[], const char *optstring) throw
*/
extern "C" int getopt(int argc, char * const argv[], const char *optstring);
static void parse_command_line_args(int argc,
                                char **argv,
                                int *n_input_histories,
                                int *max_histories per_foreman_or_worker,
                                int *max_histories_per_input_file,
                                float *linear_solver_relaxation)
{
        int c;
        enum input_sets {threeM, thirtyeightM, twoB} input;
        int error;
         /* int getopt(int argc, char * const argv[], const char *optstring); */
         extern char *optarg;
         /* extern int optind, opterr, optopt; */
         error = 0;
          /* default values */
         *n_input_histories = UNSPECIFIED_NINPUT_HISTORIES;
         *max_histories_per_input_file = UNSPECIFIED_MAX_NINPUT_HISTORIES_PER_FILE;
         *max_histories_per_foreman_or_worker = DEFAULT_MAXHISTORIES_PER_WORKER;
          input = thirtyeightM;
          *linear_solver_relaxation = DEFAULT_RELAXATION;
           while ((c = getopt(argc, argv, "sblh:f:m:r:")) != -1)
            {
```

```
                    switch (c)
                    {
                        case 's': input = threeM; break;
                        case 'b': input = thirtyeightM; break;
                        case '1': input = twoB; break;
                        case 'h': *n_input_histories = atoi(optarg); break;
                        case 'f': *max_histories_per_input_file=atoi(optarg); break;
                        case 'm': *max_histories_per_foreman_or_worker=atoi(optarg); break;
                        case 'r': *linear_solver_relaxation=atof(optarg); break;
                        default: error = 1; break;
                } /* end switch() */
        } /* endwhile */
        if (error)
        {
            if (Myid == ForemanRank)
            {
                /* only Master prints usage message */
                fprintf(stderr,
                        "\n\tusage: %s {-s | -b | -1} "
                            "{-h <N input histories>} "
                    "           {-f <max N input histories per file>} "
                    "           {-m <max N histories per worker or foreman>} "
                    "           {-r <linear solver relaxation param>} "
                            "\n\n",
                        argv[0]);
                fprintf(stderr, "where\n\n");
                fprintf(stderr, "\t-s - small input, 2D, ~3M histories\n");
                fprintf(stderr, "\t-b - big input, 3D, ~38M histories (default)\n");
                fprintf(stderr, "\t-1 - large input, 3D, ~1.9B histories\n");
                fprintf(stderr, "\n");
#ifdef NO_MPI
                exit(1);
#else
                MPI_Abort(MPI_COMM_WORLD, ABORT_INVALID_CMD_LINE_ARGS);
 #endif
            } /* endif */
        } /* endif */
        switch (input)
        {
                case threeM:
                    sprintf(InputDir, "%s/Input-2D-3.2M/", InputDirBase);
                    strcpy(InputFnamePrefix, "projections.");
                    strcpy(InputFnameSuffix, ".dat");
                break;
                case thirtyeightM:
                    sprintf(InputDir, "%s/Input-Full3D-38.6M/", InputDirBase);
                    strcpy(InputFnamePrefix, "angle_");
                    strcpy(InputFnameSuffix, ".txt");
                break;
                case twoB:
                    sprintf(InputDir, "%s/Input-Full3D-GeneratedAtNIU-1.9B/ConvertedFromPen
    fold/",
                            InputDirBase);
                    strcpy(InputFnamePrefix, "angle_");
                    strcpy(InputFnameSuffix, ".text");
                break;
                default:
                    fprintf(stderr,
                            "ERROR: parse_command_line_args(): "
                            "encountered invalid input = %d\n",
                            input);
#ifdef NO_MPI
                    exit(1);
```

```
        #else
                    MPI_Abort(MPI_COMM_WORLD, ABORT_INVALID_CMD_LINE_ARGS);
        #endif
                break;
        } /* end switch */
        return;
    } /* end parse_command_line_args() */
    static void read_and_distribute_input(int histories_per_worker,
                                          int max_histories_per_input_file,
                                          int *my_nhistories,
                                          int must_fill_foreman,
                                          float *input_floats,
                                          unsigned short *input_ushorts)
    {
        int deg;
        int histories_read_from_this_file;
    #ifndef NO_MPI
        int i;
        MPI_Status status;
    #endif
     FILE *fp;
        /* NICK: fix this - set to received for worker or last read for foreman*/
        /* max_histories_per_foreman_or_worker is always defined */
        /* in that context there are two cases: */
        /* (1) we don't know how many input histories there are: */
        /* read max_histories_per_foreman_or_worker into */
        /* each process, must fill all workers, foreman is */
        /* last to get his and so may be less than full */
        /* (2) we know how many input histories there are: */
        /* spread histories evenly across all Nprocs, make */
        /* sure under max_histories_per_foreman_or_worker, */
        /* all workers get the same number of histories and */
        /* foreman may get more due to rounding */
        /*****************************/
        /* Read and distribute input */
        /*****************************/
        if (Myid == ForemanRank)
        {
            /* I am the foreman */
            TotalHistories = 0;
            /* opening first file */
            deg = 0;
            fp = open_input_file(deg);
            histories_read_from_this_file = 0;;
    #ifndef NO_MPI
            /* must read and distribute input to other workers */
            for (i = 1; i < Nprocs; i ++)
            {
                fill_buffers_from_files(input_floats,
                            input_ushorts,
                            &deg,
                            &fp,
                            &histories_read_from_this_file,
                            max_histories_per_input_file,
                            histories_per_worker,
                            my_nhistories,
                            1); // must_fill
            MPI_Send(input_floats, /* buff */
                    histories_per_worker*INPUT_FLOAT_STRIDE, /* count */
                    MPI_FLOAT, /* type */
                    i, /* dest */
                    INPUT_FLOAT_TAG, /* tag */
                    MPI_COMM_WORLD); /* comm */
```

```
        MPI_Send(input_ushorts, /* buff */
                    histories_per_worker*INPUT_USHORT_STRIDE, /* count */
                    MPI_UNSIGNED_SHORT, /* type */
                    i, /* dest */
                    INPUT_USHORT_TAG, /* tag */
                    MPI_COMM_WORLD) ; /* comm */
         TotalHistories += histories_per_worker;
  } /* endfor */
#endif
  /* now fill foreman's buffer, which may not be full */
  fill_buffers_from_files(input_floats,
                        input_ushorts,
                        &deg,
                        &fp,
                        &histories_read_from_this_file,
                        max_histories_per_input_file,
                        *my_nhistories,
                        my_nhistories, // may reset foreman's val
                        must_fill_foreman); // must_fill
  TotalHistories += (*my_nhistories);
 }
#ifndef NO_MPI
 else
 {
 /* I am NOT the foreman */
 /* must receive my input from the foreman */
 MPI_Recv(input_floats,
            histories_per_worker*INPUT_FLOAT_STRIDE, /* count */
            MPI_FLOAT, /* type */
            ForemanRank, /* source */
            INPUT_FLOAT_TAG, /* tag */
            MPI_COMM_WORLD, /* comm */
            &status); /* status */
 MPI_Recv(input_ushorts,
            histories_per_worker*INPUT_USHORT_STRIDE, /* count */
            MPI_UNSIGNED_SHORT, /* type */
            ForemanRank, /* source */
            INPUT_USHORT_TAG, /* tag */
            MPI_COMM_WORLD, /* comm */
            &status); /* status */
 } /* endif */
#endif
 return;
} /* end read_and_distribute_input() */
static void remove_based_on_binned_stat(int nhistories,
                            unsigned char *history_bitmask,
                            float *input_floats,
                            unsigned short *input_ushorts,
                            struct stats s[],
                            float sigma_cutoff[],
                            int stat_displ)
{
 int h;
 int stat_idx;
 float val;
#ifdef DEBUG
 int n_cleared_histories = 0;
#endif
 for (h = 0; h < nhistories; h ++)
 {
  if (TEST(history_bitmask, h))
  {
      /* this history has not been previously thrown out */
```

```
        stat_idx = get_stat_cell_idx(input_floats,
                                     input_ushorts,
                                     h,
                                     stat_displ);
#ifdef DEBUG
    /***************/
    /* sanity check */
    /***************/
    /****************************************************/
    /* confirming that history has not yet been cleared */
    /* has been accumulated as part of the global stats */
    /****************************************************/
    if (s[stat_idx].n <= 0)
    {
        fprintf(stderr,
            "ERROR: %s %d: proc %d: "
            "history %d: stat_idx %d from stat_displ %d "
            "found n = %d (must be > 0)\n",
            _FILE_, _LINE_, Myid,
             h, stat_idx, stat_displ, s[stat_idx].n);
        PCT_ABORT(ABORT_STATS_ERROR)
    } /* endif */
#endif
    val = compute_stat_val(input_floats,
                           input_ushorts,
                           h,
                           stat_displ);
#ifdef DEBUG
    /***************/
    /* sanity check */
    /***************/
    /********************************************************************/
    /* confirming global_min_x <= x <= global_max_x */
    /* where "global" means across all procs for stats cell stat_idx */
    /********************************************************************/
    if (val < s[stat_idx].min)
    {
        fprintf(stderr,
            "ERROR: %s %d: proc %d: "
            "history %d: stat_idx %d from stat_displ %d "
            "computed val %f < global min %f\n",
            _FILE_, _LINE_, Myid,
            h, stat_idx, stat_displ, val, s[stat_idx].min);
        PCT_ABORT(ABORT_STATS_ERROR)
    } /* endif */
    if (val > s[stat_idx].max)
    {
        fprintf(stderr,
            "ERROR: %s %d: proc %d: "
            "history %d: stat_idx %d from stat_displ %d "
            "computed val %f > global max %f\n",
            _FILE_, LINE_, Myid,
            h, stat_idx, stat_displ, val, s[stat_idx].max);
        PCT_ABORT(ABORT_STATS_ERROR)
     } /* endif */
#endif
            if (fabs(val - s[stat_idx].mean) > sigma_cutoff[stat_idx])
            {
                CLEAR(history_bitmask, h);
#ifdef DEBUG
                n_cleared_histories ++;
#endif
            } /* endfor */
```

```
        } /* endif */
    } /* endfor */
#ifdef DEBUG
    printf("%d: stat_displ %d n_cleared_histories %d\n",
        Myid, stat_displ, n_cleared_histories);
#endif
    return;
} /* end remove_based_on_binned_stat() */
    static void remove_histories_from_binned_stats(int nhistories,
                                            unsigned char *history_bitmask,
                                            float *input_floats,
                                            unsigned short *input_ushorts)
{
    int h;
    int stat_idx;
    float val;
    /* could make s[] and sigma_cutoff[] local vars instead */
    /* of malloc/free them, but their combined size alone is -11MB */
    /* which on some systems blows the default stack size (e.g., 8MB) */
    /* ... easier to malloc/free them rather than wrestle with stack sizes */
    /* struct stats s[N_STAT_CELLS]; */
    /* float sigma_cutoff[N_STAT_CELLS]; */
    struct stats *s;
    float *sigma_cutoff;
    PCT_MALLOC(s, N_STAT_CELLS*sizeof(struct stats), struct stats);
    PCT_MALLOC(sigma_cutoff, N_STAT_CELLS*sizeof(float), float);
/* printf("%d: ENTER remove_histories_from_binned_stats()\n", Myid); */
    /* initializing stats */
    for (h = 0; h < N_STAT_CELLS; h ++)
    s[h].n = 0;
    /****************************************************/
    /* PHASE 1 of 3 - compute local running mean and SSE */
    /****************************************************/
    for (h = 0; h < nhistories; h ++)
    {
        if (TEST(history_bitmask, h))
        {
            /*********************************/
            /* accumulating stats for delta x */
            /*********************************/
            stat_idx = get_stat_cell_idx(input_floats,
                                input_ushorts,
                                h,
                                STAT_DELTA_X);
            val = compute_stat_val(input_floats,
                            input_ushorts,
                            h,
                            STAT_DELTA_X) ;
            accumulate_stats_val(&(s[stat_idx].mean),
                        &(s[stat_idx].sse),
#ifdef DEBUG
                        &(s[stat_idx].min),
                        &(s[stat_idx].max),
#endif
                        &(s[stat_idx].n),
                        val);
            /*********************************/
            /* accumulating stats for delta y */
            /*********************************/
            stat_idx = get_stat_cell_idx(input_floats,
                                input_ushorts,
                                h,
                                STAT_DELTA_Y);
```

```
                    val = compute_stat_val(input_floats,
                                    input_ushorts,
                                    h,
                                    STAT_DELTA_Y);
                    accumulate_stats_val(&(s[stat_idx].mean),
                                    &(s[stat_idx].sse),
#ifdef DEBUG
                                    &(s[stat_idx].min),
                                    &(s[stat_idx].max),
#endif
                                    &(s[stat_idx].n),
                                val);
                    /***************************************/
                    /* accumulating stats for delta energy */
                    /***************************************/
                    stat_idx = get_stat_cell_idx(input_floats,
                                        input_ushorts,
                                        h,
                                        STAT_DELTA_ENERGY);
                    val = compute_stat_val(input_floats,
                                    input_ushorts,
                                    h,
                                    STAT_DELTA_ENERGY);
                    accumulate_stats_val(&(s[stat_idx].mean),
                                    &(s[stat_idx].sse),
#ifdef DEBUG
                                    &(s[stat_idx].min),
                                    &(s[stat_idx].max),
#endif
                                    &(s[stat_idx].n),
                                val);
        } /* endif */
    } /* endfor */
#if DEBUG
    /****************/
    /* sanity check */
    /****************/
    {
        int i,j;
        for (i = 0; i < N_STAT_CELLS; i += STAT_NSTATS)
        {
            for (j = 1; j < STAT_NSTATS; j ++)
            {
                if (s[i].n != s[i+j].n)
                {
                    fprintf(stderr,
                        "ERROR: %s %d: proc %d: "
                        "LOCAL STAT: stats_idx %d: "
                        "found n_0 = %d != n_%d = %d "
                        "(must be equal)\n",
                        _FILE_, _LINE_, Myid,
                        h, s[i].n, j, s[i+j].n);
                    PCT_ABORT(ABORT_STATS_ERROR)
                } /* endif */
            } /* endfor */
        } /* endfor */
        for (i = 0; i < N_STAT_CELLS; i += STAT_NSTATS)
        {
            for (j = 0; j < STAT_NSTATS; j ++)
            {
                if (s[i+j].n > 2 && s[i+j].sse <= 0.0)
                {
                    fprintf(stderr,
```

```
                                "WARNING: %s %d: proc %d: "
                                "LOCAL STAT: stats_idx %d: found n_%d = %d sse_%d = %f "
                                "(sse probably should be > 0)\n",
                                _FILE_, _LINE_, Myid,
                                i, j, s[i+j].n, j, s[i+j].sse);
                        } /* endif */
                } /* endfor */
        } /* endfor */
    }
#endif
    /***********************************************************/
    /* PHASE 2 of 3 - compute and distribute global mean and SSE */
    /***********************************************************/
#ifndef NO_MPI
    {
        MPI_Datatype Stats_mpi;
#ifdef DEBUG
        int block_lengths[5]; /* number of "things" in each element of */
                    /* our new struct. for us, 1 each */
        MPI_Aint displs[5]; /* displacement of each element */
        MPI_Datatype types[5]; /* type of each element */
#else
                    int block_lengths[3]; /* number of "things" in each element of */
                    /* our new struct. for us, 1 each */
        MPI_Aint displs[3]; /* displacement of each element */
        MPI_Datatype types[3]; /* type of each element */
#endif
        MPI_Aint start_addr;
        MPI_Aint addr;
        MPI_Op op;
        block_lengths[0] = block_lengths[1] = block_lengths[2]
#ifdef DEBUG
        = block_lengths[3] = block_lengths[4]
#endif
        = 1;
          types[0] = MPI_FLOAT;
          types[1] = MPI_FLOAT;
          types[2] = MPI_UNSIGNED;
#ifdef DEBUG
          types[3] = MPI_FLOAT;
          types[4] = MPI_FLOAT;
#endif
        MPI_Address(&(s[0].mean), &start_addr);
        displs[0] = 0;
        MPI_Address(&(s[0].sse), &addr);
        displs[1] = addr - start_addr;
        MPI_Address(&(s[0].n), &addr);
        displs[2] = addr - start_addr;
#ifdef DEBUG
        MPI_Address(&(s[0].min), &addr);
        displs[3] = addr - start_addr;
        MPI_Address(&(s[0].max), &addr);
        displs[4] = addr - start_addr;
#endif
        * build new derived datatype */
         MPI_Type_struct(
#ifdef DEBUG
                        5, /* nelements */
#else
                        3, /* nelements */
#endif
                        block_lengths,
                        displs,
```

```
                                    types,
                                   &Stats_mpi);
                MPI_Type_commit(&Stats_mpi);
                MPI_Op_create((MPI_User_function*) combine_stats, // my user-def func
                        1,                                         // commute flag
                        &op);                                      // op handle
                MPI_Allreduce(MPI_IN_PLACE, // clobber operand with result
                                        s, // operand that gets clobbered by result
                            N_STAT_CELLS, // count
                                Stats_mpi, // data type
                                      op, // operator
                            MPI_COMM_WORLD);
                MPI_Op_free(&op);
        }
    #endif
    #if DEBUG
                /****************/
                /* sanity check */
                /****************/
                {
                        int i,j;
                        for (i = 0; i < N_STAT_CELLS; i += STAT_NSTATS)
                        {
                        for (j = 1; j < STAT_NSTATS; j ++)
                        {
                            if (s[i].n != s[i+j].n)
                            {
                                fprintf(stderr,
                                    "ERROR: %s %d: proc %d: "
                                    "GLOBAL STAT: stats_idx %d: "
                                    "found n_0 = %d != n_%d = %d "
                                    "(must be equal)\n",
                                    _FILE_, _LINE_, Myid,
                                     h, s[i].n, j, s[i+j].n);
                                PCT_ABORT(ABORT_STATS_ERROR)
                            } /* endif */
                        } /* endfor */
                } /* endfor */
                for (i = 0; i < N_STAT_CELLS; i += STAT_NSTATS)
                {
                    for (j = 0; j < STAT_NSTATS; j ++)
                    {
                        if (s[i+j].n > 2 && s[i+j].sse <= 0.0)
                        {
                            fprintf(stderr,
                                "WARNING: %s %d: proc %d: "
                                "GLOBAL STAT: "
                                "stats_idx %d: found n_%d = %d sse_%d = %f "
                                "(sse probably should be > 0)\n",
                                FILE_, _LINE_, Myid,
                                 i, j, s[i+j].n, j, s[i+j].sse);
                        } /* endif */
                    } /* endfor */
                } /* endfor */
        }
    #endif
    /* computing sigma cuttoff for each stat cell */
    for (h = 0; h < N_STAT_CELLS; h ++)
    {
        if (s[h].n == 0)
            sigma_cutoff[h] = 0; // arbitrary value, should never be used
        else
            //KIRK:sigma_cutoff[h]=s[h].mean+(MAX_SIGMA*sqrt(s[h].sse/(s[h].n)));
```

```
            sigma_cutoff[h] = MAX_SIGMA*sqrt(s[h].sse/s[h].n);
        } /* endfor */
    /* dumping stats */
    {
        FILE *fp_x, *fp_y, *fp_e;
        FILE *fp = NULL;
        char fname[300];
        if (Myid == ForemanRank)
        {
            sprintf(fname, "%s/stats.x.%s.%s",
                    OutputDirBase, Nowstring, Relaxparamstring);
             if ((fp_x = fopen(fname, "w")) == NULL)
              {
              fprintf(stderr, "ERROR: could not open output stats file >%s<\n",
                    fname);
               PCT_ABORT(ABORT_FAILED_FOPEN)
               } /* endif */
            sprintf(fname, "%s/stats.y.%s.%s",
                    OutputDirBase, Nowstring, Relaxparamstring);
             if ((fp_y = fopen(fname, "w")) == NULL)
        {
            fprintf(stderr, "ERROR: could not open output stats file >%s<\n",
                    fname);
            PCT_ABORT(ABORT_FAILED_FOPEN)
        } /* endif */
            sprintf(fname, "%s/stats.e.%s.%s",
                OutputDirBase, Nowstring, Relaxparamstring);
            if ((fp_e = fopen(fname, "w")) == NULL)
             {
             fprintf(stderr, "ERROR: could not open output stats file >%s<\n",
                  fname);
              PCT_ABORT(ABORT_FAILED_FOPEN)
        } /* endif */
        for (h = 0; h < N_STAT_CELLS; h ++)
        {
            if ((h % 3) == 0) fp = fp_x;
            if ((h % 3) == 1) fp = fp_y;
            if ((h % 3) == 2) fp = fp_e;
#ifdef DEBUG
            fprintf(fp, "%d %f %f %f %f %f\n",
                s[h].n, s[h].mean, s[h].sse, sigma_cutoff[h],
                s[h].min, s[h].max);
#else
            fprintf(fp, "%d %f %f %f\n",
                 s[h].n, s[h].mean, s[h].sse, sigma_cutoff[h]);
#endif
        } /* endfor */
        fclose(fp_x);
        fclose(fp_y);
        fclose(fp_e);
    } /* endif */
    }
        /********************************************************************/
        /* PHASE 3 of 3 - throwing out rogue histories (outside MAX_SIGMA) */
        /********************************************************************/
        remove_based_on_binned_stat(nhistories,
                    history_bitmask,
                    input_floats,
                    input_ushorts,
                    s,
                    sigma_cutoff,
                    STAT_DELTA_X);
        remove_based_on_binned_stat(nhistories,
```

```
                        history_bitmask,
                        input_floats,
                        input_ushorts,
                        s,
                        sigma_cutoff,
                        STAT_DELTA_Y);
            remove_based_on_binned_stat(nhistories,
                        history_bitmask,
                        input_floats,
                        input_ushorts,
                        s,
                        sigma_cutoff,
                        STAT_DELTA_ENERGY);
    PCT_FREE(s);
    PCT_FREE(sigma_cutoff);
    return;
} /* end remove_histories_from_binned_stats() */
// NICK: need to write test_solution_for_done()
static int test_solution_for_done(float solution[])
{
    int rc;
    Iterations ++;
    rc = (Iterations >= Niters);
    return rc;
} /* end test_solution() */
#include "pct.h"
#ifndef NO_MPI
int MPI_Init(int *argc, char ***argv)
{
                    int idx = MPI_INIT_IDX;
                    int rc;
                    int i;
                    double start_time, end_time;
                    start_time = MPI_Wtime();
                    strcpy(func_profiles[MPI_INIT_IDX].func_name,       "MPI_Init");
                    strcpy(func_profiles[MPI_COMM_SIZE_IDX].func_name,  "MPI_Comm_size");
                    strcpy(func_profiles[MPI_COMM_RANK_IDX].func_name,  "MPI_Comm_rank");
                    strcpy(func_profiles[MPI_ADDRESS_IDX].func_name,    "MPI_Address");
                    strcpy(func_profiles[MPI_TYPE_STRUCT_IDX].func_name, "MPI_Type_struct");
                    strcpy(func_profiles[MPI_TYPE_COMMIT_IDX].func_name, "MPI_Type_commit");
                    strcpy(func_profiles[MPI_OP_CREATE_IDX].func_name,  "MPI_Op_create");
                    strcpy(func_profiles[MPI_OP_FREE_IDX].func_name,    "MPI_Op_free");
                    strcpy(func_profiles[MPI_ALLREDUCE_IDX].func_name,  "MPI_Allreduce");
                    strcpy(func_profiles[MPI_BCAST_IDX].func_name,      "MPI_Bcast");
                    strcpy(func_profiles[MPI_SEND_IDX].func_name,       "MPI_Send");
                    strcpy(func_profiles[MPI_RECV_IDX].func_name,       "MPI_Recv");
                    for (i = 0; i < Nfuncs; i ++)
                    {
                        func_profiles[i].call_count = 0;
                        func_profiles[i].total_time_secs = 0.0;
                        func_profiles[i].does_communicate = 0;
                        func_profiles[i].total_bytes = 0;
                    } /* endfor */
                    /* fixing those that DO communicate */
                    func_profiles[MPI_ALLREDUCE_IDX].does_communicate
                        = func_profiles[MPI_BCAST_IDX].does_communicate
                        = func_profiles[MPI_SEND_IDX].does_communicate
                        = func_profiles[MPI_RECV_IDX].does__communicate = 1;
                    rc = PMPI_Init(argc, argv);
                    end_time = MPI_Wtime();
                    (func_profiles[idx].call_count) ++;
                    (func_profiles[idx].total_time_secs) += (end_time - start_time);
                    return rc;
```

```
} /* end MPI_Init() */
int MPI_Comm_size(MPI_Comm comm, int *size)
{
                int idx = MPI_COMM_SIZE_IDX;
                int rc;
                double start_time, end_time;
                start_time = MPI_Wtime();
                rc = PMPI_Comm_size(comm, size);
                end_time = MPI_Ntime();
                (func_profiles[idx].call_count) ++;
                (func_profiles[idx].total_time_secs) += (end_time - start_time);
                return rc;
} /* end MPI_Comm_size() */
int MPI_Comm_rank(MPI_Comm comm, int *rank)
{
                int idx = MPI_COMM_RANK_IDX;
                int rc;
                double start_time, end_time;
                start_time = MPI_Wtime();
                rc = PMPI_Comm_rank(comm, rank);
                end_time = MPI_Wtime();
                (func_profiles[idx].call_count) ++;
                (func_profiles[idx].total_time_secs) += (end_time - start_time);
                return rc;
} /* end MPI_Comm_rank() */
int MPI_Address(void *location, MPI_Aint *address)
 {
                int idx = MPI_ADDRESS_IDX;
                int rc;
                double start_time, end_time;
                start_time = MPI_Wtime();
                rc = PMPI_Address(location, address);
                end_time = MPI_Wtime();
                (func_profiles[idx].call_count) ++;
                (func_profiles[idx].total_time_secs) += (end_time - start_time);
                return rc;
} /* end MPI_Address() */
int MPI_Type_struct(int count,
        int *array_of_blocklengths,
        MPI_Aint *array_of_displacements,
        MPI_Datatype *array_of_types,
        MPI_Datatype *newtype)
{
    int idx = MPI_TYPE_STRUCT_IDX;
    int rc;
     double start_time, end_time;
     start_time = MPI_Wtime();
     rc = PMPI_Type_struct(count,
                array_of_blocklengths,
                array_of_displacements,
                array_of_types,
                newtype);
    end_time = MPI_Wtime();
    (func_profiles[idx].call_count) ++;
    (func_profiles[idx].total_time_secs) += (end_time - start_time);
     return rc;
} /* end MPI_Type_struct() */
int MPI_Type_commit(MPI_Datatype *datatype)
{
    int idx = MPI_TYPE_COMMIT_IDX;
    int rc;
    double start_time, end_time;
    start_time = MPI_Wtime();
```

```
    rc = PMPI_Type_commit(datatype);
    end_time = MPI_Wtime();
    (func_profiles[idx].call_count) ++;
    (func_profiles[idx].total_time_secs) += (end_time - start_time);
    return rc;
} /* end MPI_Type_commit() */
int MPI_Op_create(MPI_User_function *function, int commute, MPI_Op *op)
{
    int idx = MPI_OP_CREATE_IDX;
    int rc;
    double start_time, end_time;
    start_time = MPI_Wtime();
    rc = PMPI_Op_create(function, commute, op);
    end_time = MPI_Wtime();
    (func_profiles[idx].call_count) ++;
    (func_profiles[idx].total_time_secs) += (end_time - start_time);
    return rc;
} /* end MPI_Op_create() */
int MPI_Op_free(MPI_Op *op)
{
    int idx = MPI_OP_FREE_IDX;
    int rc;
    double start_time, end_time;
    start_time = MPI_Wtime();
    rc = PMPI_Op_free(op);
    end_time = MPI_Wtime();
    (func_profiles[idx].call_count) ++;
    (func_profiles[idx].total_time_secs) += (end_time - start_time);
    return rc;
} /* end MPI_Op_free() */
/*****************************/
/* Message Passing functions */
/*****************************/
int MPI_Allreduce (void *sendbuf,
        void *recvbuf,
        int count,
        MPI_Datatype datatype,
        MPI_Op op,
        MPI_Comm comm)
{
        int idx = MPI_ALLREDUCE_IDX;
        int rc;
        double start_time, end_time;
        int extent;
        start_time = MPI_Wtime();
        rc = PMPI_Allreduce(sendbuf, recvbuf, count, datatype, op, comm);
        end_time = MPI_Wtime();
        (func_profiles[idx].call_count) ++;
        (func_profiles[idx].total_time_secs) += (end_time - start_time);
        MPI_Type_size(datatype, &extent);
        (func_profiles[idx].total_bytes) += (count * extent);
        return rc;
} /* end MPI_Allreduce() */
int MPI_Bcast(void *buffer,
        int count,
        MPI_Datatype datatype,
        int root,
        MPI_Comm comm)
{
        int idx = MPI_BCAST_IDX;
        int rc;
        double start_time, end_time;
        int extent;
```

```
      start_time = MPI_Wtime();
      rc = PMPI_Bcast(buffer, count, datatype, root, comm);
      end_time = MPI_Wtime();
      (func_profiles[idx].call_count) ++;
      (func_profiles[idx].total_time_secs) += (end_time - start_time);
      MPI_Type_size(datatype, &extent);
      (func_profiles[idx].total_bytes) += (count * extent);
      return rc;
} /* end MPI_Bcast() */
int MPI_Send (void *buf,
      int count,
       MPI_Datatype datatype,
       int dest,
       int tag,
       MPI_Comm comm)
   {
   int idx = MPI_SEND_IDX;
   int rc;
   double start_time, end_time;
   int extent;
   start_time = MPI_Wtime();
   rc = PMPI_Send(buf, count, datatype, dest, tag, comm);
   end_time = MPI_Wtime();
   (func_profiles[idx].call_count) ++;
   (func_profiles[idx].total_time_secs) += (end_time - start_time);
   MPI_Type_size(datatype, &extent);
   (func_profiles[idx].total_bytes) += (count * extent);
   return rc;
} /* end MPI_Send() */
int MPI_Recv(void *buf,
      int count,
      MPI_Datatype datatype,
      int source,
      int tag,
      MPI_Comm comm,
      MPI_Status *status)
{
  int idx = MPI_RECV_IDX;
  int rc;
  double start_time, end_time;
  int extent;
  start_time = MPI_Wtime();
  rc = PMPI_Recv(buf, count, datatype, source, tag, comm, status);
  end_time = MPI_Wtime();
  (func_profiles[idx].call_count) ++;
  (func_profiles[idx].total_time_secs) += (end_time - start_time);
  MPI_Type_size(datatype, &extent);
  (func_profiles[idx].total_bytes) += (count * extent);
  return rc;
} /* end MPI_Recv() */
#endif
```

The invention has been described in an illustrative manner, and it is to be understood that the terminology, which has been used is intended to be in the nature of words of description rather than of limitation.

Obviously, many modifications and variations of the present invention are possible in light of the above teachings. It is, therefore, to be understood that within the scope of the appended claims, the invention can be practiced otherwise than as specifically described.

**Claims**

1. A proton computed tomography (pCT) detector system (10), comprising: two tracking detectors (12) in sequence on a first side of an object to be imaged, two tracking detectors (12) in sequence on an opposite side of the object (16) to be imaged, a calorimeter (14), and a computer cluster, **characterised in** said tracking detectors (12) including a plurality of silicon photomultipliers (SiPM) (20) and one or two plastic scintillation fibers (22) connected to each SiPM (20), wherein the scintillation fibers (22) include at least two layers arranged in a plane that are supported by a Rohacell support (26).

2. The pCT detector system (10) of claim 1, wherein said plastic scintillation fibers (22) have a 1 mm diameter.

3. The pCT detector system (10) of claim 1, wherein said plastic scintillation fibers (22) are packed in a row covering the full area of an imaging field.

4. The pCT detector system (10) of claim 3, wherein said imaging field is 27 by 36 cm.

5. The pCT detector system (10) of claim 1, wherein said two tracking detectors are oriented in X and Y directions.

6. The pCT detector system (10) of claim 5, further including a thin foam-like material in between said two tracking detectors.

7. The pCT detector system (10) of claim 1, wherein said plastic scintillation fibers (22) have a cross-sectional shape chosen from the group consisting of a square, circle, and hexagon.

8. The pCT detector system (10) of claim 1, wherein two plastic scintillation fibers (22) are attached to each SiPM (20).

9. The pCT detector system (10) of claim 1, wherein one plastic scintillation fiber (22) is attached to each SiPM (20).

10. The pCT detector system (10) of claim 1, wherein said tracking detectors(12) further include support means for supporting the fibers (22) and SiPMs (20).

11. The pCT detector system (10) of claim 1, wherein said plastic scintillation fibers (22) are made of polystyrene with cladding of PMMA (Poly(methyl methacrylate)).

12. The pCT detector system (10) of claim 1, wherein said calorimeter (14) includes silicon photomultipliers (SiPMs)(20) attached to wavelength shifting (WLS) fibers.

13. The pCT detector system (10) of claim 12, wherein said calorimeter (14) includes a stack of scintillator plates (34), and wherein each scintillator plate (34) includes one SiPM (20) and one WLS.

14. The pCT detector system (10) of claim 1, wherein said computer cluster further includes analyzing means for analyzing the protons detected by the tracking detectors (12) and calorimeter (14).

15. The pCT detector system (10) of claim 14, wherein said analyzing means includes a foreman computer and multiple worker computers.

16. The pCT detector system (10) of claim 1, further including a rotating stage (28) for rotating the object (16), and wherein said tracking detectors (12) are inside enclosures.

17. A method of imaging an object, including the steps of: emitting protons from a source (18) through two tracking detectors (12), through and around the object (16), and through two opposite tracking detectors (12); detecting energy of the protons with a calorimeter (14); and imaging the object (16); and wherein:

   a) the tracking detectors (12) include plastic scintillation fibers (22) packed in a row covering the full area of an imaging field and include silicon photomultipliers (20), wherein said tracking detectors (12) include a plurality of silicon photomultipliers (SiPM) (20) and one or two plastic scintillation fibers (22) connected to each SiPM (20), wherein the scintillation fibers (22) include at least two layers arranged in a plane that are supported by a Rohacell support (26), optionally wherein:

      i) said emitting step further includes the step of determining a position of the protons at the two tracking detectors (12) and two opposite tracking detectors (12);

ii) said determining step further includes the step of measuring the protons in X and Y planes of the tracking detectors (12); or

iii) the calorimeter (14) includes silicon photomultipliers (20) attached to wavelength shifting (WLS) fibers;

or

b) said imaging step includes analyzing data acquired from the tracking detectors (12) and calorimeter (14) on a cluster of multiple computers and graphic processing units with a Message Passing Interface (MPI) standard, optionally:

i) wherein said imaging step includes compact memory representation and solving an entire 3D image space at a single time; or

ii) further including the steps of a foreman computer distributing equal amounts of proton histories to multiple worker computers and the foreman computer, computing an Integrated Electron Density (IED) and Most Likely Path (MLP), solving for a solution vector and storing on computer readable memory, sending copies of the solution vector to the foreman computer, combining the solution vectors of the worker computers and the foreman computer and storing the combined solution vector on computer readable media, testing the combined solution vector, and if the combined solution vector is done, producing an image of the object, preferably wherein if the combined solution vector is not done, transmitting the combined solution vector to the worker computers, and repeating the distributing, computing, solving, sending, combining, testing steps until the combined solution vector is done, and producing an image of the object (16).

**Patentansprüche**

1. Ein Protonen-Computertomographie (pCT)-Detektorsystem (10), umfassend:

Zwei Spurdetektoren (12) in Folge auf einer ersten Seite eines abzubildenden Objekts, zwei Spurdetektoren (12) in Folge auf einer gegenüberliegenden Seite des abzubildenden Objekts (16), ein Kalorimeter (14) und ein Computer-Cluster, dadurch charakterisiert, dass jene Spurdetektoren (12) eine Vielzahl von Silicium-Photomultipliern (SiPM) (20) umfassen und eine oder zwei plastische Szintillationsfasern (22), die mit jedem SiPM (20) verbunden sind, wobei die Szintillationsfasern (22) mindestens zwei Schichten umfassen, die in einer Ebene angeordnet sind und die durch einen Rohacell-Träger (26) unterstützt werden.

2. Das pCT-Detektorsystem (10) von Anspruch 1, wobei jene plastischen Szintillationsfasern (22) einen Durchmesser von 1 mm haben.

3. Das pCT-Detektorsystem (10) von Anspruch 1, wobei jene plastischen Szintillationsfasern (22) in eine Reihe gepackt sind, die volle Fläche eines Bildfelds abdeckend.

4. Das pCT-Detektorsystem (10) von Anspruch 3, wobei jenes Bildfeld 27 mal 36 cm groß ist.

5. Das pCT-Detektorsystem (10) von Anspruch 1, wobei jene zwei Spurdetektoren in X-und Y-Richtung orientiert sind.

6. Das pCT-Detektorsystem (10) von Anspruch 5, ferner umfassend ein dünnes, schaumartiges Material zwischen jenen zwei Spurdetektoren.

7. Das pCT-Detektorsystem (10) von Anspruch 1, wobei jene plastischen Szintillationsfasern (22) eine Querschnittsfläche haben, ausgewählt aus der Gruppe bestehend aus einem Quadrat, einem Kreis und einem Sechseck.

8. Das pCT-Detektorsystem (10) von Anspruch 1, wobei zwei plastische Szintillationsfasern (22) an jedem SiPM (20) angebracht sind.

9. Das pCT-Detektorsystem (10) von Anspruch 1, wobei eine plastische Szintillationsfaser (22) an jedem SiPM (20) angebracht ist.

10. Das pCT-Detektorsystem (10) von Anspruch 1, wobei jene Spurdetektoren (12) ferner Unterstützungsmittel zur Unterstützung der Fasern (22) und SiPMs (20) umfassen.

**11.** Das pCT-Detektorsystem (10) von Anspruch 1, wobei jene plastischen Szintillationsfasern (22) aus Polystyren mit einer Hülle aus PMMA (Polymethylmethacrylat) gemacht sind.

**12.** Das pCT-Detektorsystem (10) von Anspruch 1, wobei jenes Kalorimeter (14) Silicium-Photomultiplier (SiPMs) (20) umfasst, angebracht an Wellenlängenschieber (WLS)-Fasern.

**13.** Das pCT-Detektorsystem (10) von Anspruch 12, wobei jenes Kalorimeter (14) einen Stapel von Szintillator-Platten (34) umfasst und wobei jede Szintillator-Platte (34) einen SiPM (20) und einen WLS umfasst.

**14.** Das pCT-Detektorsystem (10) von Anspruch 1, wobei jener Computer-Cluster ferner Analysemittel umfasst zur Analyse der Protonen, die von den Spurdetektoren (12) und dem Kalorimeter (14) detektiert werden.

**15.** Das pCT-Detektorsystem (10) von Anspruch 14, wobei jene Analysemittel einen leitenden Computer und mehrere Arbeitscomputer umfassen.

**16.** Das pCT-Detektorsystem (10) von Anspruch 1, ferner umfassend eine rotierende Bühne (28) zur Rotation des Objekts (16), und wobei jene Spurdetektoren (12) sich in Gehäusen befinden.

**17.** Eine Methode der Bildgebung eines Objekts, umfassend die Schritte von: Emission von Protonen von einer Quelle (18) durch zwei Spurdetektoren (12), durch und um das Objekt (16) und durch zwei gegenüberliegende Spurdetektoren (12); Detektion der Energie der Protonen mit einem Kalorimeter (14); und Bildgebung des Objekts (16); und wobei

a) die Spurdetektoren (12) plastische Szintillationsfasern (22) umfassen, die in eine Reihe gepackt sind, die volle Fläche eines Bildfelds abdeckend, und Silicium-Photomultiplier (20) umfassen, wobei jene Spurdetektoren (12) eine Vielzahl von Silicium-Photomultipliern (SiPM) (20) und eine oder zwei plastische Szintillationsfasern (22), verbunden mit jedem SiPM (20), umfassen, wobei die Szintillationsfasern (22) mindestens zwei Schichten umfassen, die in einer Ebene angeordnet sind und die durch einen Rohacell-Träger (26) unterstützt werden, wobei optional:

i) jener Emissionsschritt ferner den Schritt der Bestimmung einer Position der Protonen bei den zwei Spurdetektoren (12) und zwei gegenüberliegenden Spurdetektoren (12) umfasst;
ii) jener Detektionsschritt ferner den Schritt der Messung der Protonen in X-und Y-Ebenen der Spurdetektoren (12) umfasst; oder
iii) das Kalorimeter (14) Silicium-Photomultiplier (20), angebracht an Wellenlängenschieber (WLS)-Fasern umfasst; oder

b) jener Bildgebungsschritt die Analyse von Daten, die von den Spurdetektoren (12) und Kalorimeter (14) aufgenommen wurden auf einem Cluster von mehreren Computern und graphischen Prozessierungeinheiten mit einem Message-Passing-Interface (MPI)-Standard umfasst, optional

i) wobei jener Bildgebungsschritt kompakte Speicherdarstellung und das Lösen eines gesamten 3D-Bildraums zu einer einzigen Zeit umfasst; oder
ii) ferner umfassend die Schritte des Verteilens gleicher Mengen von Protonen-Verläufen auf mehrere Arbeitscomputer und den leitenden Computer durch den leitenden Computer, das Berechnen einer integrierten Elektronendichte (IED) und eines wahrscheinlichsten Wegs (MLP), das Lösen für einen Lösungsvektor und Speichern auf einem computerlesbaren Speicher, das Senden von Kopien des Lösungsvektors an den leitenden Computer, das Kombinieren der Lösungsvektoren der Arbeitscomputer und des leitenden Computers und das Speichen des kombinierten Lösungsvektors auf computerlesbaren Medien, das Testen des kombinierten Lösungsvektors, und falls der kombinierte Lösungsvektor gemacht wurde, das Erstellen eines Bild des Objekts, wobei vorzugsweise, falls der kombinierte Lösungsvektor nicht gemacht wurde, das Übertragen des kombinierten Lösungsvektors an die Arbeitscomputer und die Wiederholung der Verteil-, Rechen-, Löse-, Sende-, Kombinier-, Testschritte bis der kombinierte Lösungsvektor gemacht ist, und das Erstellen eines Bilds des Objekts (16).

**Revendications**

1. Système de détecteur (10) à tomographie informatisée par protons (pCT), comprenant : deux trajectographes (12) en séquence sur un premier côté d'un objet à imager, deux trajectographes (12) en séquence sur un côté opposé de l'objet (16) à imager, un calorimètre (14) et un groupement d'ordinateurs, **caractérisé en ce que** lesdits trajectographes (12) incluent une pluralité de photomultiplicateurs au silicium (SiPM) (20) et une ou deux fibres de scintillation en plastique (22) connectées à chaque SiPM (20), dans lequel les fibres de scintillation (22) incluent au moins deux couches agencées dans un plan qui sont supportées par un support Rohacell (26).

2. Le système de détecteur pCT (10) selon la revendication 1, dans lequel lesdites fibres de scintillation en plastique (22) ont un diamètre de 1 mm.

3. Le système de détecteur pCT (10) selon la revendication 1, dans lequel lesdites fibres de scintillation en plastique (22) sont condensées dans une rangée couvrant toute la surface d'un champ d'imagerie.

4. Le système de détecteur pCT (10) selon la revendication 3, dans lequel ledit champ d'imagerie fait 27 par 36 cm.

5. Le système de détecteur pCT (10) selon la revendication 1, dans lequel lesdits deux trajectographes sont orientés dans des directions X et Y.

6. Le système de détecteur pCT (10) selon la revendication 5, incluant en outre un matériau mince de type mousse entre lesdits deux trajectographes.

7. Le système de détecteur pCT (10) selon la revendication 1, dans lequel lesdites fibres de scintillation en plastique (22) ont une forme en coupe transversale choisie dans le groupe constitué d'un carré, d'un cercle et d'un hexagone.

8. Le système de détecteur pCT (10) selon la revendication 1, dans lequel deux fibres de scintillation en plastique (22) sont attachées à chaque SiPM (20).

9. Le système de détecteur pCT (10) selon la revendication 1, dans lequel une fibre de scintillation en plastique (22) est attachée à chaque SiPM (20).

10. Le système de détecteur pCT (10) selon la revendication 1, dans lequel lesdits trajectographes (12) incluent en outre des moyens de support pour supporter les fibres (22) et les SiPM (20).

11. Le système de détecteur pCT (10) selon la revendication 1, dans lequel lesdites fibres de scintillation en plastique (22) sont composées de polystyrène avec un enrobage de PMMA (poly(méthacrylate de méthyle)).

12. Le système de détecteur pCT (10) selon la revendication 1, dans lequel ledit calorimètre (14) inclut des photomultiplicateurs au silicium (SiPM) (20) attachés à des fibres à décalage de longueur d'onde (WLS).

13. Le système de détecteur pCT (10) selon la revendication 12, dans lequel ledit calorimètre (14) inclut une pile de plaques de scintillateur (34), et dans lequel chaque plaque de scintillateur (34) inclut un SiPM (20) et une WLS.

14. Le système de détecteur pCT (10) selon la revendication 1, dans lequel ledit groupement d'ordinateurs inclut en outre des moyens d'analyse pour analyser les protons détectés par les trajectographes (12) et le calorimètre (14).

15. Le système de détecteur pCT (10) selon la revendication 14, dans lequel lesdits moyens d'analyse incluent un ordinateur meneur et de multiples ordinateurs travailleurs.

16. Le système de détecteur pCT (10) selon la revendication 1, incluant en outre un étage rotatif (28) pour faire tourner l'objet (16), et dans lequel lesdits trajectographes (12) se trouvent à l'intérieur d'enceintes.

17. Procédé d'imagerie d'un objet, incluant les étapes consistant à: émettre des protons à partir d'une source (18) à travers deux trajectographes (12), à travers et autour de l'objet (16), et à travers deux trajectographes opposés (12) ; détecter l'énergie des protons avec un calorimètre (14) ; et imager l'objet (16) ; et dans lequel :

    a) les trajectographes (12) incluent des fibres de scintillation en plastique (22) condensées dans une rangée couvrant toute la surface d'un champ d'imagerie et incluent des photomultiplicateurs au silicium (20), dans lequel lesdits trajectographes (12) incluent une pluralité de photomultiplicateurs au silicium (SiPM) (20) et une ou deux fibres de scintillation en plastique (22) connectées à chaque SiPM (20), dans lequel les fibres de scintillation (22) incluent au moins deux couches agencées dans un plan qui sont supportées par un support Rohacell (26), optionnellement dans lequel :

i) ladite étape d'émission inclut en outre l'étape de détermination d'une position des protons au niveau des deux trajectographes (12) et des deux trajectographes opposés (12) ;
ii) ladite étape de détermination inclut en outre l'étape de mesure des protons dans des plans X et Y des trajectographes (12) ; ou
iii) le calorimètre (14) inclut des photomultiplicateurs au silicium (20) attachés à des fibres à décalage de longueur d'onde (WLS) ;
ou

b) ladite étape d'imagerie inclut l'analyse de données acquises depuis les trajectographes (12) et du calorimètre (14) sur un groupement de multiples ordinateurs et unités de traitement graphique avec une norme d'Interface de Passage de Message (MPI), optionnellement :

i) dans lequel ladite étape d'imagerie inclut la représentation de mémoire compacte et la résolution d'un espace d'image en 3D entier en même temps ; ou
ii) incluant en outre les étapes de distribution par un ordinateur meneur distribuant des quantités égales de trajectoires de protons à de multiples ordinateurs travailleurs et à l'ordinateur meneur, de calcul d'une densité électronique intégrée (IED) et d'un chemin le plus probable (MLP), de résolution d'un vecteur de solution et de stockage sur une mémoire lisible par ordinateur, d'envoi de copies du vecteur de solution à l'ordinateur meneur, de combinaison du vecteur de solution des ordinateurs travailleurs et de l'ordinateur meneur et le stockage du vecteur de solution combiné sur des supports lisibles par ordinateur, de test du vecteur de solution combiné, et si le vecteur de solution combiné est réalisé, de production d'une image de l'objet,

de préférence, dans lequel, si le vecteur de solution combiné n'est pas réalisé, la transmission du vecteur de solution combiné aux ordinateurs travailleurs, et la répétition des étapes de distribution, de calcul, de résolution, d'envoi, de combinaison, de test jusqu'à ce que le vecteur de solution combiné soit réalisé, et la production d'une image de l'objet (16).

FIGURE 1

FIGURE 2

FIGURE 3A

B

C

FIGURE 3

FIGURE 4

FIGURE 5

Phantom Orientation

Proton Beam Parameters

Tracker and Calorimeter Hits

Data Aquisition (DAQ) and Storage

Track and Energy/Range Reconstruction

Event Selection

Proton Histories (data)

Image Reconstruction Software

Image

FIGURE 6

FIGURE 7

FIGURE 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **B.P. WELFORD.** Note on a Method for Calculating Corrected Sums of Squares and Products. *Technometrics,* August 1962, vol. 4 (3), 419-420
- **D.E. KNUTH.** The Art of Computer Programming. vol. 2, 216
- **D.E. KNUTH.** The Art of Computer Programming. vol. 2, 232

- Updating Formulae and a Pairwise Algorithm for Computer Sample Variances. **T.F. CHAN ; G.H. GOLUB ; R.J. LEVEQUE.** Technical Report STAN-CS-79-773. Department of Computer Science, 1979